# EUROPEAN PATENT SPECIFICATION

(11) **EP 3 696 283 B1**
(45) Date of publication and mention of the grant of the patent: **16.02.2022**
(21) Application number: 20161862.6
(22) Date of filing: 07.03.2014
(51) Int. Cl.: C12Q 1/6886

(54) **MOLECULAR MARKERS IN BLADDER CANCER**
MOLEKULARE MARKER BEI BLASENKREBS
MARQUEURS MOLÉCULAIRES DANS LE CANCER DE LA VESSIE

(30) Priority: 08.03.2013 WO PCT/EP2013/054777
(43) Date of publication of application: 19.08.2020
(62) Divisional of application: 18150727.8
(73) Proprietor: MDxHealth Research B.V., 6534 AT Nijmegen (NL)
(72) Inventor: Smit, Franciscus Petrus, 6534 AT Nijmegen (NL); Hessels, Daphne, 6534 AT Nijmegen (NL); Schalken, Jacobus A., 6534 AT Nijmegen (NL)
(74) Representative: Arnold & Siedsma

(56) References cited:
- EP-A1- 2 311 983
- US-A1- 2011 306 514
- US-A1- 2012 282 167

## Description

The present invention relates to methods for establishing the presence, or absence, of a bladder tumour and/or establishing the prediction of prognosis and disease outcome for a human individual suffering from bladder cancer. The present invention further relates to the use of expression analysis of the indicated genes, or molecular markers, for establishing the presence, or absence, of a bladder tumour and/or establishing the prediction of prognosis and disease outcome for a human individual suffering from bladder cancer and to kit of parts for establishing the presence, or absence, of a bladder tumour and/or establishing the prediction of prognosis and disease outcome for a human individual suffering from bladder cancer.

Urinary bladder (or bladder) cancer is one of the most common cancers worldwide, with the highest incidence in industrialized countries. In the Western world, the chances of developing this type of cancer is 1 in 26, for women the chance is 1 in 90. Bladder cancer is the 4th most common cancer in men.

Two main histological types of bladder cancer are the urothelial cell carcinomas (UCC) and the squamous cell carcinomas (SCC). The UCCs are the most prevalent in Western and industrialized countries and are related to cigarette smoking and occupational exposure. The squamous cell carcinomas (SCC) are more frequently seen in some Middle Eastern and African countries where the schistosoma haematobium parasite is endemic.

In the Western world, 90% of the bladder tumours are UCCs, 3 to 5% are SCCs, and 1 to 2% are adenocarcinomas. Two third of the patients with UCC can be catogorized into non-muscle invasive bladder cancer (NMIBC) and one third in muscle invasive bladder cancer (MIBC).

In NMIBC, the disease is generally confined to the bladder mucosa (stage Ta, carcinoma in situ (CIS)) or bladder submucosa (stage T1). In MIBC, the patient has a tumour initially invading the detrusor muscle (stage T2), followed by the perivesical fat (stage T3) and the organs surrounding the bladder (stage T4). The management of these two types of UCC differs significantly. The management of NMIBC consists of transurethral resection of the bladder tumour (TURBT). However, after TURBT, 30% to 85% of patients develop recurrences. This high risk of recurrence makes bladder cancer one of the most prevalent human tumours.

Patients with NMIBC can be divided into 3 groups. 20% to 30% of patients have a relatively benign type of UCC, with a low recurrence rate. These low risk tumours do not exhibit progression. 40% to 50% of patients have so-called intermediate risk tumours. These patients often develop a superficial recurrence, but seldom progression. A small group of patients (20% to 30%) has a relatively aggressive superficial tumour at presentation and despite maximum treatment and 70% to 80% of these patients will have recurrent disease. 50% of these patients will develop muscle invasive disease associated with a poor prognosis. Therefore, there is a need to identify the patient group at risk for progression.

The primary treatment for MIBC is cystectomy. Despite this radical treatment, 50% of patients with primary MIBC develop metastases within 2 years after cystectomy and subsequently die of the disease. The 5-year tumour-specific survival of these patients is 55%. In comparison, patients with NMIBC have a 5-year tumour-specific survival of 88-90%. However, patients with MIBC who have a history of NMIBC, the 5-year tumour-specific survival drops to only 28%. These percentages emphasize the need for the identification of patients with a high risk of progression of their NMIBC.

The risk for progression and cancer related death is associated with tumour stage and grade. Currently, staging and grading of the tumour is used for making treatment decisions. Unfortunately, this procedure has led to overtreatment (*e*.*g*. cystectomy in patients who would have survived without this treatment) or undertreatment (*i*.*e*. patients with progressive disease dying after TURBT and who would have survived if they underwent cystectomy at an earlier stage). At present no reliable methods are available to accurately predict prognosis of individual patients with bladder cancer. The limited value of the established prognostic markers requires the analysis of new molecular parameters in predicting the prognosis and treatment of bladder cancer patients.

Bladder cancer is a genetic disorder driven by the progressive accumulation of multiple genetic and epigenetic changes. At the molecular level, these genetic changes result in uncontrolled cell proliferation, decreased cell death, invasion, and metastasis. The specific alterations in gene expression that occur as a result of interactions between various cellular pathways determine the biological behavior of the tumor, including growth, recurrence, progression, and metastasis, and may influence patient survival. To detect and monitor cancer and determine the likely prognosis, it is necessary to identify molecular markers of the disease that can be used in the clinic. EP 2 311 983 discloses the diagnosis of bladder cancer using sets of marker genes.

Considering the above, there is a need in the art for molecular markers capable of establishing the presence, or absence, of a bladder tumour and/or establishing the prediction of prognosis and disease outcome for a human individual suffering from bladder cancer. A suitable molecular marker preferably fulfils the following criteria:
1) it must be reproducible (intra- and inter- institutional); and
2) it must have an impact on clinical management.

It is an object of the present invention, amongst other objects, to meet at least partially, if not completely, the above stated needs of the art.

According to the present invention, the above object, amongst other objects, is met by bladder tumour markers and methods as outlined in the appended claims.

Specifically, the above object, amongst other objects, is met by an (*in vitro*) method for establishing the presence, or absence, of a bladder tumour in a human individual; or classification of the tumours according to aggressiveness, prediction of prognosis and/or disease outcome for a human individual suffering from bladder cancer comprising:
a) determining the expression of CTHRC1 in a sample originating from said human individual; andb) establishing upregulation of expression of CTHRC1 as compared to expression of CTHRC1 in a sample originating from said human individual not comprising tumour cells or tissue, or from an individual, or group of individuals, not suffering from bladder cancer; andc) establishing the presence, or absence, of a bladder tumour based on the established upregulation of CTHRC 1; or establishing the prediction of prognosis and disease outcome for a human individual suffering from bladder cancer based on the established upregulation of CTHRC 1.

According to the present invention, establishing the presence, or absence, of bladder cancer in a human individual preferably includes diagnosis, prognosis and/or prediction of disease survival.

It should be noted that the present method, when taken alone, does not suffice to diagnose an individual as suffering from bladder cancer. For this, a trained physician is required capable of taking into account factors not related to the present invention such as disease symptoms, history, pathology, general condition, age, sex, and/or other indicators. The present methods and molecular markers provide the trained physician with additional tools, or aids, to arrive at a reliable diagnosis.

According to the present invention, expression analysis comprises establishing an increased, or decreased, expression of a gene as compared to expression of this gene in non-bladder cancer tissue, i.e., under non-disease conditions.

For example, establishing an increased expression of CTHRC1 as compared to expression of this gene under non-bladder cancer conditions, allows establishing the presence, or absence, of a bladder tumour in a human individual suspected of suffering from bladder cancer and allows establishing the prediction of prognosis and disease outcome for an individual patient suffering from bladder cancer.

CTHRC1: collagen triple helix repeat containing-1 is a 30 kDa secreted protein that has the ability to inhibit collagen matrix synthesis. It is typically expressed at epithelial-mesenchymal interfaces. CTHRC1 is a cell-type-specific inhibitor TGF-β. Increased CTHRC1 expression results in morphological cell changes, increased cell proliferation, and decreased apoptosis.

According to the present invention, the method as described above is an *ex vivo* or *in vitro* method. In this embodiment, expression analysis of CTHRC1 is performed on a sample derived, originating or obtained from an individual suspected of suffering from bladder cancer. Such sample can be a body fluid such as saliva, lymph, blood or urine, or a tissue sample such as a transurethral resection of a bladder tumour (TURBT). Samples of, derived or originating from blood, such as plasma or cells, and urine, such as urine sediments, are preferably contemplated within the context of the present invention as are samples of, derived or originating from TURBT specimens.

According to another preferred embodiment of the present method, determining the expression comprises determining mRNA expression of CTHRC1

Expression analysis based on mRNA is generally known in the art and routinely practiced in diagnostic labs world-wide. For example, suitable techniques for mRNA analysis are Northern blot hybridisation and amplification based techniques such as PCR, and especially real time PCR, and NASBA.

According to a particularly preferred embodiment, expression analysis comprises high-throughput DNA array chip analysis not only allowing the simultaneous analysis of multiple samples but also an automatic processing of the data obtained.

According to another preferred embodiment of the present method, determining the expression comprises determining protein levels of CTHRC1 . Suitable techniques are, for example, matrix-assisted laser desorption-ionization time-of-flight mass spectrometer (MALDI-TOF).

In the present description, reference is made to CTHRC1 as biomarker for bladder cancer by referring to its arbitrarily assigned name

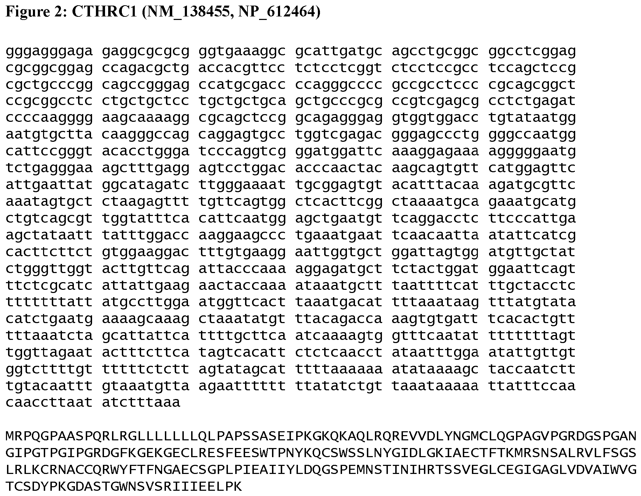

. Although the skilled person is readily capable of identifying and using CTHRC1 as biomarker based on the indicated name the appended figure 2 provides the cDNA and amino acid sequences of CTHRC1 , thereby readily allowing the skilled person to develop expression analysis assays based on analysis techniques commonly known in the art.

Such analysis techniques can, for example, be based on the genomic sequence of the gene or the provided cDNA or amino acid sequences. This sequence information can either be derived from the provided sequences, or can be readily obtained from public databases, for example by using the provided accession numbers.

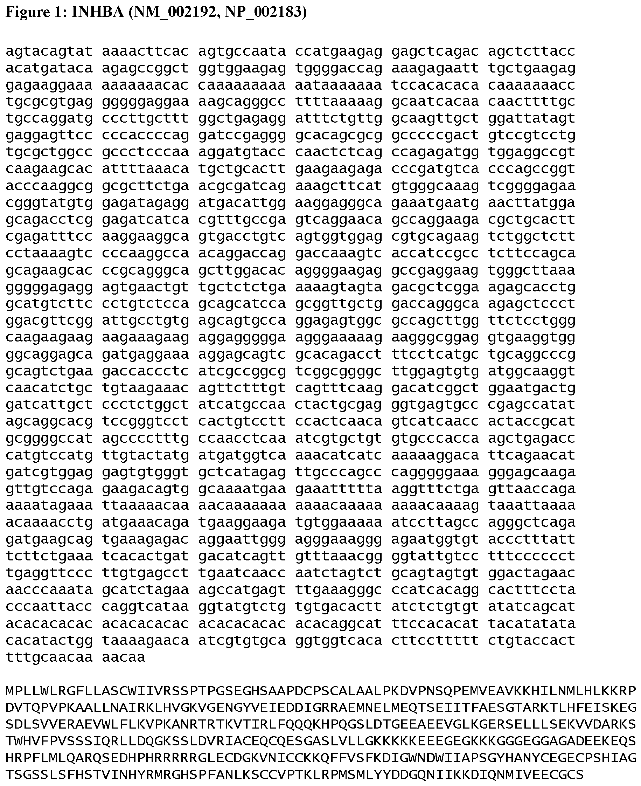

- **Figures 1-18:**: show the cDNA and amino acid sequences of the INHBA gene (NM_002192, NP_002183); the CTHRC1 gene (NM_138455, NP_612464); the CHI3L1 gene (NM_001276, NP_001267); the COL10A1 gene (NM_000493, NP_000484); the FAP gene (NM_004460, NP_004451); the sequence of transcript cluster 2526896 (no assigned mRNA and protein sequences); the ASPN gene (NM_017680, NP_060150); the sequence of transcript cluster 2526893 (no assigned mRNA and protein sequences); the ADAMTS12 gene (NM_030955, NP_112217); the IGF2BP2 gene (NM_006548, NP_006539); the PDCD1LG2 gene (NM_025239, NP_079515); the SFRP4 gene (NM_003014, NP_003005); the KRT6A gene (NM_005554, NP _005545); the TPX2 gene (NM_012112, NP_036244); the CCNB2 gene (NM_004701, NP_004692); the ANLN gene (NM_018685, NP_061155); the FOXM1 gene (NM_202002, NP_973731) and the CDC20 gene (NM_001255, NP_001246), respectively;
- **Figure 19:**: shows boxplots for the identified five best performing individual biomarkers that could distinguish NBl from BCa (NMIBC+MIBC) in tissue;
- **Figure 20:**: shows boxplots for the identified five best performing individual biomarkers that could distinguish MIBC tissue from NMIBC tissue;
- **Figure 21:**: shows boxplots for the identified best performing individual biomarkers for the detection of BCa in urine and/or that could distinguish MIBC from NMIBC in urine;
- **Figure 22:**: shows receiver under Operation Curves (ROC) showing a combination of biomarkers for predicting the occurrence of BCa (NMIBC and MIBC) based on the expression of the markers in urine. The Area Under the Curve (AUC) for the combination of CCNB2+CDC20+PDCD1LG2+INHBA expression in urine is 0.991 (95%CI: 0.977-1.000)
- **Figure 23:**: shows Receiver under Operation Curves (ROC) showing a combination of biomarkers for predicting the occurrence of MIBC based on the expression of the markers in tissue. The Area Under the Curve (AUC) for the combination of IGF2BP2+FAP+CHI3L1+CDC20 expression in tissue is 0.955(95%CI: 0.929-0.980).

### EXAMPLES

### Example 1:

To identify markers for bladder cancer, the gene expression profile (GeneChip^{®} Human Exon 1.0 ST arrays, Affymetrix) of samples from patients with and without bladder cancer were used. The expression analysis was performed according to standard protocols.

Briefly, tissue was obtained after radical cystectomy from patients with bladder cancer. The tissues were snap frozen and cryostat sections were hematoxylin-eosin (H.E.) stained for classification by a pathologist.

Malignant- and and non-malignant areas were dissected and total RNA was extracted with TRIpure^{®} (Roche, Indianapolis, IN, CA, USA) following manufacturer's instructions. Total RNA was purified with the Qiagen RNeasy mini kit (Qiagen, Valencia, CA, USA). The integrity of the RNA was checked by electrophoresis using the Agilent 2100 Bioanalyzer.

From the purified total RNA, 1 µg was used for the GeneChip^{®} Whole Transcript (WT) Sense Target Labeling Assay. (Affymetrix, Santa Clara, CA, USA). Using a random hexamer incorporating a T7 promoter, double-stranded cDNA was synthesized.

Then, cRNA was generated from the double-stranded cDNA template through an *in vitro* transcription reaction and purified using the Affymetrix sample clean-up module. Single-stranded cDNA was regenerated through a random-primed reverse transcription using a dNTP mix containing dUTP. The RNA was hydrolyzed with RNaseH and the cDNA was purified. Subsequently, the cDNA was fragmented by incubation with a mixture of UDG (uracil DNA glycosylase) and APE1 (apurinic/apyrimidinic endonuclease 1) restriction endonucleases and, finally, end-labeled via a terminal transferase reaction incorporating a biotinylated dideoxynucleotide. Of the fragmented, biotinylated cDNA, 5.5 µg was added to a hybridization mixture, loaded on a GeneChip^{®} Human Exon 1.0 ST array and hybridized for 16 hours at 45 °C and 60 rpm.

Using the GeneChip^{®} Human Exon 1.0 ST array, genes are indirectly measured by exon analysis which measurements can be combined into transcript clusters measurements. There are more than 300,000 transcript clusters on the array, of which 90,000 contain more than one exon. Of these 90,000 there are more than 17,000 high confidence (CORE) genes which are used in the default analysis. In total there are more than 5.5 million features per array.

Following hybridization, the array was washed and stained according to the Affymetrix protocol. The stained array was scanned at 532 nm using a GeneChip^{®} Scanner 3000, generating CEL files for each array.

Exon-level and gene level expression values were derived from the CEL file probe-level hybridization intensities using Partek Genomics Suite 6.2, (Partek Incorporated, Saint Louis, MO, USA). Data analysis with this software was performed with the GeneChip^{®} array core meta probe sets as well as the extended meta probe sets.

Differentially expressed genes between conditions, *e.g.* NMIBC *versus* MIBC and MIBC *versus* NBl, are calculated using Anova (ANalysis Of Variance), a T-test for more than two groups. The target identification is biased since clinically well-defined risk groups were analyzed. The markers are categorized based on their role in cancer biology. For the identification of markers the non-muscle invasive bladder cancer (NMIBC) group (N=48), the muscle invasive bladder cancer (MIBC) group (N=49), the bladder cancer metastasis (BC-Meta) group (N=5) and the normal bladder(NBl) group ( N=12)were compared.

Based on the GeneChip^{®} microarrays expression analysis data, the most differentially expressed genes between NBl and NMIBC/MIBC (diagnostic genes) and also the most differentially expressed genes between the NMIBC and MIBC (prognostic genes) were selected.

In total, a group of 46 genes of interest were selected which will be further elucidated in example 2 and listed in **Table 2**. Based on the selected 18 genes in example 2, the GeneChip^{®} expression data for these genes are shown in **Table 1.**
- **Table 1**:: GeneChip^{®} Microarray data showing the *expression characteristics of 18 targets characterizing bladder cancer tissue, based on the analysis of 12 well annotated NBl, 48 NMIBC, 49 MIBC and 5 BC-Meta tissue specimens.*

**Table 1A:**

| Gene symbol | Gene Name | Gene | **up in MIBC vs NMIBC** | | up in MIBC vs NBl | |
|---|---|---|---|---|---|---|
| | | assignment | Fold-Change | P-value | Fold-Change | P-value |
| INHBA | inhibin, beta A | NM_002192 | **10.1** | 3.3E-1 | 5.7 | 1.7E-8 |
| CTHRC1 | collagen triple helix repeat containing 1 | NM_138455 | **4.5** | 5.1E-2 | 2.4 | 7.9E-6 |
| CHI3L1 | chitinasc 3-like 1 (cartilage glycoprotein-39) | NM_001276 | **13.5** | 1.5E-19 | 6.8 | 4.1E-7 |
| COL10A1 | collagen, type X, alpha 1 | NM_000493 | **3.7** | 1.0E-1 | 3.8 | 1.3E-9 |
| FAP | fibroblast activation protein, alpha | NM_004460 | **6.3** | 2.5E-1 | 3.5 | 1.3E-5 |
| TC2526896^{∗} | Transcript cluster 2526896, N/A^{∗∗} | N/A^{∗∗} | **7.6** | 1.1E-1 | 7.8 | 5.6E-9 |
| ASPN | Asporin | NM_017680 | **7.6** | 1.1E-1 | 2.0 | 2.7E-2 |
| TC2526893^{∗} | Transcript cluster 2526893, N/A^{∗∗} | N/A^{∗∗} | **4.2** | 1.2E-1 | 4.2 | 1.8E-7 |
| ADAMTS12 | ADAM metallopeptidase with thrombospondin type 1 motif, 12 | NM_016568 | **4.3** | 8.2E-21 | 3.8 | 1.9E-10 |
| IGF2BP2 | insulin-like growth factor 2 mRNA binding protein 2 | NM_006548 | **4.0** | 1.8E-12 | 2.7 | 2.5E-4 |
| PDCD1LG2 | programmed cell death 1 ligand 2 | NM_025239 | **5.6** | 1.2E-13 | 1.8 | 7.1E-2 |
| SFRP4 | secreted frizzled-related protein 4 | NM_003014 | **6.6** | 1.6E-12 | 2.9 | 3.5E-3 |
| KRT6A | keratin 6A | NM_005554 | **6.1** | 2.3E-07 | 4.6 | 2.6E-3 |

| | | | | | | |
|---|---|---|---|---|---|---|
| ^{∗}data based on the GeneChip^{®} extended meta probesets ^{∗∗} N/A = there are no assigned mRNA sequences for this transcript cluster. | | | | | | |

**Table 1B:**

| Gene symbol | Gene Name | Gene | **up in MIBC vs NBl** | | up in MIBC vs NMIBC | |
|---|---|---|---|---|---|---|
| | | assignment | Fold-Change | P-value | Fold-Change | P-value |
| TPX2 | TPX2, microtubule-associated, homolog (Xenopus laevis) | NM_012112 | **18.4** | 1.6E-19 | 2.6 | 3.9E-07 |
| CCNB2 | cyclin B2 | NM_004701 | **10.0** | 3.6E-19 | 1.6 | 5.6E-04 |
| ANLN | anilin, actin binding protein | NM_018685 | **16.3** | 1.8E-18 | 3.1 | 2.0E-09 |
| FOXM1 | forkhead box M1 | NM_202002 | **8.5** | 5.5E-18 | 2.4 | 2.2E-09 |
| CDC20 | cell devision cycle 20 homolog | NM_001255 | **29.0** | 6.4E-18 | 2.4 | 6.4E-05 |

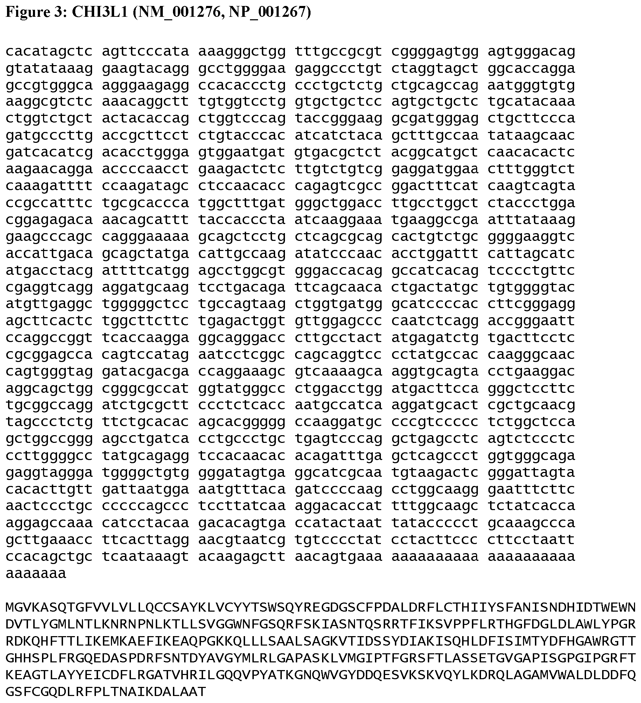

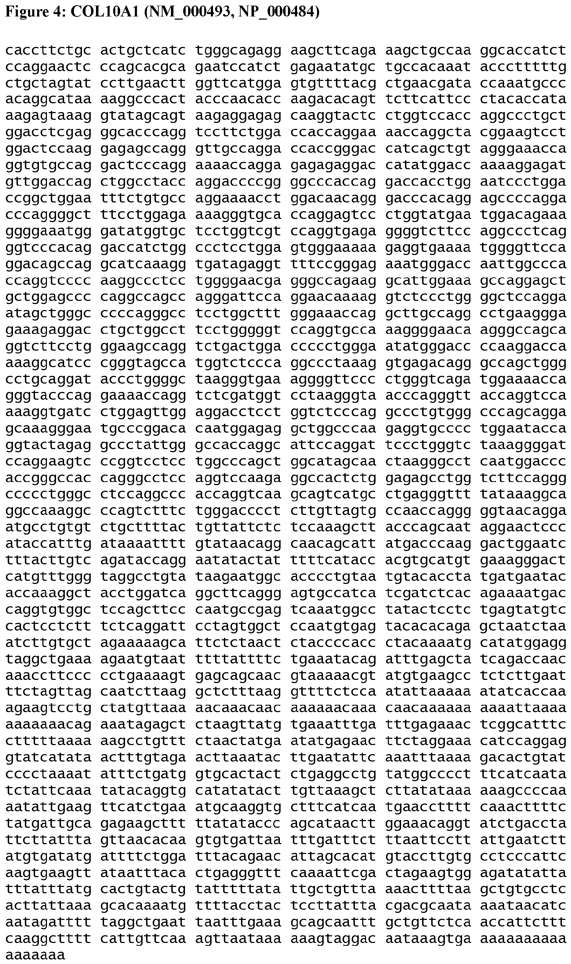

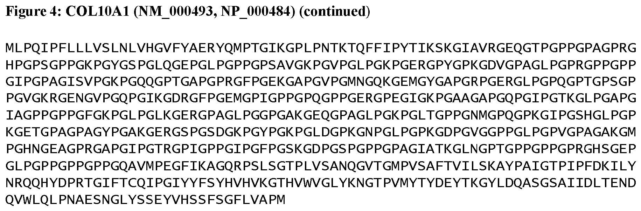

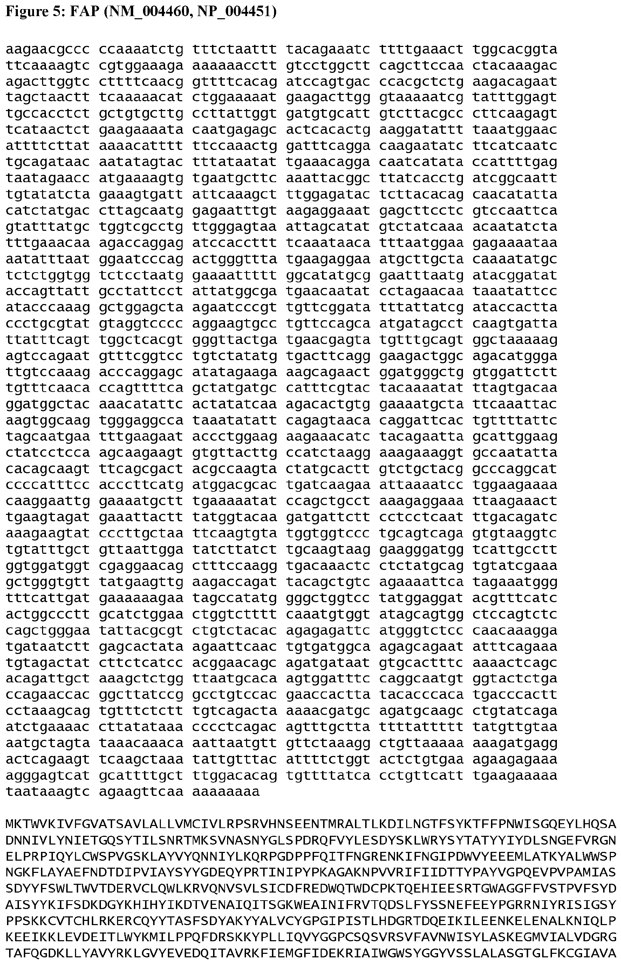

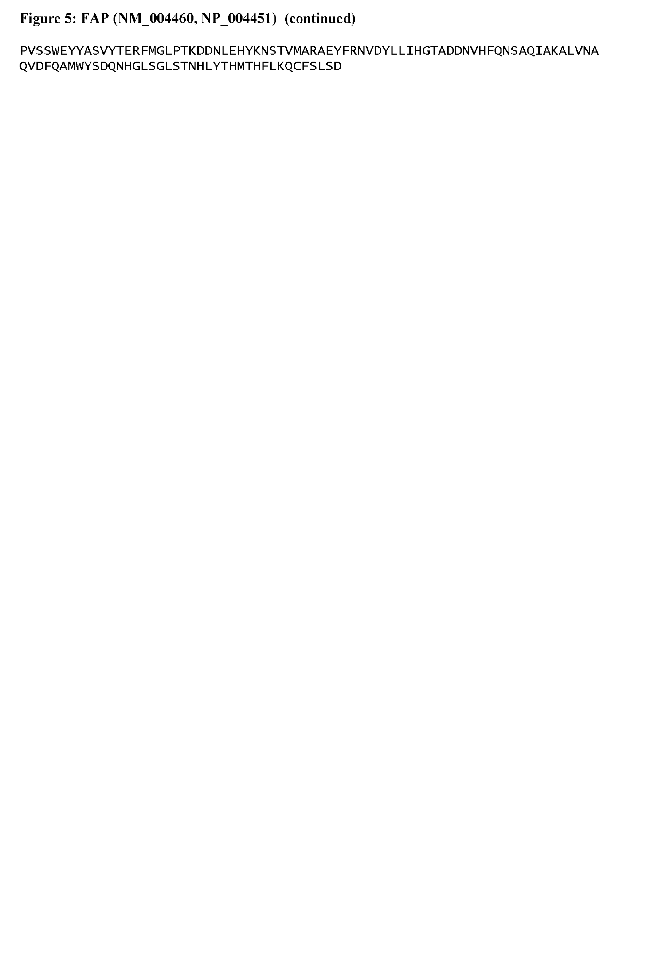

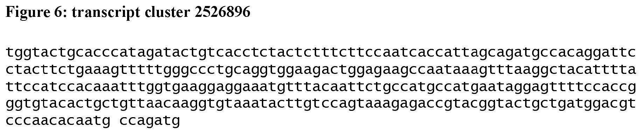

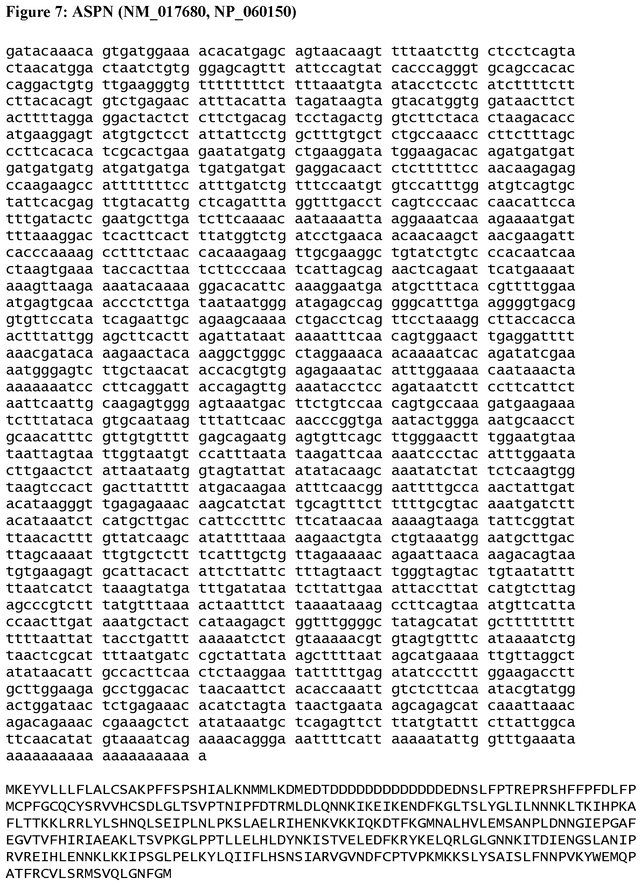

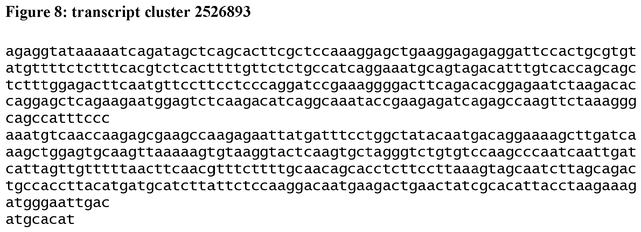

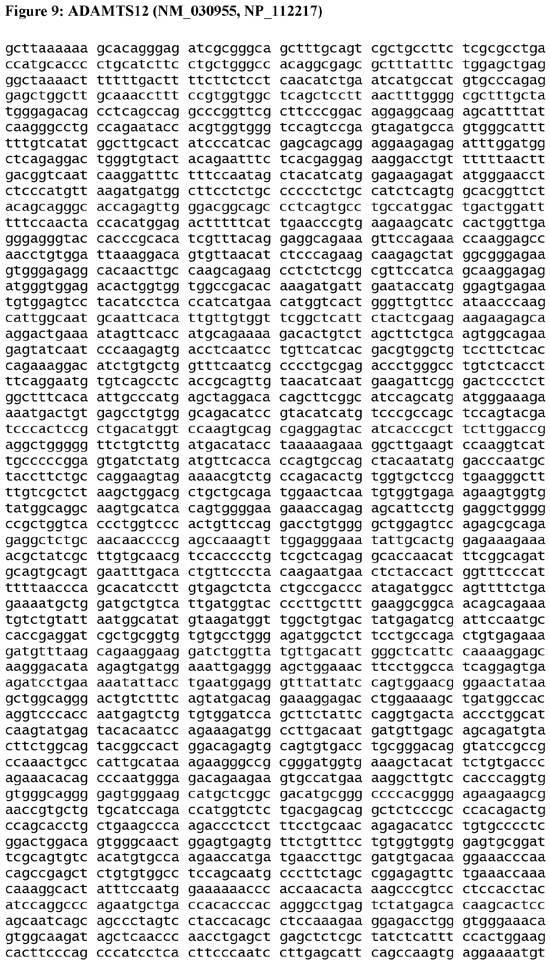

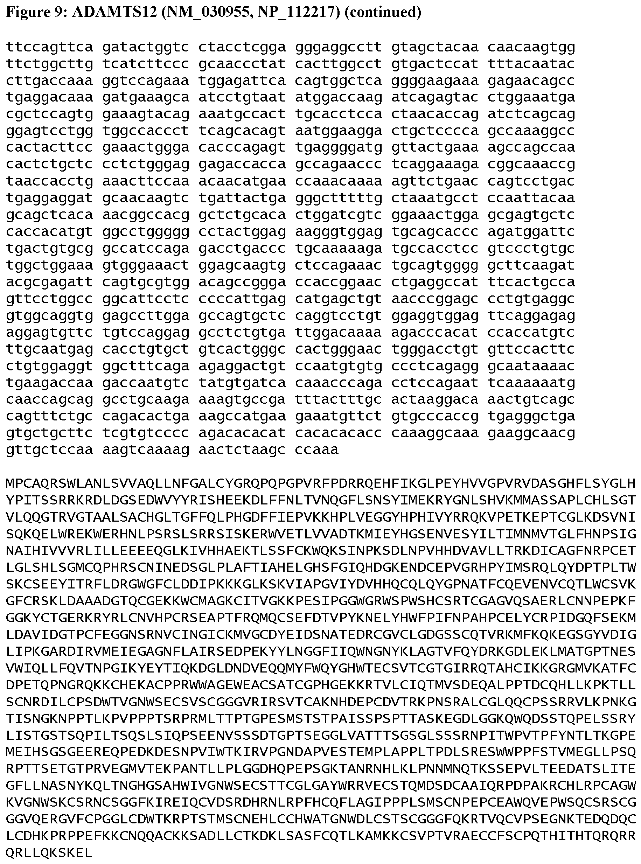

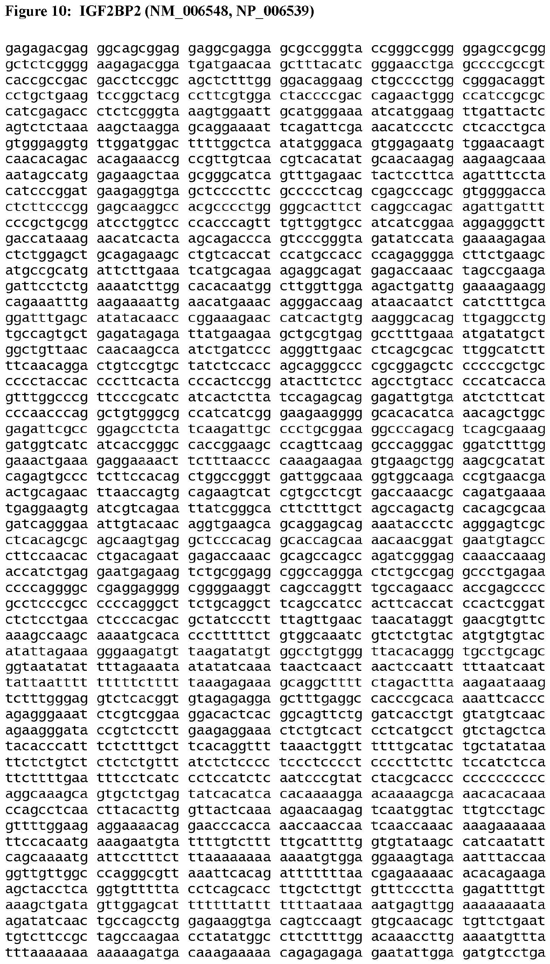

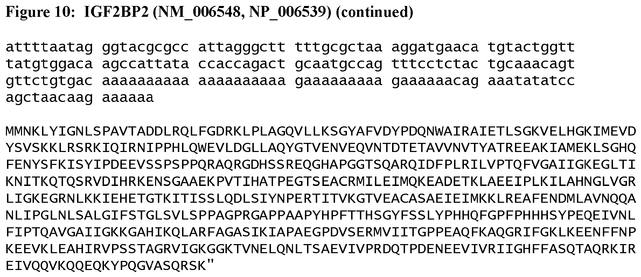

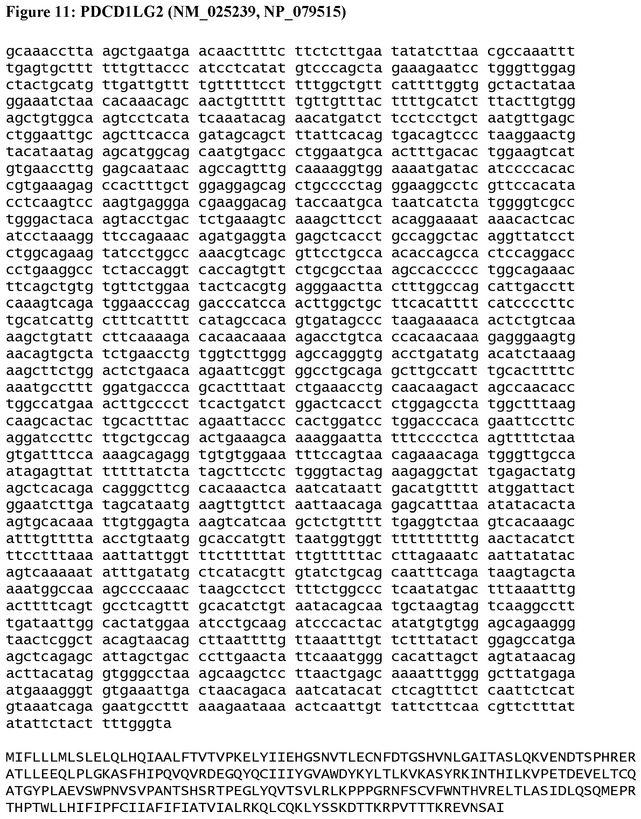

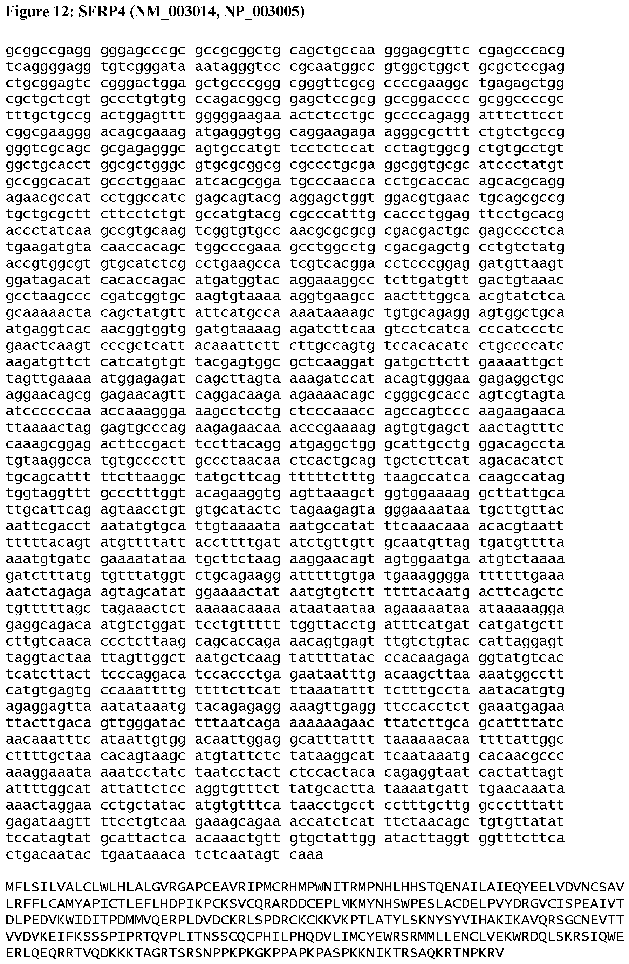

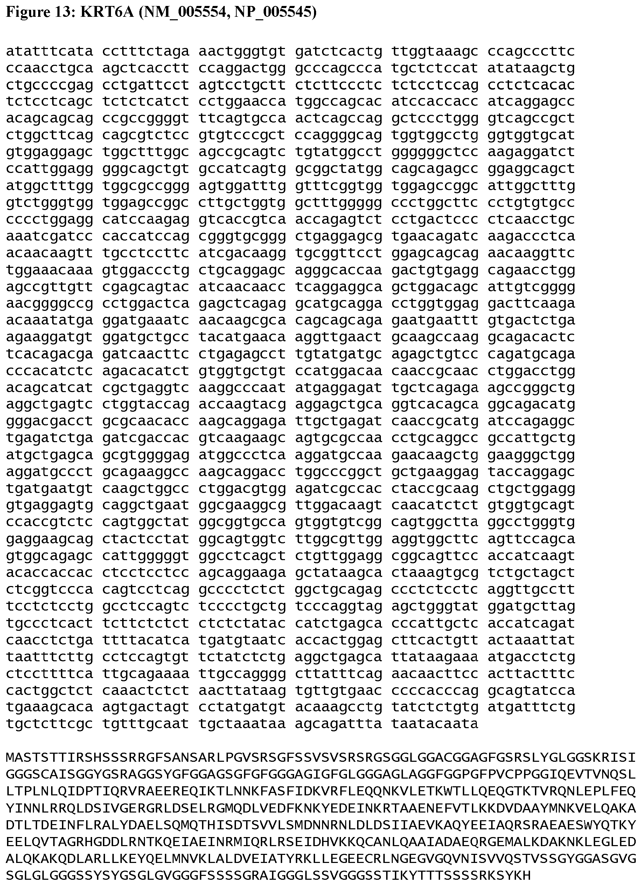

As can be clearly seen in **Table 1A** an up regulation of expression of INHBA (**Figures 1**), CTHRC1 (**Figures 2**), CHI3L1 (**Figures 3**), COL10A1 (**Figures 4**), FAP (**Figures 5**), transcript cluster 2526896 (**Figures 6**), ASPN (**Figures 7**), transcript cluster 2526893(**Figures 8**), ADAMTS12 (**Figures 9**), IGF2BP2 (**Figures 10**), PDCD1LG2 (**Figures 11**), SFRP4 (**Figure 12**), KRT6A (**Figure 13**) was associated with MIBC and as such has prognostic value. Eleven out of thirteen were identified using the core probe sets, two were identified using the extended probe sets and have no assigned mRNA sequence and gene symbol.

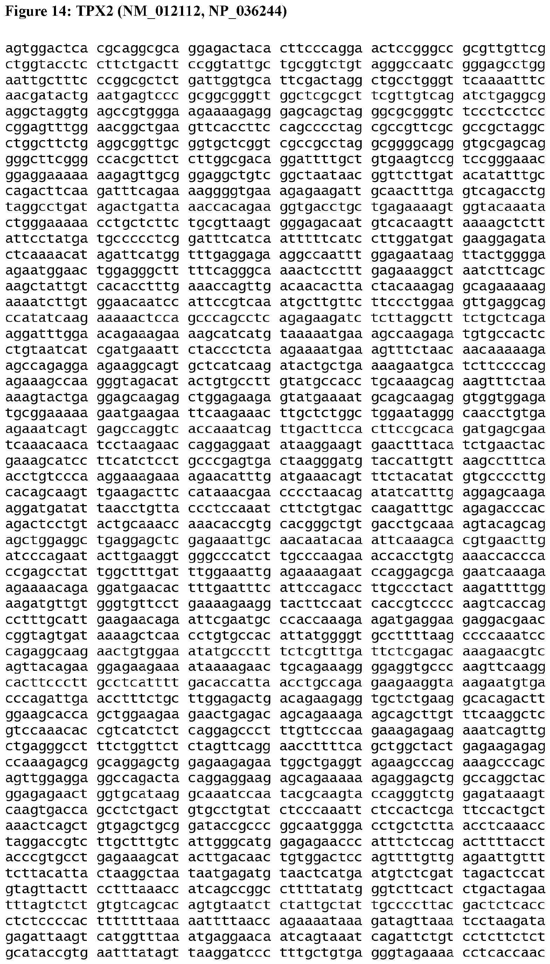

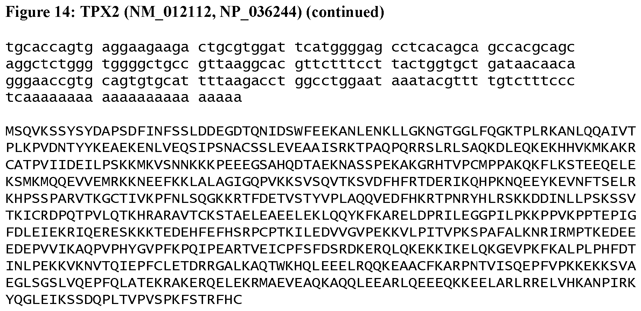

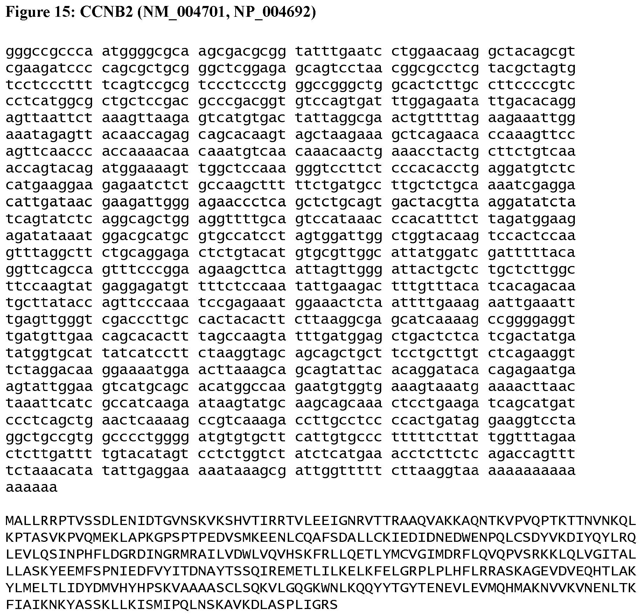

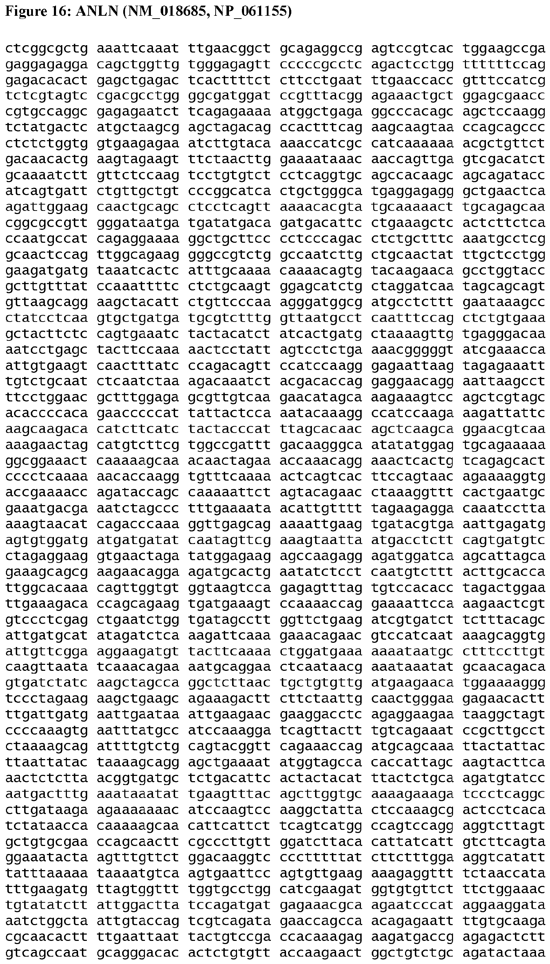

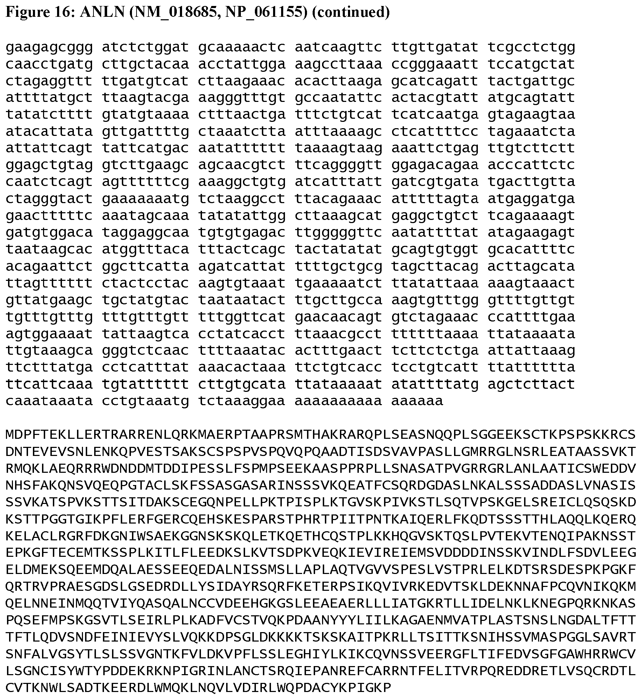

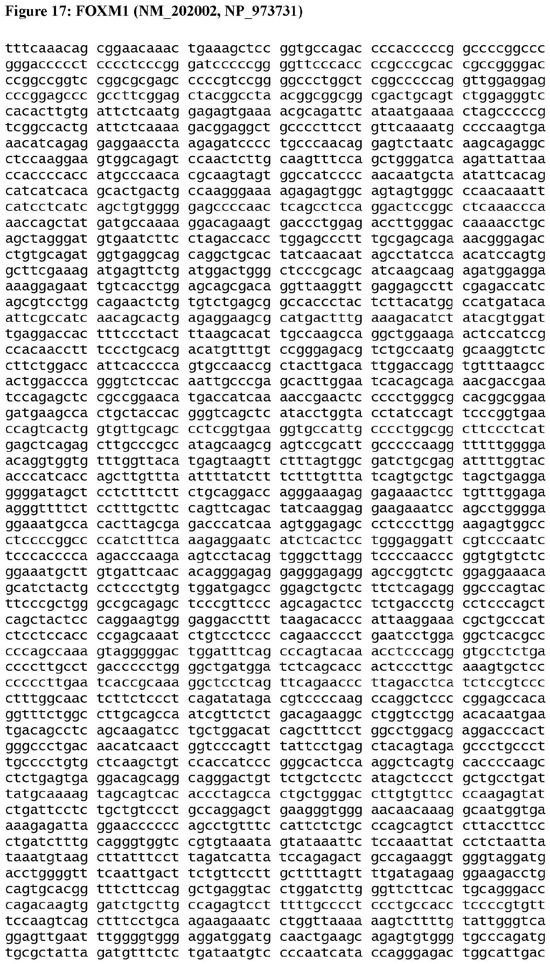

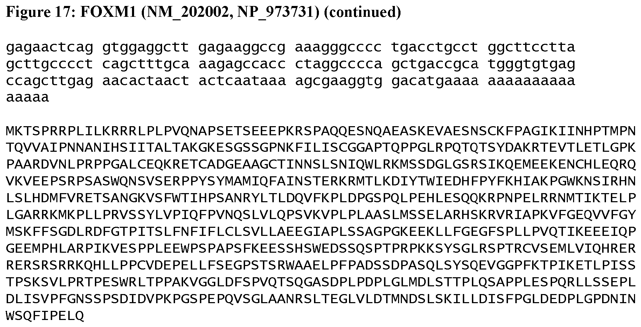

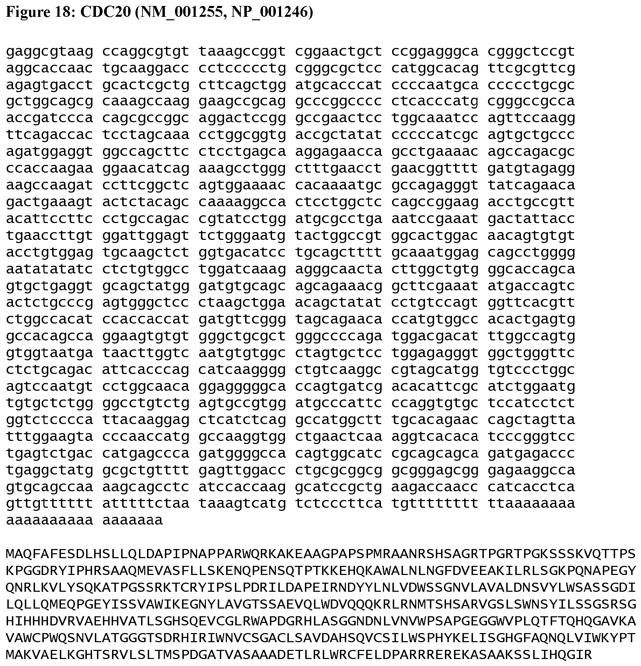

As can be clearly seen in **Table 1B** an up regulation of expression of TPX2 (**Figure 14**), CCNB2 (**Figure 15**), ANLN (**Figure 16**), FOXM1 (**Figure 17**) and CDC20 (**Figure 18**) was associated with the presence bladder cancer and as such has diagnostic value.

### Example 2

Using the gene expression profile (GeneChip^{®} Human Exon 1.0 ST Array, Affymetrix) on 114 tissue specimens of normal bladder(NBl) , non-muscle invasive bladder cancer (NMIBC), muscle invasive bladder cancer (MIBC) and bladder cancer metastasis (BC-Meta) several genes were found to be differentially expressed. The expression levels of 46 of these differentially expressed genes, together with the expression level of a housekeeping gene (GAPDH) and reference gene (TBP) were validated using the TaqMan^{®} Low Density arrays (TLDA, Applied Biosystems). In **Table 2** an overview of the validated genes is shown.

**Table 2: Gene expression assays used for TLDA analysis**

| Gene symbol | Accesion nr. | Assay number |
|---|---|---|
| LOXL2 | NM_002318 | Hs00158757_m1 |
| INHBA | NM_002192 | Hs01081598_m1 |
| ADAMTS12 | NM_030955 | Hs00229594_m1 |
| CTHRC1 | NM_138455 | Hs00298917_m1 |
| SULF1 | NM_001128205 | Hs00290918_m1 |
| CHI3L1 | NM_001276 | Hs00609691_m1 |
| MMP11 | NM_005940 | Hs00968295_m1 |
| OLFML2B | NM_015441 | Hs00295836_m1 |
| CD109 | NM_133493 | Hs00370347_m1 |
| COL10A1 | NM_000493 | Hs00166657_m1 |
| NID2 | NM_007361 | Hs00201233_m1 |
| LOX | NM_002317 | Hs00942480_m1 |
| ADAMTS2 | NM_014244 | Hs01029111_m1 |
| FAP | NM_004460 | Hs00990806_m1 |
| GREM1 | NM_013372 | Hs01879841_s1 |
| WISP1 | NM_003882 | Hs00365573_m1 |
| ITGA11 | NM_001004439 | Hs00201927_m1 |
| ASPN | NM_017680 | Hs00214395_m1 |
| NTM | NM_001144058 | Hs00275411_m1 |
| PRR11 | NM_018304 | Hs00383634_m1 |
| BMP8A | NM_181809 | Hs00257330 s1 |
| SLC12A8 | NM_024628 | Hs00226405_m1 |
| SFRP4 | NM_003014 | Hs00180066_m1 |
| KRT6A | NM_005554 | Hs01699178_g1 |
| PDCD1LG2 | NM_025239 | Hs00228839_m1 |
| BCAT1 | NM_001178094 | Hs00398962_m1 |
| IGF2BP2 | NM_006548 | Hs01118009_m1 |
| TPX2 | NM_012112 | Hs00201616_m1 |
| CCNB2 | NM_004701 | Hs00270424_m1 |
| PLK1 | NM_005030 | Hs00153444_m1 |
| ANLN | NM_018685 | Hs01122612_m1 |
| AURKA | NM_198433 | Hs01582072_m1 |
| FOXM1 | NM_202002 | Hs01073586_m1 |
| CDC20 | NM_001255 | Hs00426680_mH |
| ECT2 | NM_018098 | Hs00216455_m1 |
| PLXNA1 | NM_032242 | Hs00413698_m1 |
| BUB1 | NM_004336 | Hs01557701_m1 |
| CKAP2 | NM_018204 | Hs00217068_m1 |
| TOP2A | NM_001067 | Hs00172214_m1 |
| TTK | NM_003318 | Hs01009870_m1 |
| CYB561D1 | NM_001134404 | Hs00699482_m1 |
| HMGB3 | NM_005342 | Hs00801334_s1 |
| SKP2 | NM_005983 | Hs01021864_m1 |
| FAPP6 | NM_001130958 | Hs01031183_m1 |
| FAM107A | NM_007177 | Hs00200376 m1 |
| NTRK3 | NM_001007156 | Hs00176797_m1 |
| TBP | NM_003194 | Hs00427620_m1 |
| GAPDH | NM_002046 | Hs99999905_m1 |

The validation with TLDA analysis was performed with 66 bladder tissue samples. Among these, 64 samples were newly selected and isolated, 2 normal bladder samples had been used before in the identification step with the GeneChip^{®} Human Exon 1.0 ST Array.

Bladder cancer specimens in the following categories were used: Normal bladder (NB1, n=7), non-muscle invasive bladder cancer (NMIBC, n=29), muscle invasive bladder cancer (MIBC, n=27) and bladder cancer metastasis (BC-Meta, n=3).

To determine whether the identified biomarkers for bladder cancer could be used in a kit for specific detection in urine, 16 urinary sediments from patients suffering from bladder cancer (8 NMIBC and 8 MIBC) were included in the TLDA analysis and validation.

All tissue samples were snap frozen and cryostat sections were stained with hematoxylin and eosin (H.E.). These H.E.-stained sections were classified by a pathologist. Tumor areas were dissected. RNA was extracted from 10 µm thick serial sections that were collected from each tissue specimen at several levels. Tissue was evaluated by HE-staining of sections at each level and verified microscopically. Total RNA was extracted with TRIpure^{®} (Roche, Indianapolis, IN, CA, USA) according to the manufacturer's instructions. Total RNA was purified using the RNeasy mini kit (Qiagen, Valencia, CA, USA).

The 16 urine samples of patients with bladder cancer were immediately cooled to 4°C and were processed within 48h after collection to guarantee good sample quality. The urine, EDTA stabilized, was centrifuged at 4°C and 1.800xg for 10 minutes. The obtained urinary sediment were washed twice with icecold buffered sodium chloride solution. On centrifugation at 4°C and 1.000xg for 10 minutes, the sediments were snap frozen in liquid nitrogen and stored at - 70°C. RNA was extracted from the urinary sediments using a modified TriPure reagent protocol. After the chloroform extraction, GlycoBlue was added to the aquous phase to precipitate the RNA using isopropanol. Total RNA from the sediments was used to generate amplified sense-strand cDNA using the Whole Transcriptome Expression kit according to the manufacturers protocol.

RNA quantity and quality were assessed on a NanoDrop 1000 spectrophotometer (NanoDrop Technologies, Wilmington, DE, USA) and on an Agilent 2100 Bioanalyzer (Agilent Technologies Inc., Santa Clara, CA, USA).

Two µg total RNA was eliminated from genomic DNA and reverse transcribed using the Quantitect^{®} Reverse Transcription Kit Qiagen gMBH, Hilden, D) according to the manufacturer's instructions. Gene expression levels were measured using the TaqMan^{®} Low Density Arrays (TLDA; Applied Biosystems).

A list of assays used in this study is given in **Table 2**. Of the individual cDNAs, 3 µl is added to 50 µl Taqman^{®} Universal Probe Master Mix (Applied Biosystems)and 47 µl milliQ. One hundred µl of each sample was loaded into 1 sample reservoir of a TaqMan^{®} Array (384-Well Micro Fluidic Card) (Applied Biosystems). The TaqMan^{®} Array was centrifuged twice for 1 minute at 280g and sealed to prevent well-to-well contamination. The cards were placed in the micro-fluid card sample block of an 7900 HT Fast Real-Time PCR System (Applied Biosystems). The thermal cycle conditions were: 2 minutes 50°C, 10 minutes at 94.5°C, followed by 40 cycles for 30 seconds at 97°C and 1 minute at 59.7°C.

Raw data were recorded with the Sequence detection System (SDS) software of the instruments. Micro Fluidic Cards were analyzed with RQ documents and the RQ Manager Software for automated data analysis. Delta cycle threshold (Ct) values were determined as the difference between the Ct of each test gene and the Ct of glyceraldehyde 3-phosphate dehydrogenase (GAPDH) (endogenous control gene).

Furthermore, gene expression values were calculated based on the comparative threshold cycle (Ct) method, in which a normal bladder RNA sample was designated as a calibrator to which the other samples were compared.

For the validation of the differentially expressed genes found by the GeneChip^{®} Human Exon 1.0 ST array, 66 bladder tissue specimens and 16 urinary sediments from bladder cancer patients were used in Taqman Low Density Arrays (TLDAs). In these TLDAs, expression levels were determined for the 48 genes of interest. The bladder tissue specimens were put in order from normal bladder, bladder cancer with low to high T-stage and finally bladder cancer metastasis.

Both GeneChip^{®} Human Exon 1.0 ST array and TLDA data were analyzed using scatter- and box plots.

After analysis of the data a list of genes, shown in **Table 3**, was derived the expression of which is indicative for establishing the presence, or absence, of bladder tumour in a human individual suspected of suffering from bladder cancer comprising and, accordingly, indicative for bladder cancer and prognosis thereof.

**Table 3: List of genes identified**

| **Gene Symbol** | **Gene description** | **Figure** |
|---|---|---|
| INHBA | inhibin, beta A | 1 |
| CTHRC1 | collagen triple helix repeat containing 1 | 2 |
| CHI3L1 | chitinase 3-like 1 (cartilage glycoprotein-39) | 3 |
| COL10A1 | collagen, type X, alpha 1 | 4 |
| FAP | fibroblast activation protein, alpha | 5 |
| TC2526896^{∗} | transcript cluster 2526896, N/A^{∗∗} | 6 |
| ASPN | Aspirin | 7 |
| TC2526893^{∗} | transcript cluster 2526893, N/A^{∗∗} | 8 |
| ADAMTS12 | ADAM metallopeptidase with thrombospondin type 1 motif, 12 | 9 |
| IGF2BP2 | insulin-like growth factor 2 mRNA binding protein 2 | 10 |
| PDCD1LG2 | programmed cell death 1 ligand 2 | 11 |
| SFRP4 | secreted frizzled-related protein 4 | 12 |
| KRT6A | keratin 6A | 13 |
| TPX2 | TPX2, microtubule-associated, homolog (Xenopus laevis) | 14 |
| CCNB2 | cyclin B2 | 15 |
| ANLN | anilin, actin binding protein | 16 |
| FOXM1 | forkhead box M1 | 17 |
| CDC20 | cell devision cycle 20 homolog | 18 |

| | | |
|---|---|---|
| ^{∗}data based on the GeneChip^{®} extended meta probesets ^{∗∗} N/A = there are no assigned mRNA sequences for this transcript cluster. | | |

Below detailed GeneChip^{®} Human Exon 1.0 ST array data and TLDA validation data is presented for the 16 genes and only GeneChip^{®} array data for the two transcript clusters, based on the groups normal bladder(NBl), non-muscle invasive bladder cancer (NMIBC), muscle invasive bladder cancer (MIBC) and bladder cancer metastasis (BC-Meta). For the identification of markers the non-muscle invasive bladder cancer (NMIBC) group, the muscle invasive bladder cancer (MIBC) group, the bladder cancer metastasis (BC-Meta) group and the normal bladder(NBl) group were compared.

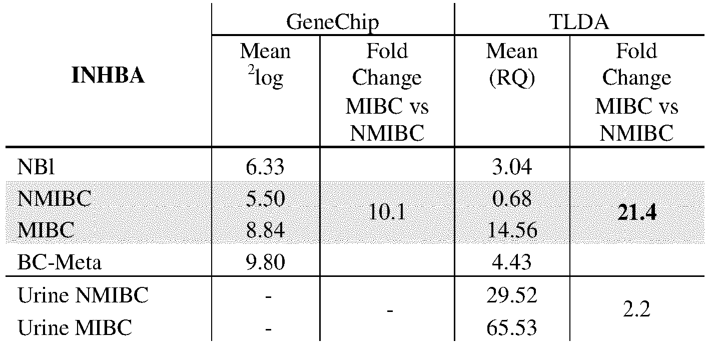

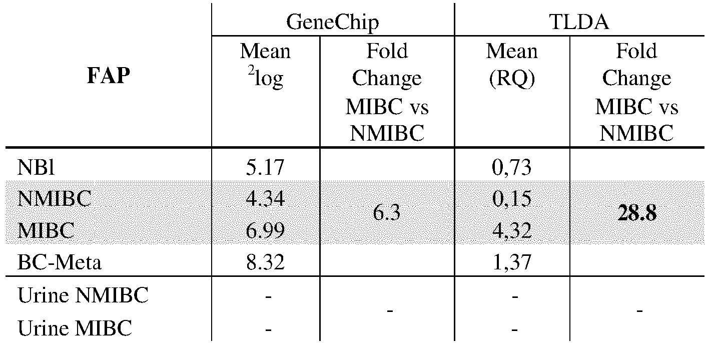

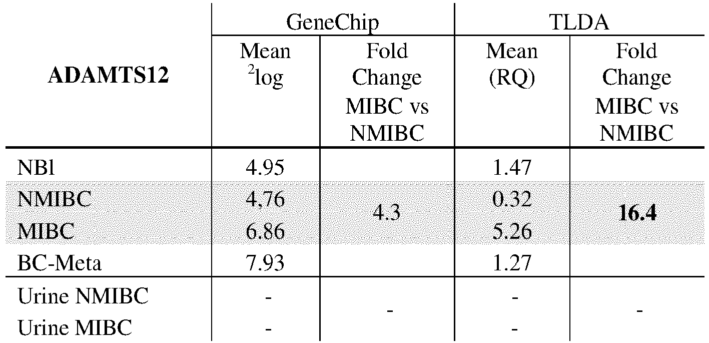

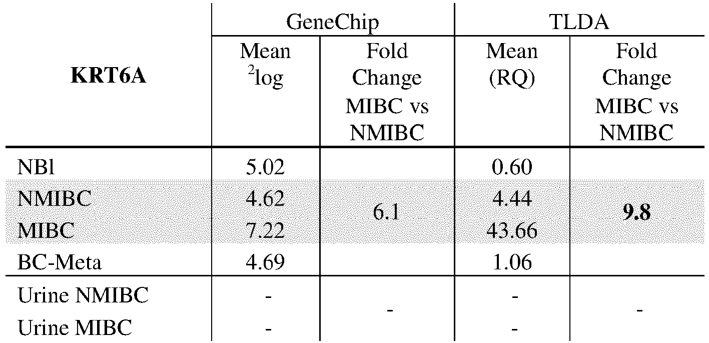

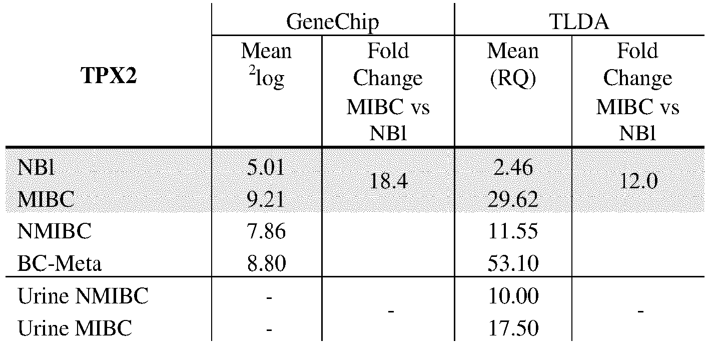

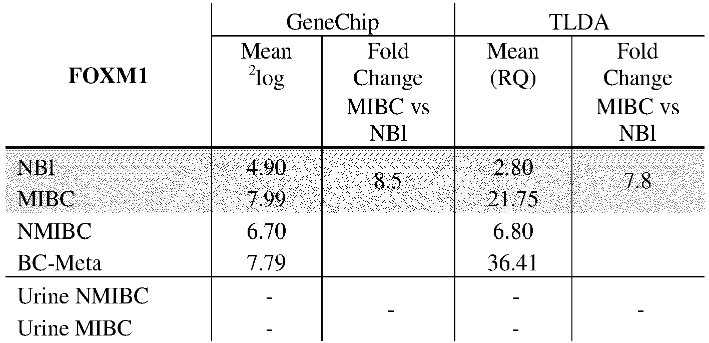

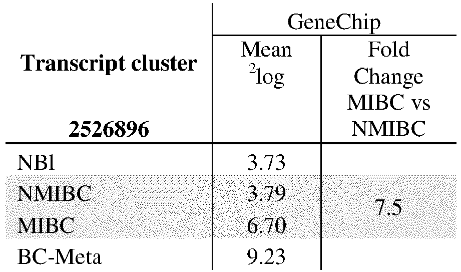

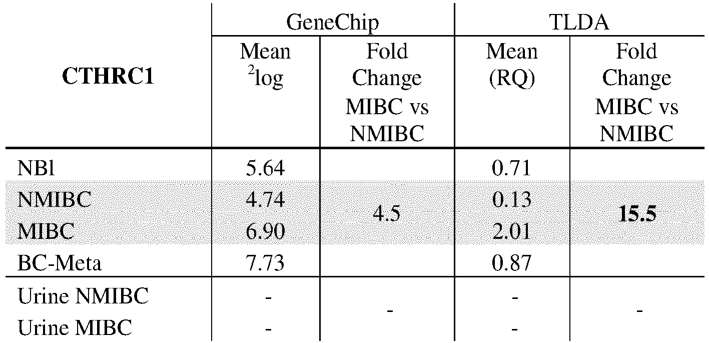

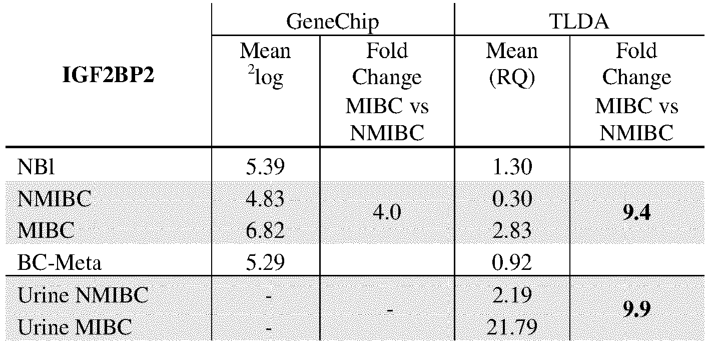

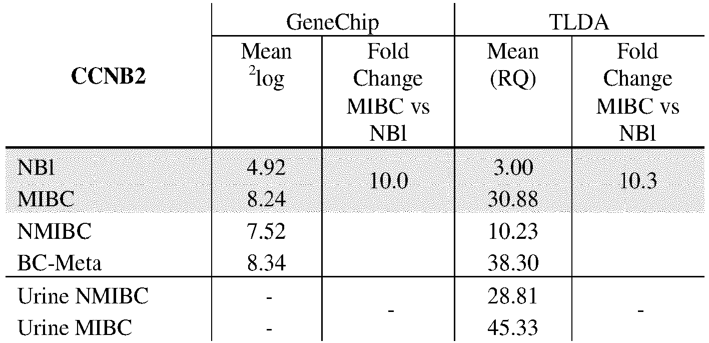

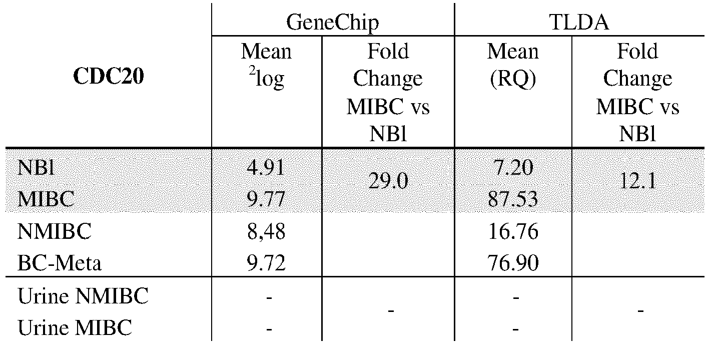

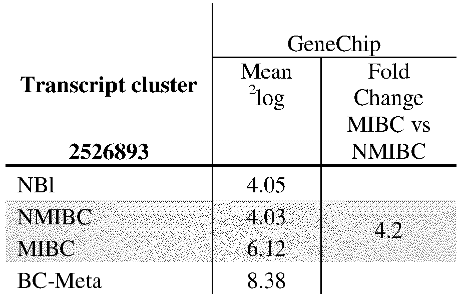

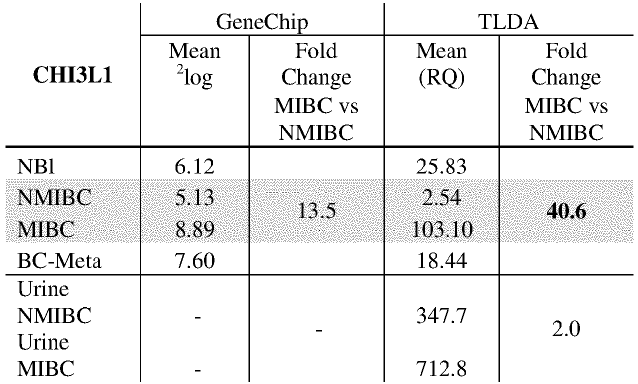

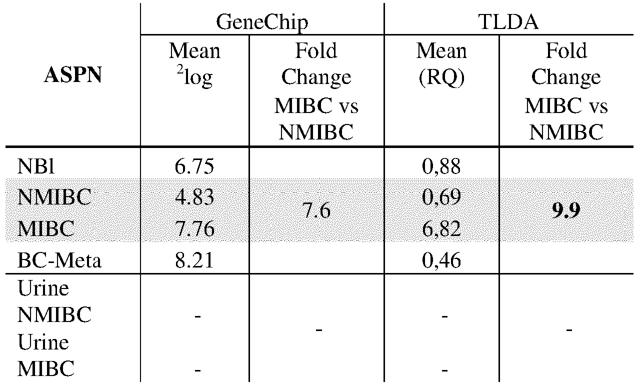

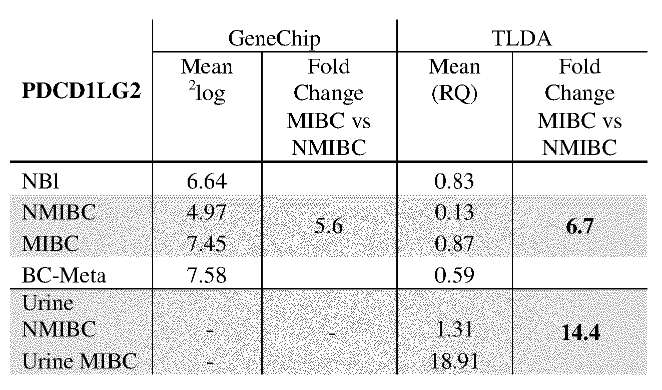

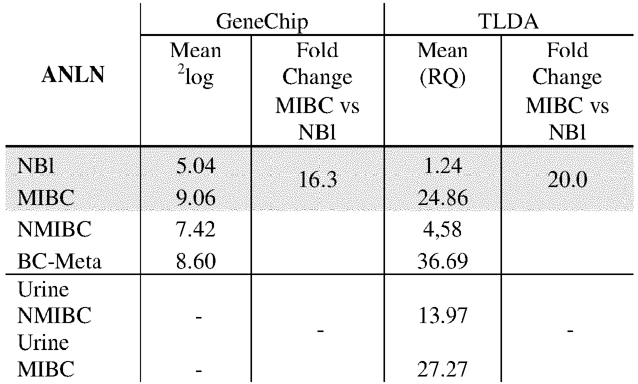

### Example 3:

The identified genes mentioned in example 2 and listed in **Table 3** were used for further validation and selection in a larger cohort of patient samples. For 17 of the 18 identified genes and for the control gene TBP used for normalization, fluorescence based real-time qPCR assays were designed and established according the MIQE guidelines. The performance of transcript clusters 2526896 and 2526893 were very similar. Therefore, no qPCR assay was established for transcript cluster 2526893. PCR products were cloned in either the pCR2.1-TOPO cloning vector (Invitrogen). Calibration curves with a wide linear dynamic range (10 - 1,000,000 copies) were generated using serial dilutions of the plasmids. The amplification efficiency of the primer pair was determined using the calibration curve and was > 1.85. Control samples with known template concentrations were used as a reference. Two µl of each cDNA sample were amplified in a 20 µl PCR reaction containing optimized amounts of forward primer and reverse primer, 2 pmol of hydrolysis probe and 1x Probes Master mix (Roche, Cat No. 04902343001). The following amplification conditions were used: 95°C for 10 minutes followed by 50 cycles at 95°C for 10 seconds, 60°C for 30 seconds and a final cooling step at 40°C for 55 seconds (LightCycler LC480, Roche). The crossing point (Cp) values were determined using the Lightcycler 480 SW 1.5 software (Roche). The Cp values of the samples were converted to concentrations by interpolation in the generated calibration curve. The assay performance of the real-time PCR experiments was evaluated during in-study validation. The reference control samples had an inter- and intra-assay variation < 30%.

Total RNA was extracted from bladder tissue and urinary sediments and used for reverse transcription to generate cDNA. In total 211 bladder tissue specimen and 100 urinary sediments were used. The group of 206 bladder tissue specimen consisted of 10 normal bladders, 124 NMIBC, 72 MIBC. The group of 100 urinary sediments consisted of urinary sediments from 15 healthy controls (defined as normal) , and from 65 patients with NMIBC, and 18 patients with MIBC.

Statistical analyses were performed with SPSS^{®} version 20.0. All data were log-transformed prior to statistical analysis as a transformation to a normal distribution. Two-tailed P values of 0.05 or less were considered to indicate statistical significance. The nonparametric Mann Whitney test (for continuous variables) was used to test if biomarker levels were significantly correlated with the presence of BCa and/or BCa prognosis (muscle invasiveness, metastasis).

The assay results for the 17 selected biomarkers are shown in **Tables 4-7.**

**Table 4: Absolute and relative expression of the 17 biomarkers in NBl and BCa tissue**

| | copy numbers | | | copy numbers | | | relative expression¹ | | | |
|---|---|---|---|---|---|---|---|---|---|---|
| | NBl | | | BCa (NMIBC+MIBC) | | | NBl | BCa | Fold Change | P-value |
| | N=10 | | | N=196 | | | N=10 | N=196 | | |
| Biomarker | Mean | Median | Range | Mean | Median | Range | Mean | Mean | | MW² |
| CTHRC1 | 1041 | 1263 | 1-1810 | 3129 | 835 | 1-42200 | 917.8 | 1891.7 | 2.1 | 0.29 |
| IGF2BP2 | 203 | 130 | 69-651 | 544 | 85 | 0-5750 | 182.1 | 248.8 | 1.4 | 0.74 |
| ADAMTS12 | 91 | 74 | 24-259 | 472 | 103 | 1-4180 | 75.5 | 275.8 | 3.7 | 0.31 |
| INHBA | 411 | 437 | 30-869 | 3472 | 386 | 1-61500 | 400.5 | 1895.9 | 4.7 | 0.38 |
| SFRP4 | 231 | 92 | 55-896 | 1365 | 26 | 1-37300 | 201.7 | 911.5 | 4.5 | 0.18 |
| FAP | 568 | 403 | 146-1440 | 1239 | 270 | 1-12500 | 469.2 | 738.0 | 1.6 | 0.52 |
| CHI3L1 | 81 | 52 | 1-339 | 1468 | 158 | 1-21700 | 104.5 | 883.2 | 8.5 | 0.45 |
| COL10A1 | 275 | 205 | 58-585 | 2991 | 984 | 1-88200 | 225.6 | 1881.5 | 8.3 | 0.56 |
| ASPN | 548 | 205 | 23-3500 | 608 | 210 | 1-15700 | 397.8 | 372.0 | -1.1 | 0.8 |
| ANLN | 92 | 35 | 1-412 | 1297 | 772 | 17-8000 | 56.9 | 521.8 | 9.2 | <0.05 |
| TPX2 | 73 | 28 | 1-325 | 1768 | 964 | 1-13000 | 45.8 | 715.0 | 15.6 | <0.05 |
| FOXM1 | 57 | 16 | 1-251 | 806 | 397 | 1-5650 | 32.9 | 271.3 | 8.2 | <0.05 |
| CCNB2 | 156 | 47 | 1-669 | 2160 | 1485 | 1-11100 | 93.8 | 811.1 | 8.6 | <0.05 |
| CDC20 | 185 | 54 | 1-795 | 2727 | 1650 | 1-22000 | 122.0 | 1057.5 | 8.7 | <0.05 |
| KRT6A | 1983 | 26 | 1-19500 | 32220 | 132 | 1-1470000 | 1386.7 | 12483.6 | 9.0 | 0.48 |
| PDCD1LG2 | 288 | 223 | 119-536 | 570 | 336 | 1-3440 | 262.1 | 298.0 | 1.1 | 0.14 |
| TC2526896 | 3 | 1 | 1-11 | 110 | 1 | 1-1920 | 1.9 | 63.8 | 33.6 | 0.67 |
| TBP | 1230 | 1058 | 492-2820 | 2890 | 2585 | 77-9320 | - | - | - | - |

| | | | | | | | | | | |
|---|---|---|---|---|---|---|---|---|---|---|
| Relative expression¹: ratio (copy numbers biomarker/copy number TBP)^{∗}1000 MW²: Mann-Whitney test | | | | | | | | | | |

**Table 5: Absolute and relative expression of the 17 biomarkers in NMIBC and MIBC tissue**

| | copy numbers | | | copy numbers | | | | relative expression¹ | | | |
|---|---|---|---|---|---|---|---|---|---|---|---|
| | NMIBC | | | MIBC | | | NMIBC | MIBC | Fold Change | P-value | P-value |
| | N=124 | | | N=72 | | | N=124 | N=72 | | | |
| Biomarker | Mean | Median | Range | Mean | Median | Range | Mean | Mean | | MW² | T-test |
| CTHRC1 | 898 | 585 | 24-5360 | 6972 | 4625 | 1-42200 | 364.6 | 4521.7 | 12.4 | <0.05 | 3.5E-20 |
| IGF2BP2 | 206 | 38 | 0-1950 | 1126 | 611 | 1-5750 | 74.2 | 549.4 | 7.4 | <0.05 | 1.0E-20 |
| ADAMTS12 | 124 | 65 | 1-1880 | 1070 | 515 | 1-4180 | 48.2 | 667.9 | 13.9 | <0.05 | 1.4E-19 |
| INHBA | 529 | 230 | 1-7440 | 8540 | 3775 | 29-61500 | 183.0 | 4846.0 | 26.5 | <0.05 | 1.1E-22 |
| SFRP4 | 76 | 1 | 1-2300 | 3567 | 509 | 1-37300 | 41.6 | 2367.6 | 56.9 | <0.05 | 3.3E-23 |
| FAP | 323 | 167 | 1-2510 | 2817 | 1915 | 39-12500 | 135.2 | 1776.3 | 13.1 | <0.05 | 9.6E-34 |
| CHI3L1 | 475 | 37 | 1-21700 | 3177 | 1420 | 15-14800 | 170.6 | 2110.5 | 12.4 | <0.05 | 2.6E-30 |
| COL10A1 | 1087 | 804 | 1-4670 | 6271 | 2040 | 1-88200 | 337.4 | 4540.6 | 13.5 | <0.05 | 2.3E-10 |
| ASPN | 223 | 134 | 1-1450 | 1271 | 551 | 1-15700 | 105.7 | 830.6 | 7.9 | <0.05 | 2.2E-18 |
| ANLN | 1001 | 582 | 17-6930 | 1806 | 1380 | 25-8000 | 318.5 | 871.9 | 2.7 | <0.05 | 2.6E-16 |
| TPX2 | 1358 | 579 | 1-8760 | 2474 | 1620 | 1-13000 | 430.5 | 1205.1 | 2.8 | <0.05 | 3.2E-10 |
| FOXM1 | 723 | 336 | 1-5650 | 948 | 515 | 1-3660 | 196.1 | 400.8 | 2.0 | <0.05 | 4.6E-07 |
| CCNB2 | 2075 | 1275 | 17-11100 | 2306 | 1575 | 1-7360 | 637.3 | 1110.4 | 1.7 | <0.05 | 6.2E-07 |
| CDC20 | 2062 | 1210 | 1-15300 | 3873 | 2445 | 1-22000 | 642.0 | 1773.0 | 2.8 | <0.05 | 3.7E-10 |
| KRT6A | 2257 | 71 | 1-181000 | 83822 | 2320 | 1-1470000 | 721.9 | 32739.8 | 45.4 | <0.05 | 1.3E-11 |
| PDCD1LG2 | 477 | 292 | 1-3200 | 728 | 413 | 1-3440 | 185.4 | 492.0 | 2.7 | <0.05 | 6.3E-10 |
| TC2526896 | 14 | 1 | 1-606 | 276 | 70 | 1-1920 | 4.4 | 166.7 | 37.9 | <0.05 | 1.1E-16 |
| TBP | 3354 | 3250 | 129-9320 | 2090 | 1675 | 77-9260 | - | - | - | - | - |

| | | | | | | | | | | | |
|---|---|---|---|---|---|---|---|---|---|---|---|
| Relative expression¹: ratio (copy numbers biomarker/copy number TBP)^{∗}1000 MW²: Mann-Whitney test | | | | | | | | | | | |

**Table 6: Expression levels of the 17 biomarkers in normal bladder and BCa urine samples**

| | copy numbers | | | copy numbers | | | Fold Change | P-value |
|---|---|---|---|---|---|---|---|---|
| | NBl | | | BCa (NMIB+MIBC) | | | | |
| | N=15 | | | N=85 | | | | |
| Biomarker | Mean | Median | Range | Mean | Median | Range | | MW¹ |
| CTHRC1 | 1764 | 1030 | 58-5140 | 17901 | 10200 | 12-190000 | 10.1 | <0.005 |
| IGF2BP2 | 9994 | 6160 | 94-34600 | 135158 | 36700 | 1040-4000000 | 13.5 | <0.005 |
| ADAMTS12 | 1 | 1 | 1-1 | 110 | 1 | 1-1490 | 110.0 | 0.037 |
| INHBA | 10487 | 4220 | 1-70300 | 373104 | 89400 | 64-6070000 | 35.6 | <0.005 |
| SFRP4 | 24 | 1 | 1-210 | 181 | 50 | 1-1520 | 7.5 | 0.007 |
| FAP | 8 | 1 | 1-106 | 758 | 107 | 1-21000 | 94.8 | <0.005 |
| CHI3L1 | 53324 | 24600 | 2220-368000 | 754615 | 234000 | 2900-5380000 | 14.2 | <0.005 |
| COL10A1 | 1296 | 160 | 1-5630 | 7700 | 6130 | 1-43300 | 5.9 | <0.005 |
| ASPN | 122 | 1 | 1-632 | 75 | 1 | 1-1000 | 0.6 | 0.881 |
| ANLN | 2283 | 832 | 1-10700 | 37092 | 13600 | 25-454000 | 16.2 | <0.005 |
| TPX2 | 946 | 521 | 1-3120 | 28382 | 8860 | 1-310000 | 30.0 | <0.005 |
| FOXM1 | 231 | 209 | 1-649 | 12502 | 4000 | 16-165000 | 54.1 | <0.005 |
| CCNB2 | 1951 | 1290 | 12-10000 | 30857 | 16000 | 1120-240000 | 15.8 | <0.005 |
| CDC20 | 3490 | 2600 | 189-11100 | 40674 | 15800 | 280-61200 | 11.7 | <0.005 |
| KRT6A | 140105 | 70000 | 1230-762000 | 70419 | 27200 | 13-576000 | 0.5 | 0.362 |
| PDCD1LG2 | 1869 | 877 | 1-8650 | 52224 | 16300 | 1-679000 | 27.9 | <0.005 |
| TC2526896 | 2876 | 2840 | 557-6590 | 4356 | 3100 | 73-28600 | 1.5 | 0.382 |
| TBP | 29783 | 21100 | 1160-94400 | 199980 | 161000 | 6000-950000 | - | |

**Table 7: Expression levels of the 17 biomarkers in NMIBC and MIBC urine samples**

| | copy numbers | | | copy numbers | | | Fold Change | P-value |
|---|---|---|---|---|---|---|---|---|
| | NMIBC | | | MIBC | | | | |
| | N=66 | | | N=19 | | | | |
| Biomarker | Mean | Median | Range | Mean | Median | Range | | MW¹ |
| CTHRC1 | 13991 | 9860 | 12-76100 | 31483 | 11400 | 1190-190000 | 2.3 | 0.143 |
| IGF2BP2 | 71976 | 31500 | 1040-1740000 | 365297 | 85250 | 2290-4000000 | 5.1 | 0.087 |
| ADAMTS12 | 71 | 1 | 1-790 | 253 | 1 | 1-1490 | 3.6 | 0.070 |
| INHBA | 288446 | 79500 | 64-3420000 | 667174 | 101000 | 13200-6070000 | 2.3 | 0.451 |
| SFRP4 | 153 | 13 | 1-919 | 276 | 123 | 1-1520 | 1.8 | 0.085 |
| FAP | 427 | 100 | 1-8390 | 1905 | 352 | 1-21000 | 4.5 | 0.023 |
| CHI3L1 | 689779 | 204000 | 2900-5050000 | 979837 | 394000 | 28300-5380000 | 1.4 | 0.117 |
| COL10A1 | 7596 | 6170 | 1-43300 | 8060 | 6130 | 409-28200 | 1.1 | 0.587 |
| ASPN | 54 | 1 | 1-436 | 151 | 1 | 1-1000 | 2.8 | 0.203 |
| ANLN | 31291 | 12450 | 25-454000 | 57243 | 21300 | 350-273000 | 1.8 | 0.083 |
| TPX2 | 16665 | 7180 | 1-188000 | 69081 | 14200 | 232-310000 | 4.1 | 0.017 |
| FOXM1 | 7218 | 3465 | 16-76800 | 30299 | 10600 | 101-156000 | 4.2 | 0.008 |
| CCNB2 | 22387 | 14900 | 1120-161000 | 60280 | 27300 | 1130-240000 | 2.7 | 0.014 |
| CDC20 | 22898 | 12700 | 396-184000 | 102422 | 23700 | 280-612000 | 4.5 | 0.013 |
| KRT6A | 6226 | 27650 | 166-500000 | 99574 | 26200 | 13-576000 | 16.0 | 0.780 |
| PDCD1LG2 | 38805 | 16200 | 1-679000 | 98840 | 22600 | 1900-477000 | 2.5 | 0.207 |
| TC2526896 | 4294 | 3185 | 73-27300 | 4571 | 2810 | 274-28600 | 1.1 | 0.609 |
| TBP | 189411 | 167000 | 6000-596000 | 236691 | 142000 | 9730-950000 | 1.2 | |

| | | | | | | | | |
|---|---|---|---|---|---|---|---|---|
| MW¹: Mann-Whitney test | | | | | | | | |

In **Table 4** the expression data of the 17 selected biomarkers in tissue are shown for the groups NBl and BCa total (NMIBC+MIBC). The difference (Fold-Change) between the groups and P-value provide information about/determine the diagnostic performance of the markers. In **Table 5** the data in tissue are shown for the groups NMIBC and MIBC and thereby provide information about the prognostic performance of the biomarkers. In **Tables 6 and 7** the data in the urine samples are shown.

### Summary results examples 1, 2 and 3

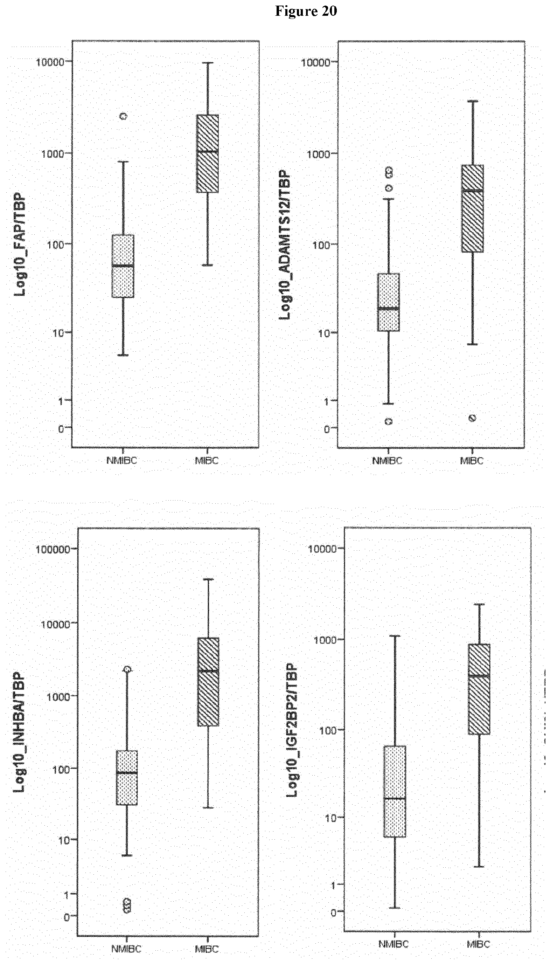

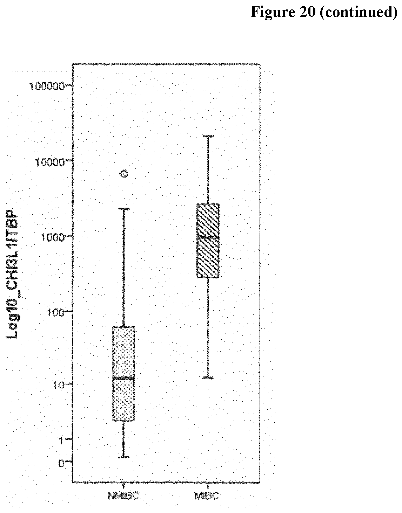

INHBA (**Figures 1****,** **20****; Tables 1, 4-7**): The present GeneChip^{®} Human Exon 1.0 ST Array data, TLDA validation and qPCR assay data showed that INHBA was highly and significantly up-regulated in tissue from MIBC and BC-meta compared to NMIBC tissue. INHBA could also be detected in urine and was highly and significantly up-regulated in urine from BCa patients vs. normal urine. Therefore, INHBA has prognostic value in tissue of patients with BCa and diagnostic value in the detection of BCa in urine.

CTHRC1 (**Figure 2****, Tables 1, 4-7**): The present GeneChip^{®} Human Exon 1.0 ST Array data, TLDA validation and qPCR assay data showed that CTHRC1 was highly and significantly up-regulated in tissue from MIBC and BC-meta compared to NMIBC. CTHRC1 could also be detected in urine and was significantly and highly up-regulated in urine from BCa patients vs. normal urine. Therefore, CTHRC1 has prognostic value in tissue of patients with BCa and diagnostic value in the detection of BCa in urine.

CHI3L1 (**Figures 3****,** **20****: Tables 1, 4-7**): The present GeneChip^{®} Human Exon 1.0 ST Array data, TLDA validation and qPCR assay data showed that CHI3L1 was highly and significantly up-regulated in tissue from MIBC compared to NMIBC. CHI3L1 could also be detected in urine and was significantly and highly up-regulated in urine from BCa patients vs. normal urine. Therefore, CHI3L1 has prognostic value in tissue of patients with BCa and diagnostic value in the detection of BCa in urine.

COL10A1 (**Figures 4****, Tables 1, 4-7**): The present GeneChip^{®} Human Exon 1.0 ST Array data, TLDA validation and qPCR assay data showed that COL10A1 was highly and significantly up-regulated in MIBC and BC-meta compared to NMIBC. COL10A1 could also be detected in urine and was significantly up-regulated in urine from BCa patients vs. normal urine. Therefore, COL10A1 has prognostic value in tissue of patients with BCa and diagnostic value in the detection of BCa in urine.

FAP (**Figures 5****,** **20****,** **21****; Tables 1, 4-7**): The present GeneChip^{®} Human Exon 1.0 ST Array data, TLDA validation and qPCR assay data showed that FAP was highly and significantly up-regulated in tissue from MIBC and BC-meta compared to NMIBC. FAP could also be detected in urine and was significantly and highly up-regulated in urine from BCa patients vs. normal urine and significantly up-regulated in urine from MIBC patients vs. NMIBC patients. Therefore, FAP has prognostic value in urine and in tissue of patients with BCa and diagnostic value in the detection of BCa in urine.

Transcript cluster 2526896 (**Figures 6****, Tables 1, 4-7**): The present GeneChip^{®} Human Exon 1.0 ST Array data, and qPCR assay data showed that transcript cluster TC2526896 was highly and significantly up-regulated in tissue from MIBC and BC-meta compared to NMIBC. Therefore, transcript cluster 2526896 has prognostic value in tissue of patients with BCa.

ASPN (**Figure 7****, Tables 1, 4-7**): The present GeneChip^{®} Human Exon 1.0 ST Array data, TLDA validation and qPCR assay data showed that ASPN was highly and significantly up-regulated in tissue from MIBC compared to NMIBC. Therefore, ASPN has prognostic value in tissue of patients with BCa.

Transcript cluster 2526893 (**Figure 8**): The present GeneChip^{®} Human Exon 1.0 ST Array data showed that transcript cluster 2526893 was highly up-regulated in tissue from MIBC and BC-meta compared to NMIBC. Therefore, transcript cluster 2526893 has prognostic value in tissue of patients with BCa.

ADAMTS12 (**Figures 9****,** **20****; Tables 1, 4-7**): The present GeneChip^{®} Human Exon 1.0 ST Array data, TLDA validation and qPCR assay data showed that ADAMTS12 was highly and significantly up-regulated in tissue from MIBC and BC-meta compared to NMIBC. Low copy numbers of ADAMTS12 could also be detected in urine. ADAMTS12 was significantly up-regulated in urine from BCa patients vs. normal urine and significantly up-regulated in urine from MIBC patients vs. NMIBC patients. Therefore, ADAMTS12 has prognostic value in urine and tissue of patients with BCa and diagnostic value in the detection of BCa in urine.

IGF2BP2 (**Figures 10****,** **20****; Tables 1, 4-7**): The present GeneChip^{®} Human Exon 1.0 ST Array data, TLDA validation and qPCR assay data showed that IGF2BP2 was highly and significantly up-regulated in tissue from MIBC compared to NMIBC. IGF2BP2 could also be detected in urine and was significantly and highly up-regulated in urine from BCa patients vs. normal urine. Therefore, IGF2BP2 has prognostic value in tissue of patients with BCa and diagnostic value in the detection of BCa in urine.

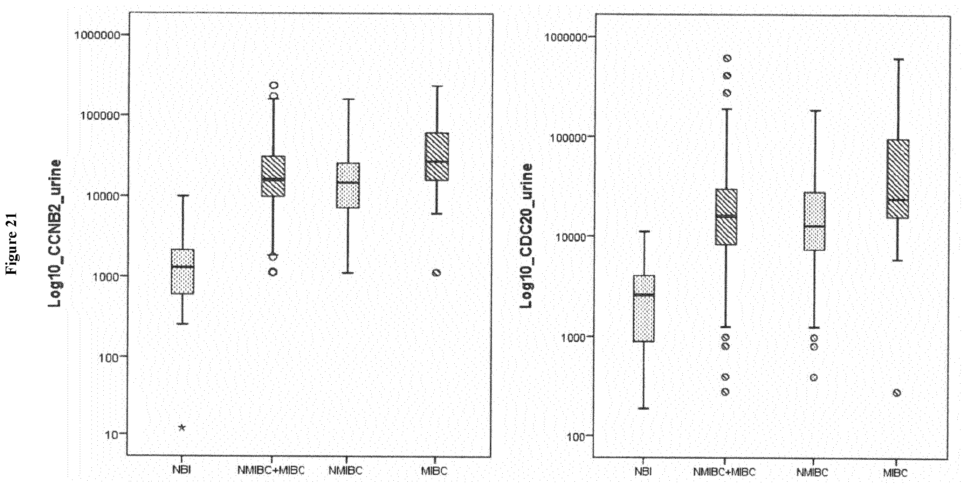

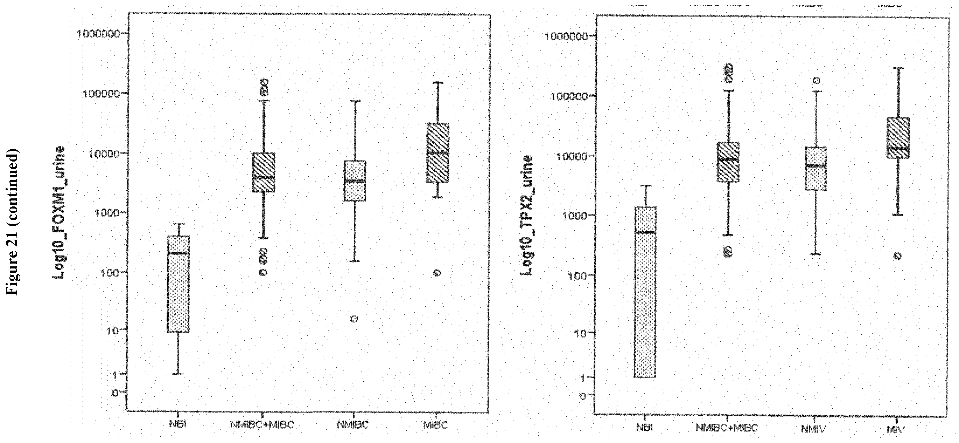

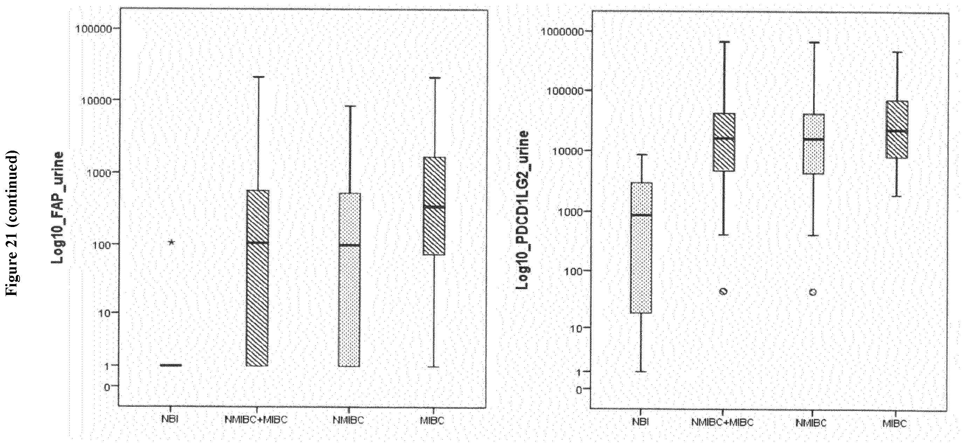

PDCD1LG2 (**Figures 11****,** **21****; Tables 1, 4-7**): The present GeneChip^{®} Human Exon 1.0 ST Array data, TLDA validation and qPCR assay data showed that PDCD1LG2 was significantly up-regulated in tissue from MIBC and BC-meta compared to NMIBC. PDCD1LG2 could also be detected in urine and was significantly and highly up-regulated in urine from BCa patients vs. normal urine. Therefore, PDCD1LG2 has prognostic value in tissue of patients with BCa and diagnostic value in the detection of BCa in urine.

SFRP4 (**Figure 12****; Tables 1, 4-7**): The present GeneChip^{®} Human Exon 1.0 ST Array data, TLDA validation and qPCR assay data showed that SFRP4 was highly and significantly up-regulated in tissue from MIBC and BC-meta compared to NMIBC. Low copy numbers of SFRP4 could also be detected in urine. SFRP4 was significantly up-regulated in urine from BCa patients vs. normal urine. Therefore, SFRP4 has prognostic value in tissue of patients with BCa and diagnostic value in the detection of BCa in urine.

KRT6A (**Figure 13****; Tables 1, 4-7**): The present GeneChip^{®} Human Exon 1.0 ST Array data, TLDA validation and qPCR assay data showed that KRT6A was highly and significantly up-regulated in tissue from MIBC compared to NMIBC. Therefore, KRT6A has prognostic value in tissue of patients with BCa.

TPX2 (**Figures 14****,** **19****,** **21****; Tables 1, 4-7**): The present GeneChip^{®} Human Exon 1.0 ST Array data, TLDA validation and qPCR assay data showed that TPX2 was highly and significantly up-regulated in tissue as well as in urine from patients with BCa compared to normal bladder and significantly up-regulated in tissue and urine from MIBC and BC-meta patients compared to NMIBC patients. Therefore, TPX2 has diagnostic value in tissue and in the detection of BCa in urine and has prognostic value in urine and in tissue of patients with BCa.

CCNB2 (**Figures 15****,** **19****,** **21****; Tables 1, 4-7**): The present GeneChip^{®} Human Exon 1.0 ST Array data, TLDA validation and qPCR assay data showed that CCNB2 was highly and significantly up-regulated in tissue as well as in urine from patients with BCa compared to NBl and significantly up-regulated in tissue and urine from MIBC and BC-meta patients compared to NMIBC patients. Therefore, CCNB2 has diagnostic value in tissue and in the detection of BCa in urine and has prognostic value in urine and in tissue of patients with BCa.

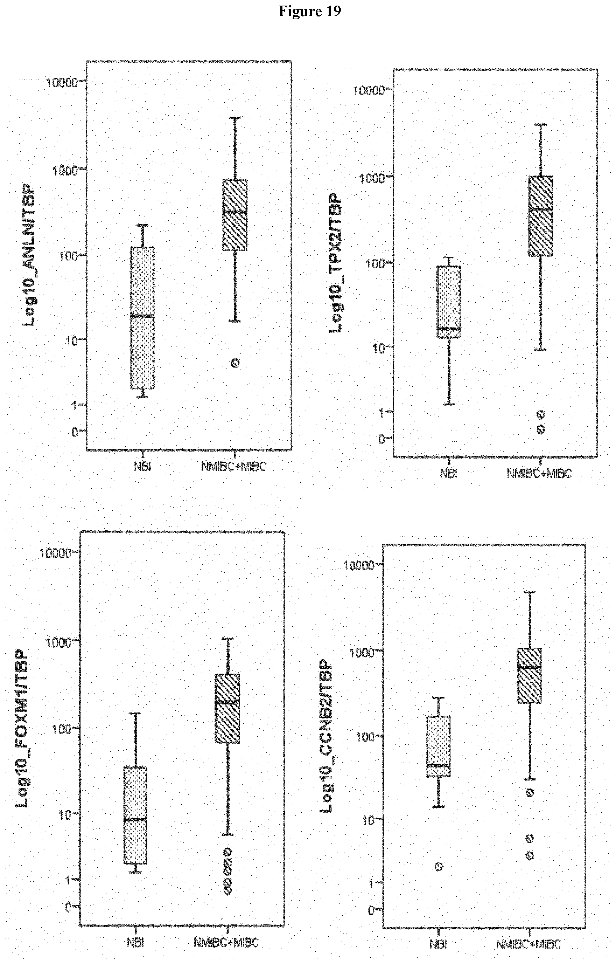

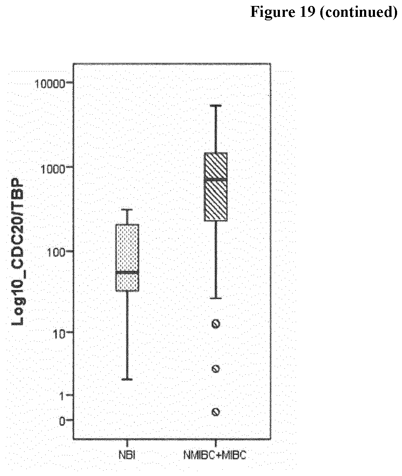

ANLN (**Figures 16****,** **19****; Tables 1, 4-7**): The present GeneChip^{®} Human Exon 1.0 ST Array data, TLDA validation and qPCR assay data showed that ANLN was highly and significantly up-regulated in tissue as well as in urine from patients with BCa compared to NBl and significantly up-regulated in tissue from MIBC and BC-meta patients compared to NMIBC patients. Therefore, ANLN has diagnostic value in tissue and in the detection of BCa in urine and has prognostic value in tissue of patients with BCa.

FOXM1 (**Figures 17****,** **19****,** **21****; Tables 1, 4-7**): The present GeneChip^{®} Human Exon 1.0 ST Array data, TLDA validation and qPCR assay data showed that FOXM1 was highly and significantly up-regulated in tissue as well as in urine from patients with BCa compared to NBl and significantly up-regulated in tissue and urine from MIBC and BC-meta patients compared to NMIBC patients. Therefore, FOXM1 has diagnostic value in tissue and in the detection of BCa in urine and has prognostic value in urine and in tissue of patients with BCa.

CDC20 (**Figures 18****,** **19****,** **21****; Tables 1, 4-7**): The present GeneChip^{®} Human Exon 1.0 ST Array data, TLDA validation and qPCR assay data showed that CDC20 was highly and significantly up-regulated in tissue as well as in urine from patients with BCa compared to NBl and significantly up-regulated in tissue and urine from MIBC and BC-meta patients compared to NMIBC patients. Therefore, CDC20 has diagnostic value in tissue and in the detection of BCa in urine and has prognostic value in urine and in tissue of patients with BCa.

### Example 4: Selection of the best candidate biomarkers

Based on the highest up-regulation in BCa vs NBl and MIBC vs. NMIBC, lowest P-value and high copy numbers the best performing diagnostic and prognostic individual biomarkers in tissue and urine were identified. The five best performing individual biomarkers for the detection of BCa in tissue were identified and are shown in boxplots in **Figure 19****:** ANLN, TPX2, FOXM1, CCNB2 and CDC20.

The five best performing individual biomarkers that could distinguish MIBC tissue from NMIBC tissue were identified and are shown in boxplots **in** **Figure 20****:** IGF2BP2, INHBA, ADAMTS12 FAP and CHI3L1.

The six best performing individual biomarkers for the detection of BCa in urine were identified and are shown in a boxplot in **Figure 21****:** FAP, TPX2, CCNB2, CDC20, FOXM1 and PDCD1LG2. The first five genes could also significantly distinguish MIBC from NMIBC in urine.

Given that the nature of these tumors is very heterogeneous, it is likely that combination of markers can identify different patients and have additional diagnostic and/or prognostic value to eachother. For the identification of the best combinations of biomarkers for the diagnosis of BCa in urine and/or tissue and for the best combinations of markers that had prognostic value by distinguishing MIBC from NMIBC in tissue and/or urine the method of binary logistic regression analysis was performed. All data were log-transformed prior to statistical analysis as a transformation to a normal distribution. Binary logistic regression analysis (stepwise forward) was performed with the 17 biomarkers in order to find regression models and marker combinations for predicting the presence of bladder cancer (NMIBC and MIBC) in urine or for predicting whether BCa is muscle invasive or not. The statistical significant level for all tests was set at P=0.05.

As example two possible identified combinations of biomarkers are described, one for predicting the occurrence of BCa based on the expression of the markers in urine and one for predicting the occurence of muscle invasive disease based on the expression of the markers in tissue.

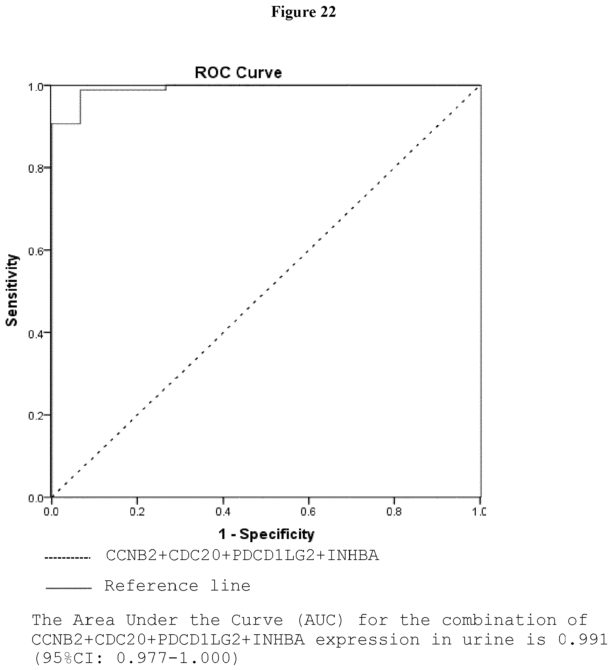

In urine, CCNB2 is a key predictor and predicts that 66.7% of healthy controls have no cancer and that 96.5% from the cancer patients do have cancer. With the addition of CDC20 and PDCD1LG2 the new model predicts that 80% of the healthy controls have no cancer and 96.5% of the cancer patients are correctly classified. When INHBA is added to this model the model model predicts that 93.3% of the healthy controls have no cancer and that 98.8% of the cancer patients are correctly calssified. This four biomarker model is higly significant (P=1.9E-13 ) showing that the biomarkers can predict the presence of bladdercancer in urine well. To visualize the performance of the biomarker combinations a ROC curve is shown (**Figure 22**).

In a ROC curve, the true positive rate to detect BCa or MIBC (sensitivity) is plotted in function of the false positive rate (i.e. positives in the control group, 1-specificity) for different cut-off points. Each point on the curve represents a sensitivity/specificity pair corresponding to a particular decision threshold. The Area Under the Curve (AUC) of the ROC curve is a measure how well a parameter can distinguish between two groups and is maximum 1.0 (all samples correctly classified). The AUC for the combination of CCNB2, CDC20, PDCD1LG2 and INHBA expression is 0.991 (95%CI: 0.977-1.000).

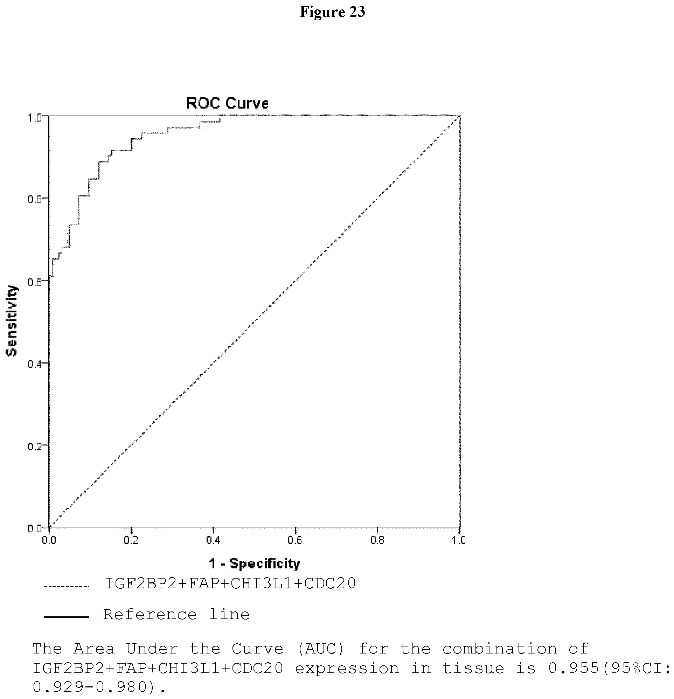

In tissue, FAP is a key predictor and predicts that 87% of the NMIBC are NMIBC and that 80.3% of the MIBC specimen are correctly classified. When CDC20 and CHI3L1 are added 89.3 of the NMIBC are correctly classified and 83.1% of the MIBC are correctly classified. The addition of IGF2BP2 leads to the correct classification of 90.2% of the NMIBC and 83.1% of the MIBC. This four marker model is higly significant (P=4.9 x10-32) showing that the biomarkers can predict the occurence of muscle invasive disease well. The Area Under the Curve (AUC) for the combination of IGF2BP2+FAP+CHI3L1+CDC20 expression is 0.955 (95%CI: 0.929-0.980). See **Figure 23****.**

Based on the binary logistic regression model the following genes and combinations were identified. For predicting the occurrence of BCa (diagnosis) based on the detection and quantification expression of the markers in tissue: at least ANLN combined with one or more markers from the list: IGF2BP2, FAP, CTHRC1, CCNB2, COL10A1 and/or TPX2.

For predicting the occurence of muscle invasive disease (prognosis) based on the expression of the markers in tissue: at least FAP, combined with one or more markers from the list: CDC20, CHI3L1, IGF2BP2, INHBA, ADAMTS12, CCNB2 and/or ANLN or at least CHI3L1, combined with one or more markers from the list: CDC20, FAP, IGF2BP2, INHBA, ADAMTS12, CCNB2 and/or ANLN.

For predicting the occurrence of BCa based on the expression of the markers in urine at least CCNB2, combined with one or more markers from the list: CDC20, PDCD1LG2, TPX2, SFRP4, COL10A1, INHBA and/or TC2526896 or at least PDCD1LG2, combined with one or more markers from the list: CCNB2, CDC20, TPX2, SFRP4, COL10A1, INHBA and/or CTHRC1

For predicting the occurence of muscle invasive disease based on the expression of the markers in urine at least FAP, combined with one or more from the list FOXM1, CCNB2, CDC20 and/or TC2526896

### CONCLUSIONS:

The present invention relates to biomarkers and their diagnostic and prognostic uses for bladder cancer. The biomarkers can be used alone or in combination. The invention provides methods for diagnosing bladder cancer in a subject, comprising measuring the levels of a single or a plurality of biomarkers in a biological sample derived from a subject suspected of having bladder cancer. Differential expression of one or more biomarkers in the biological sample is compared to one or more biomarkers in a healthy control sample indicates that a subject has cancer. Furthermore, the invention provides methods for determing classification of tumors according to the aggressiveness or establishing the prediction of prognosis and disease outcome for a human individual suffering from bladder cancer, comprising measuring the levels of a single or a plurality of biomarkers in a biological sample derived from a subject having bladder cancer. Differential expression of one or more biomarkers in the biological sample is compared to one or more biomarkers in a NMIBC control sample that indicates that a subject has an aggressive type of bladder cancer.

## Claims

1. An *in vitro* method for establishing the presence, or absence, of a bladder tumour in a human individual; or establishing the prediction of prognosis and disease outcome for a human individual suffering from bladder cancer comprising:
a) determining the expression of CTHRC1 in a sample originating from said human individual; and
b) establishing upregulation of expression of CTHRC1 as compared to expression of CTHRC1 in a sample originating from said human individual not comprising tumour cells or tissue, or from an individual, or group of individuals, not suffering from bladder cancer; and
c) establishing the presence, or absence, of a bladder tumour based on the established upregulation of CTHRC1; or establishing the prediction of prognosis and disease outcome for a human individual suffering from bladder cancer based on the established upregulation of CTHRC1.

2. Method according to claim 1, wherein establishing the presence, or absence, of bladder cancer in a human individual preferably includes diagnosis, prognosis and/or prediction of disease survival.

3. Method according to claim 1 or claim 2, wherein expression analysis is performed on a sample selected from the group consisting of body fluid, saliva, lymph, blood, urine, tissue sample and a transurethral resection of a bladder tumour (TURBT), preferably blood, urine, urine sediment, and samples of, derived or originating from TURBT specimens.

4. Method according to any of the claims 1 to 3, wherein determining the expression comprises determining mRNA expression of the one or more genes, preferably by Northern blot hybridisation or amplification based techniques, preferably PCR, real time PCR, or NASBA.

5. Method according to any of the claims 1 to 4, wherein expression analysis comprises high-throughput array chip analysis.

6. Method according to any of the claims 1 to 4, wherein expression analysis comprises determining protein levels of the said genes, preferably by matrix-assisted laser desorption-ionization time-of-flight mass spectrometer (MALDI-TOF).

7. Use of expression analysis of CTHRC1 for establishing the presence, or absence, of a bladder tumour or establishing the prediction of prognosis and disease outcome for an individual patient suffering from bladder cancer.

## Patentansprüche

1. Ein in vitro Verfahren zur Bestimmung der Anwesenheit oder Abwesenheit eines Blasentumors in einem menschlichen Individuum; oder zur Vorhersage einer Prognose und eines Krankheitsverlaufs für ein menschliches Individuum, welches an Blasenkrebs leidet, aufweisend:
a) Bestimmen der Expression von CTHRC1 in einer Probe, welche von genanntem menschlichen Individuum stammt; und
b) Etablieren einer Hochregulierung der Expression von CTHRC1 im Vergleich zu einer Expression von CTHRC1 in einer Probe von genanntem menschlichem Individuum, welche keine Tumorzellen oder Tumorgewebe beinhaltet, oder von einem Individuum, oder einer Gruppe von Individuen, welche nicht an Blasenkrebs leiden; und
c) Etablieren der Anwesenheit oder Abwesenheit eines Blasentumors basierend auf der etablierten Hochregulierung von CTHRC1; oder Etablieren der Vorhersage einer Prognose und des Krankheitsverlaufs für das menschliche Individuum basierend auf der etablierten Hochregulierung von CTHRC1.

2. Verfahren nach Anspruch 1, wobei das Etablieren der Anwesenheit oder Abwesenheit von Blasenkrebs in einem menschlichen Individuum vorzugsweise eine Diagnose, Prognose und/oder Vorhersage eines Krankheitsüberlebens beinhaltet.

3. Verfahren nach Anspruch 1 oder Anspruch 2, wobei eine Expressionsanalyse an einer Probe aus einer Gruppe, bestehend aus Körperflüssigkeit, Speichel, Lymphe, Blut, Urin, Gewebeprobe, transurethrale Resektion eines Blasentumors (TURBT), vorzugsweise Blut, Urin, Urinsediment, und Proben gewonnen aus oder ursprünglich stammend aus einer TURBT Probe, durchgeführt wird.

4. Verfahren nach einem der Ansprüche 1 bis 3, wobei das Bestimmen der Expression ein Bestimmen einer mRNA Expression eines oder mehrerer Gene, vorzugsweise durch Northern-Blot-Hybridisierung oder verstärkungsbasierten Verfahren, vorzugsweise PCR, Realzeit PCR oder NASBA beinhaltet.

5. Verfahren nach einem der Ansprüche 1 bis 4, wobei die Expressionsanalyse eine Hoch-Durchsatz-Chip-Array-Analyse beinhaltet.

6. Verfahren nach einem der Ansprüche 1 bis 4, wobei die Expressionsanalyse ein Bestimmen von Proteingehalten genannter Gene, vorzugsweise durch matrixunterstütztes laser desorbtions-ionisations Flugzeit Massenspektrometer, (MALDI-TOF).

7. Verwendung einer Expression von CTHRC1 zur Bestimmung der Anwesenheit, oder Abwesenheit eines Blasentumors in einem menschlichen Individuum; oder zur Vorhersage einer Prognose und eines Krankheitsverlaufs für ein menschliches Individuum, welches an Blasenkrebs leidet.

## Revendications

1. Procédé d'établissement de la présence ou de l'absence d'une tumeur de la vessie in vitro chez un individu humain ; ou d'établissement de la prévision de pronostic et d'issue de la maladie pour un individu humain souffrant d'un cancer de la vessie comprenant:
a) la détermination de l'expression de CTHRC1 dans un échantillon provenant dudit individu humain; et
b) l'établissement d'une régulation à la hausse de l'expression de CTHRC1 comparée à l'expression de CTHRC1 dans un échantillon provenant dudit individu humain ne comprenant pas de cellules ou de tissus tumoraux, ou à partir d'un individu, ou groupe d'individus, ne souffrant pas d'un cancer de la vessie; et
c) l'établissement de la présence ou de l'absence d'une tumeur de la vessie sur la base de la régulation à la hausse de CTHRC1 établie ; ou l'établissement de la prévision de pronostic et de l'issue de la maladie pour un individu humain souffrant d'un cancer de vessie sur la base de la régulation à la hausse de CTHRC1 établie.

2. Procédé selon la revendication 1, dans lequel l'établissement de la présence ou de l'absence d'un cancer de la vessie chez un individu humain comporte, de préférence, le diagnostic, le pronostic et/ou la prévision de survie à la maladie.

3. Procédé selon la revendication 1 ou 2, dans lequel l'analyse d'expression est mise en œuvre sur un échantillon sélectionné à partir du groupe constitué par un fluide corporel, de la salive, de la lymphe, du sang, de l'urine, un échantillon de tissu et une résection transurétrale d'une tumeur de la vessie (TURBT), de préférence, du sang, de l'urine, un sédiment d'urine et des échantillons de spécimens de TURBT, dérivés ou provenant de ces derniers.

4. Procédé selon l'une quelconque des revendications 1 à 3, dans lequel la détermination de l'expression comprend la détermination de l'expression d'ARNm du ou des gènes, de préférence, par des procédés basés l'hybridation ou l'amplification de tache de Northern, de préférence, par PCR, PCR en temps réel, ou NASBA.

5. Procédé selon l'une quelconque des revendications 1 à 4, dans lequel l'analyse d'expression comporte l'analyse par puces matricielles à haut débit.

6. Procédé selon l'une quelconque des revendications 1 à 4, dans lequel l'analyse d'expression comprend la détermination de niveaux de protéine desdits gènes, de préférence, par spectromètre de masse à désorption/ionisation laser assistée par matrice, à temps de vol (MALDI-TOF).

7. Utilisation de l'analyse d'expression de CTHRC1 afin d'établir la présence ou l'absence d'une tumeur de la vessie ou d'établir la prévision de pronostic et de l'issue de la maladie pour un patient individuel souffrant d'un cancer de la vessie.


### SEQUENCE LISTING

<110> MDxHealth Research B.V.
<120> MOLECULAR MARKERS IN BLADDER CANCER
<130> P117005EP02
<141> 2014-03-07
<150> PCT/EP2013/054777
   <151> 2013-03-08
<160> 34
<170> BiSSAP 1.2
<210> 1
   <211> 2175
   <212> DNA
   <213> Homo sapiens
<220>
   <221> source
   <222> 1..2175
   <223> /mol_type="unassigned DNA"
   /note="INHBA"
   /organism="Homo sapiens"
<400> 1
   agtacagtat aaaacttcac agtgccaata ccatgaagag gagctcagac agctcttacc      60
   acatgataca agagccggct ggtggaagag tggggaccag aaagagaatt tgctgaagag     120
   gagaaggaaa aaaaaaacac caaaaaaaaa aataaaaaaa tccacacaca caaaaaaacc     180
   tgcgcgtgag gggggaggaa aagcagggcc ttttaaaaag gcaatcacaa caacttttgc     240
   tgccaggatg cccttgcttt ggctgagagg atttctgttg gcaagttgct ggattatagt     300
   gaggagttcc cccaccccag gatccgaggg gcacagcgcg gcccccgact gtccgtcctg     360
   tgcgctggcc gccctcccaa aggatgtacc caactctcag ccagagatgg tggaggccgt     420
   caagaagcac attttaaaca tgctgcactt gaagaagaga cccgatgtca cccagccggt     480
   acccaaggcg gcgcttctga acgcgatcag aaagcttcat gtgggcaaag tcggggagaa     540
   cgggtatgtg gagatagagg atgacattgg aaggagggca gaaatgaatg aacttatgga     600
   gcagacctcg gagatcatca cgtttgccga gtcaggaaca gccaggaaga cgctgcactt     660
   cgagatttcc aaggaaggca gtgacctgtc agtggtggag cgtgcagaag tctggctctt     720
   cctaaaagtc cccaaggcca acaggaccag gaccaaagtc accatccgcc tcttccagca     780
   gcagaagcac ccgcagggca gcttggacac aggggaagag gccgaggaag tgggcttaaa     840
   gggggagagg agtgaactgt tgctctctga aaaagtagta gacgctcgga agagcacctg     900
   gcatgtcttc cctgtctcca gcagcatcca gcggttgctg gaccagggca agagctccct     960
   ggacgttcgg attgcctgtg agcagtgcca ggagagtggc gccagcttgg ttctcctggg    1020
   caagaagaag aagaaagaag aggaggggga agggaaaaag aagggcggag gtgaaggtgg 1080
   ggcaggagca gatgaggaaa aggagcagtc gcacagacct ttcctcatgc tgcaggcccg 1140
   gcagtctgaa gaccaccctc atcgccggcg tcggcggggc ttggagtgtg atggcaaggt 1200
   caacatctgc tgtaagaaac agttctttgt cagtttcaag gacatcggct ggaatgactg 1260
   gatcattgct ccctctggct atcatgccaa ctactgcgag ggtgagtgcc cgagccatat 1320
   agcaggcacg tccgggtcct cactgtcctt ccactcaaca gtcatcaacc actaccgcat 1380
   gcggggccat agcccctttg ccaacctcaa atcgtgctgt gtgcccacca agctgagacc 1440
   catgtccatg ttgtactatg atgatggtca aaacatcatc aaaaaggaca ttcagaacat 1500
   gatcgtggag gagtgtgggt gctcatagag ttgcccagcc cagggggaaa gggagcaaga 1560
   gttgtccaga gaagacagtg gcaaaatgaa gaaattttta aggtttctga gttaaccaga 1620
   aaaatagaaa ttaaaaacaa aacaaaaaaa aaaacaaaaa aaaacaaaag taaattaaaa 1680
   acaaaacctg atgaaacaga tgaaggaaga tgtggaaaaa atccttagcc agggctcaga 1740
   gatgaagcag tgaaagagac aggaattggg agggaaaggg agaatggtgt accctttatt 1800
   tcttctgaaa tcacactgat gacatcagtt gtttaaacgg ggtattgtcc tttcccccct 1860
   tgaggttccc ttgtgagcct tgaatcaacc aatctagtct gcagtagtgt ggactagaac 1920
   aacccaaata gcatctagaa agccatgagt ttgaaagggc ccatcacagg cactttccta 1980
   cccaattacc caggtcataa ggtatgtctg tgtgacactt atctctgtgt atatcagcat 2040
   acacacacac acacacacac acacacacac acacaggcat ttccacacat tacatatata 2100
   cacatactgg taaaagaaca atcgtgtgca ggtggtcaca cttccttttt ctgtaccact 2160
   tttgcaacaa aacaa 2175
<210> 2
   <211> 426
   <212> PRT
   <213> Homo sapiens
<400> 2
   Met Pro Leu Leu Trp Leu Arg Gly Phe Leu Leu Ala Ser Cys Trp Ile
   1               5                   10                  15
   Ile Val Arg Ser Ser Pro Thr Pro Gly Ser Glu Gly His Ser Ala Ala
               20                  25                  30
   Pro Asp Cys Pro Ser Cys Ala Leu Ala Ala Leu Pro Lys Asp Val Pro
           35                  40                  45
   Asn Ser Gln Pro Glu Met Val Glu Ala Val Lys Lys His Ile Leu Asn
       50                  55                  60
   Met Leu His Leu Lys Lys Arg Pro Asp Val Thr Gln Pro Val Pro Lys
   65                  70                  75                  80
   Ala Ala Leu Leu Asn Ala Ile Arg Lys Leu His Val Gly Lys Val Gly
                   85                  90                  95
   Glu Asn Gly Tyr Val Glu Ile Glu Asp Asp Ile Gly Arg Arg Ala Glu
               100                 105                 110
   Met Asn Glu Leu Met Glu Gln Thr Ser Glu Ile Ile Thr Phe Ala Glu
           115                 120                 125
   Ser Gly Thr Ala Arg Lys Thr Leu His Phe Glu Ile Ser Lys Glu Gly
       130                 135                 140
   Ser Asp Leu Ser Val Val Glu Arg Ala Glu Val Trp Leu Phe Leu Lys
   145                 150                 155                 160
   Val Pro Lys Ala Asn Arg Thr Arg Thr Lys Val Thr Ile Arg Leu Phe
                   165                 170                 175
   Gln Gln Gln Lys His Pro Gln Gly Ser Leu Asp Thr Gly Glu Glu Ala
               180                 185                 190
   Glu Glu Val Gly Leu Lys Gly Glu Arg Ser Glu Leu Leu Leu Ser Glu
           195                 200                 205
   Lys Val Val Asp Ala Arg Lys Ser Thr Trp His Val Phe Pro Val Ser
       210                 215                 220
   Ser Ser Ile Gln Arg Leu Leu Asp Gln Gly Lys Ser Ser Leu Asp Val
   225                 230                 235                 240
   Arg Ile Ala Cys Glu Gln Cys Gln Glu Ser Gly Ala Ser Leu Val Leu
                   245                 250                 255
   Leu Gly Lys Lys Lys Lys Lys Glu Glu Glu Gly Glu Gly Lys Lys Lys
               260                 265                 270
   Gly Gly Gly Glu Gly Gly Ala Gly Ala Asp Glu Glu Lys Glu Gln Ser
           275                 280                 285
   His Arg Pro Phe Leu Met Leu Gln Ala Arg Gln Ser Glu Asp His Pro
       290                 295                 300
   His Arg Arg Arg Arg Arg Gly Leu Glu Cys Asp Gly Lys Val Asn Ile
   305                 310                 315                 320
   Cys Cys Lys Lys Gln Phe Phe Val Ser Phe Lys Asp Ile Gly Trp Asn
                   325                 330                 335
   Asp Trp Ile Ile Ala Pro Ser Gly Tyr His Ala Asn Tyr Cys Glu Gly
               340                 345                 350
   Glu Cys Pro Ser His Ile Ala Gly Thr Ser Gly Ser Ser Leu Ser Phe
           355                 360                 365
   His Ser Thr Val Ile Asn His Tyr Arg Met Arg Gly His Ser Pro Phe
       370                 375                 380
   Ala Asn Leu Lys Ser Cys Cys Val Pro Thr Lys Leu Arg Pro Met Ser
   385                 390                 395                 400
   Met Leu Tyr Tyr Asp Asp Gly Gln Asn Ile Ile Lys Lys Asp Ile Gln
                   405                 410                 415
   Asn Met Ile Val Glu Glu Cys Gly Cys Ser
               420                 425
<210> 3
   <211> 1279
   <212> DNA
   <213> Homo sapiens
<220>
   <221> source
   <222> 1..1279
   <223> /mol_type="unassigned DNA"
   /organism="Homo sapiens"
<400> 3
   gggagggaga gaggcgcgcg ggtgaaaggc gcattgatgc agcctgcggc ggcctcggag      60
   cgcggcggag ccagacgctg accacgttcc tctcctcggt ctcctccgcc tccagctccg     120
   cgctgcccgg cagccgggag ccatgcgacc ccagggcccc gccgcctccc cgcagcggct     180
   ccgcggcctc ctgctgctcc tgctgctgca gctgcccgcg ccgtcgagcg cctctgagat     240
   ccccaagggg aagcaaaagg cgcagctccg gcagagggag gtggtggacc tgtataatgg     300
   aatgtgctta caagggccag caggagtgcc tggtcgagac gggagccctg gggccaatgg     360
   cattccgggt acacctggga tcccaggtcg ggatggattc aaaggagaaa agggggaatg     420
   tctgagggaa agctttgagg agtcctggac acccaactac aagcagtgtt catggagttc     480
   attgaattat ggcatagatc ttgggaaaat tgcggagtgt acatttacaa agatgcgttc     540
   aaatagtgct ctaagagttt tgttcagtgg ctcacttcgg ctaaaatgca gaaatgcatg     600
   ctgtcagcgt tggtatttca cattcaatgg agctgaatgt tcaggacctc ttcccattga     660
   agctataatt tatttggacc aaggaagccc tgaaatgaat tcaacaatta atattcatcg     720
   cacttcttct gtggaaggac tttgtgaagg aattggtgct ggattagtgg atgttgctat     780
   ctgggttggt acttgttcag attacccaaa aggagatgct tctactggat ggaattcagt     840
   ttctcgcatc attattgaag aactaccaaa ataaatgctt taattttcat ttgctacctc     900
   tttttttatt atgccttgga atggttcact taaatgacat tttaaataag tttatgtata     960
   catctgaatg aaaagcaaag ctaaatatgt ttacagacca aagtgtgatt tcacactgtt    1020
   tttaaatcta gcattattca ttttgcttca atcaaaagtg gtttcaatat tttttttagt    1080
   tggttagaat actttcttca tagtcacatt ctctcaacct ataatttgga atattgttgt    1140
   ggtcttttgt tttttctctt agtatagcat ttttaaaaaa atataaaagc taccaatctt    1200
   tgtacaattt gtaaatgtta agaatttttt ttatatctgt taaataaaaa ttatttccaa    1260
   caaccttaat atctttaaa                                                 1279
<210> 4
   <211> 243
   <212> PRT
   <213> Homo sapiens
<400> 4
   Met Arg Pro Gln Gly Pro Ala Ala Ser Pro Gln Arg Leu Arg Gly Leu
   1               5                   10                  15
   Leu Leu Leu Leu Leu Leu Gln Leu Pro Ala Pro Ser Ser Ala Ser Glu
               20                  25                  30
   Ile Pro Lys Gly Lys Gln Lys Ala Gln Leu Arg Gln Arg Glu Val Val
           35                  40                  45
   Asp Leu Tyr Asn Gly Met Cys Leu Gln Gly Pro Ala Gly Val Pro Gly
       50                  55                  60
   Arg Asp Gly Ser Pro Gly Ala Asn Gly Ile Pro Gly Thr Pro Gly Ile
   65                  70                  75                  80
   Pro Gly Arg Asp Gly Phe Lys Gly Glu Lys Gly Glu Cys Leu Arg Glu
                   85                  90                  95
   Ser Phe Glu Glu Ser Trp Thr Pro Asn Tyr Lys Gln Cys Ser Trp Ser
               100                 105                 110
   Ser Leu Asn Tyr Gly Ile Asp Leu Gly Lys Ile Ala Glu Cys Thr Phe
           115                 120                 125
   Thr Lys Met Arg Ser Asn Ser Ala Leu Arg Val Leu Phe Ser Gly Ser
       130                 135                 140
   Leu Arg Leu Lys Cys Arg Asn Ala Cys Cys Gln Arg Trp Tyr Phe Thr
   145                 150                 155                 160
   Phe Asn Gly Ala Glu Cys Ser Gly Pro Leu Pro Ile Glu Ala Ile Ile
                   165                 170                 175
   Tyr Leu Asp Gln Gly Ser Pro Glu Met Asn Ser Thr Ile Asn Ile His
               180                 185                 190
   Arg Thr Ser Ser Val Glu Gly Leu Cys Glu Gly Ile Gly Ala Gly Leu
           195                 200                 205
   Val Asp Val Ala Ile Trp Val Gly Thr Cys Ser Asp Tyr Pro Lys Gly
       210                 215                 220
   Asp Ala Ser Thr Gly Trp Asn Ser Val Ser Arg Ile Ile Ile Glu Glu
   225                 230                 235                 240
   Leu Pro Lys
<210> 5
   <211> 1867
   <212> DNA
   <213> Homo sapiens
<220>
   <221> source
   <222> 1..1867
   <223> /mol_type="unassigned DNA"
   /organism="Homo sapiens"
<400> 5
   cacatagctc agttcccata aaagggctgg tttgccgcgt cggggagtgg agtgggacag      60
   gtatataaag gaagtacagg gcctggggaa gaggccctgt ctaggtagct ggcaccagga     120
   gccgtgggca agggaagagg ccacaccctg ccctgctctg ctgcagccag aatgggtgtg     180
   aaggcgtctc aaacaggctt tgtggtcctg gtgctgctcc agtgctgctc tgcatacaaa     240
   ctggtctgct actacaccag ctggtcccag taccgggaag gcgatgggag ctgcttccca     300
   gatgcccttg accgcttcct ctgtacccac atcatctaca gctttgccaa tataagcaac     360
   gatcacatcg acacctggga gtggaatgat gtgacgctct acggcatgct caacacactc     420
   aagaacagga accccaacct gaagactctc ttgtctgtcg gaggatggaa ctttgggtct     480
   caaagatttt ccaagatagc ctccaacacc cagagtcgcc ggactttcat caagtcagta     540
   ccgccatttc tgcgcaccca tggctttgat gggctggacc ttgcctggct ctaccctgga     600
   cggagagaca aacagcattt taccacccta atcaaggaaa tgaaggccga atttataaag     660
   gaagcccagc cagggaaaaa gcagctcctg ctcagcgcag cactgtctgc ggggaaggtc     720
   accattgaca gcagctatga cattgccaag atatcccaac acctggattt cattagcatc     780
   atgacctacg attttcatgg agcctggcgt gggaccacag gccatcacag tcccctgttc     840
   cgaggtcagg aggatgcaag tcctgacaga ttcagcaaca ctgactatgc tgtggggtac     900
   atgttgaggc tgggggctcc tgccagtaag ctggtgatgg gcatccccac cttcgggagg     960
   agcttcactc tggcttcttc tgagactggt gttggagccc caatctcagg accgggaatt    1020
   ccaggccggt tcaccaagga ggcagggacc cttgcctact atgagatctg tgacttcctc    1080
   cgcggagcca cagtccatag aatcctcggc cagcaggtcc cctatgccac caagggcaac    1140
   cagtgggtag gatacgacga ccaggaaagc gtcaaaagca aggtgcagta cctgaaggac    1200
   aggcagctgg cgggcgccat ggtatgggcc ctggacctgg atgacttcca gggctccttc    1260
   tgcggccagg atctgcgctt ccctctcacc aatgccatca aggatgcact cgctgcaacg    1320
   tagccctctg ttctgcacac agcacggggg ccaaggatgc cccgtccccc tctggctcca    1380
   gctggccggg agcctgatca cctgccctgc tgagtcccag gctgagcctc agtctccctc    1440
   ccttggggcc tatgcagagg tccacaacac acagatttga gctcagccct ggtgggcaga    1500
   gaggtaggga tggggctgtg gggatagtga ggcatcgcaa tgtaagactc gggattagta    1560
   cacacttgtt gattaatgga aatgtttaca gatccccaag cctggcaagg gaatttcttc    1620
   aactccctgc cccccagccc tccttatcaa aggacaccat tttggcaagc tctatcacca    1680
   aggagccaaa catcctacaa gacacagtga ccatactaat tataccccct gcaaagccca    1740
   gcttgaaacc ttcacttagg aacgtaatcg tgtcccctat cctacttccc cttcctaatt    1800
   ccacagctgc tcaataaagt acaagagctt aacagtgaaa aaaaaaaaaa aaaaaaaaaa    1860
   aaaaaaa                                                              1867
<210> 6
   <211> 383
   <212> PRT
   <213> Homo sapiens
<400> 6
   Met Gly Val Lys Ala Ser Gln Thr Gly Phe Val Val Leu Val Leu Leu
   1               5                   10                  15
   Gln Cys Cys Ser Ala Tyr Lys Leu Val Cys Tyr Tyr Thr Ser Trp Ser
               20                  25                  30
   Gln Tyr Arg Glu Gly Asp Gly Ser Cys Phe Pro Asp Ala Leu Asp Arg
           35                  40                  45
   Phe Leu Cys Thr His Ile Ile Tyr Ser Phe Ala Asn Ile Ser Asn Asp
       50                  55                  60
   His Ile Asp Thr Trp Glu Trp Asn Asp Val Thr Leu Tyr Gly Met Leu
   65                  70                  75                  80
   Asn Thr Leu Lys Asn Arg Asn Pro Asn Leu Lys Thr Leu Leu Ser Val
                   85                  90                  95
   Gly Gly Trp Asn Phe Gly Ser Gln Arg Phe Ser Lys Ile Ala Ser Asn
               100                 105                 110
   Thr Gln Ser Arg Arg Thr Phe Ile Lys Ser Val Pro Pro Phe Leu Arg
           115                 120                 125
   Thr His Gly Phe Asp Gly Leu Asp Leu Ala Trp Leu Tyr Pro Gly Arg
       130                 135                 140
   Arg Asp Lys Gln His Phe Thr Thr Leu Ile Lys Glu Met Lys Ala Glu
   145                 150                 155                 160
   Phe Ile Lys Glu Ala Gln Pro Gly Lys Lys Gln Leu Leu Leu Ser Ala
                   165                 170                 175
   Ala Leu Ser Ala Gly Lys Val Thr Ile Asp Ser Ser Tyr Asp Ile Ala
               180                 185                 190
   Lys Ile Ser Gln His Leu Asp Phe Ile Ser Ile Met Thr Tyr Asp Phe
           195                 200                 205
   His Gly Ala Trp Arg Gly Thr Thr Gly His His Ser Pro Leu Phe Arg
       210                 215                 220
   Gly Gln Glu Asp Ala Ser Pro Asp Arg Phe Ser Asn Thr Asp Tyr Ala
   225                 230                 235                 240
   Val Gly Tyr Met Leu Arg Leu Gly Ala Pro Ala Ser Lys Leu Val Met
                   245                 250                 255
   Gly Ile Pro Thr Phe Gly Arg Ser Phe Thr Leu Ala Ser Ser Glu Thr
               260                 265                 270
   Gly Val Gly Ala Pro Ile Ser Gly Pro Gly Ile Pro Gly Arg Phe Thr
           275                 280                 285
   Lys Glu Ala Gly Thr Leu Ala Tyr Tyr Glu Ile Cys Asp Phe Leu Arg
       290                 295                 300
   Gly Ala Thr Val His Arg Ile Leu Gly Gln Gln Val Pro Tyr Ala Thr
   305                 310                 315                 320
   Lys Gly Asn Gln Trp Val Gly Tyr Asp Asp Gln Glu Ser Val Lys Ser
                   325                 330                 335
   Lys Val Gln Tyr Leu Lys Asp Arg Gln Leu Ala Gly Ala Met Val Trp
               340                 345                 350
   Ala Leu Asp Leu Asp Asp Phe Gln Gly Ser Phe Cys Gly Gln Asp Leu
           355                 360                 365
   Arg Phe Pro Leu Thr Asn Ala Ile Lys Asp Ala Leu Ala Ala Thr
       370                 375                 380
<210> 7
   <211> 3307
   <212> DNA
   <213> Homo sapiens
<220>
   <221> source
   <222> 1..3307
   <223> /mol_type="unassigned DNA"
   /organism="Homo sapiens"
<400> 7
   caccttctgc actgctcatc tgggcagagg aagcttcaga aagctgccaa ggcaccatct      60
   ccaggaactc ccagcacgca gaatccatct gagaatatgc tgccacaaat accctttttg     120
   ctgctagtat ccttgaactt ggttcatgga gtgttttacg ctgaacgata ccaaatgccc     180
   acaggcataa aaggcccact acccaacacc aagacacagt tcttcattcc ctacaccata     240
   aagagtaaag gtatagcagt aagaggagag caaggtactc ctggtccacc aggccctgct     300
   ggacctcgag ggcacccagg tccttctgga ccaccaggaa aaccaggcta cggaagtcct     360
   ggactccaag gagagccagg gttgccagga ccaccgggac catcagctgt agggaaacca     420
   ggtgtgccag gactcccagg aaaaccagga gagagaggac catatggacc aaaaggagat     480
   gttggaccag ctggcctacc aggaccccgg ggcccaccag gaccacctgg aatccctgga     540
   ccggctggaa tttctgtgcc aggaaaacct ggacaacagg gacccacagg agccccagga     600
   cccaggggct ttcctggaga aaagggtgca ccaggagtcc ctggtatgaa tggacagaaa     660
   ggggaaatgg gatatggtgc tcctggtcgt ccaggtgaga ggggtcttcc aggccctcag     720
   ggtcccacag gaccatctgg ccctcctgga gtgggaaaaa gaggtgaaaa tggggttcca     780
   ggacagccag gcatcaaagg tgatagaggt tttccgggag aaatgggacc aattggccca     840
   ccaggtcccc aaggccctcc tggggaacga gggccagaag gcattggaaa gccaggagct     900
   gctggagccc caggccagcc agggattcca ggaacaaaag gtctccctgg ggctccagga     960
   atagctgggc ccccagggcc tcctggcttt gggaaaccag gcttgccagg cctgaaggga     1020
   gaaagaggac ctgctggcct tcctgggggt ccaggtgcca aaggggaaca agggccagca     1080
   ggtcttcctg ggaagccagg tctgactgga ccccctggga atatgggacc ccaaggacca     1140
   aaaggcatcc cgggtagcca tggtctccca ggccctaaag gtgagacagg gccagctggg     1200
   cctgcaggat accctggggc taagggtgaa aggggttccc ctgggtcaga tggaaaacca     1260
   gggtacccag gaaaaccagg tctcgatggt cctaagggta acccagggtt accaggtcca     1320
   aaaggtgatc ctggagttgg aggacctcct ggtctcccag gccctgtggg cccagcagga     1380
   gcaaagggaa tgcccggaca caatggagag gctggcccaa gaggtgcccc tggaatacca     1440
   ggtactagag gccctattgg gccaccaggc attccaggat tccctgggtc taaaggggat     1500
   ccaggaagtc ccggtcctcc tggcccagct ggcatagcaa ctaagggcct caatggaccc     1560
   accgggccac cagggcctcc aggtccaaga ggccactctg gagagcctgg tcttccaggg     1620
   ccccctgggc ctccaggccc accaggtcaa gcagtcatgc ctgagggttt tataaaggca     1680
   ggccaaaggc ccagtctttc tgggacccct cttgttagtg ccaaccaggg ggtaacagga     1740
   atgcctgtgt ctgcttttac tgttattctc tccaaagctt acccagcaat aggaactccc     1800
   ataccatttg ataaaatttt gtataacagg caacagcatt atgacccaag gactggaatc     1860
   tttacttgtc agataccagg aatatactat ttttcatacc acgtgcatgt gaaagggact     1920
   catgtttggg taggcctgta taagaatggc acccctgtaa tgtacaccta tgatgaatac     1980
   accaaaggct acctggatca ggcttcaggg agtgccatca tcgatctcac agaaaatgac     2040
   caggtgtggc tccagcttcc caatgccgag tcaaatggcc tatactcctc tgagtatgtc     2100
   cactcctctt tctcaggatt cctagtggct ccaatgtgag tacacacaga gctaatctaa     2160
   atcttgtgct agaaaaagca ttctctaact ctaccccacc ctacaaaatg catatggagg     2220
   taggctgaaa agaatgtaat ttttattttc tgaaatacag atttgagcta tcagaccaac     2280
   aaaccttccc cctgaaaagt gagcagcaac gtaaaaacgt atgtgaagcc tctcttgaat     2340
   ttctagttag caatcttaag gctctttaag gttttctcca atattaaaaa atatcaccaa     2400
   agaagtcctg ctatgttaaa aacaaacaac aaaaaacaaa caacaaaaaa aaaattaaaa     2460
   aaaaaaacag aaatagagct ctaagttatg tgaaatttga tttgagaaac tcggcatttc     2520
   ctttttaaaa aagcctgttt ctaactatga atatgagaac ttctaggaaa catccaggag     2580
   gtatcatata actttgtaga acttaaatac ttgaatattc aaatttaaaa gacactgtat     2640
   cccctaaaat atttctgatg gtgcactact ctgaggcctg tatggcccct ttcatcaata     2700
   tctattcaaa tatacaggtg catatatact tgttaaagct cttatataaa aaagccccaa     2760
   aatattgaag ttcatctgaa atgcaaggtg ctttcatcaa tgaacctttt caaacttttc     2820
   tatgattgca gagaagcttt ttatataccc agcataactt ggaaacaggt atctgaccta     2880
   ttcttattta gttaacacaa gtgtgattaa tttgatttct ttaattcctt attgaatctt     2940
   atgtgatatg attttctgga tttacagaac attagcacat gtaccttgtg cctcccattc     3000
   aagtgaagtt ataatttaca ctgagggttt caaaattcga ctagaagtgg agatatatta     3060
   tttatttatg cactgtactg tatttttata ttgctgttta aaacttttaa gctgtgcctc     3120
   acttattaaa gcacaaaatg ttttacctac tccttattta cgacgcaata aaataacatc     3180
   aatagatttt taggctgaat taatttgaaa gcagcaattt gctgttctca accattcttt     3240
   caaggctttt cattgttcaa agttaataaa aaagtaggac aataaagtga aaaaaaaaaa     3300
   aaaaaaa                                                               3307
<210> 8
   <211> 680
   <212> PRT
   <213> Homo sapiens
<400> 8
<210> 9
   <211> 2788
   <212> DNA
   <213> Homo sapiens
<220>
   <221> source
   <222> 1..2788
   <223> /mol_type="unassigned DNA"
   /organism="Homo sapiens"
<400> 9
   aagaacgccc ccaaaatctg tttctaattt tacagaaatc ttttgaaact tggcacggta 60
   ttcaaaagtc cgtggaaaga aaaaaacctt gtcctggctt cagcttccaa ctacaaagac     120
   agacttggtc cttttcaacg gttttcacag atccagtgac ccacgctctg aagacagaat     180
   tagctaactt tcaaaaacat ctggaaaaat gaagacttgg gtaaaaatcg tatttggagt     240
   tgccacctct gctgtgcttg ccttattggt gatgtgcatt gtcttacgcc cttcaagagt     300
   tcataactct gaagaaaata caatgagagc actcacactg aaggatattt taaatggaac     360
   attttcttat aaaacatttt ttccaaactg gatttcagga caagaatatc ttcatcaatc      420
   tgcagataac aatatagtac tttataatat tgaaacagga caatcatata ccattttgag     480
   taatagaacc atgaaaagtg tgaatgcttc aaattacggc ttatcacctg atcggcaatt     540
   tgtatatcta gaaagtgatt attcaaagct ttggagatac tcttacacag caacatatta     600
   catctatgac cttagcaatg gagaatttgt aagaggaaat gagcttcctc gtccaattca     660
   gtatttatgc tggtcgcctg ttgggagtaa attagcatat gtctatcaaa acaatatcta     720
   tttgaaacaa agaccaggag atccaccttt tcaaataaca tttaatggaa gagaaaataa     780
   aatatttaat ggaatcccag actgggttta tgaagaggaa atgcttgcta caaaatatgc     840
   tctctggtgg tctcctaatg gaaaattttt ggcatatgcg gaatttaatg atacggatat     900
   accagttatt gcctattcct attatggcga tgaacaatat cctagaacaa taaatattcc     960
   atacccaaag gctggagcta agaatcccgt tgttcggata tttattatcg ataccactta    1020
   ccctgcgtat gtaggtcccc aggaagtgcc tgttccagca atgatagcct caagtgatta    1080
   ttatttcagt tggctcacgt gggttactga tgaacgagta tgtttgcagt ggctaaaaag    1140
   agtccagaat gtttcggtcc tgtctatatg tgacttcagg gaagactggc agacatggga    1200
   ttgtccaaag acccaggagc atatagaaga aagcagaact ggatgggctg gtggattctt    1260
   tgtttcaaca ccagttttca gctatgatgc catttcgtac tacaaaatat ttagtgacaa    1320
   ggatggctac aaacatattc actatatcaa agacactgtg gaaaatgcta ttcaaattac    1380
   aagtggcaag tgggaggcca taaatatatt cagagtaaca caggattcac tgttttattc    1440
   tagcaatgaa tttgaagaat accctggaag aagaaacatc tacagaatta gcattggaag    1500
   ctatcctcca agcaagaagt gtgttacttg ccatctaagg aaagaaaggt gccaatatta    1560
   cacagcaagt ttcagcgact acgccaagta ctatgcactt gtctgctacg gcccaggcat    1620
   ccccatttcc acccttcatg atggacgcac tgatcaagaa attaaaatcc tggaagaaaa    1680
   caaggaattg gaaaatgctt tgaaaaatat ccagctgcct aaagaggaaa ttaagaaact    1740
   tgaagtagat gaaattactt tatggtacaa gatgattctt cctcctcaat ttgacagatc    1800
   aaagaagtat cccttgctaa ttcaagtgta tggtggtccc tgcagtcaga gtgtaaggtc    1860
   tgtatttgct gttaattgga tatcttatct tgcaagtaag gaagggatgg tcattgcctt    1920
   ggtggatggt cgaggaacag ctttccaagg tgacaaactc ctctatgcag tgtatcgaaa    1980
   gctgggtgtt tatgaagttg aagaccagat tacagctgtc agaaaattca tagaaatggg    2040
   tttcattgat gaaaaaagaa tagccatatg gggctggtcc tatggaggat acgtttcatc    2100
   actggccctt gcatctggaa ctggtctttt caaatgtggt atagcagtgg ctccagtctc    2160
   cagctgggaa tattacgcgt ctgtctacac agagagattc atgggtctcc caacaaagga    2220
   tgataatctt gagcactata agaattcaac tgtgatggca agagcagaat atttcagaaa    2280
   tgtagactat cttctcatcc acggaacagc agatgataat gtgcactttc aaaactcagc    2340
   acagattgct aaagctctgg ttaatgcaca agtggatttc caggcaatgt ggtactctga    2400
   ccagaaccac ggcttatccg gcctgtccac gaaccactta tacacccaca tgacccactt    2460
   cctaaagcag tgtttctctt tgtcagacta aaaacgatgc agatgcaagc ctgtatcaga    2520
   atctgaaaac cttatataaa cccctcagac agtttgctta ttttattttt tatgttgtaa    2580
   aatgctagta taaacaaaca aattaatgtt gttctaaagg ctgttaaaaa aaagatgagg    2640
   actcagaagt tcaagctaaa tattgtttac attttctggt actctgtgaa agaagagaaa    2700
   agggagtcat gcattttgct ttggacacag tgttttatca cctgttcatt tgaagaaaaa    2760
   taataaagtc agaagttcaa aaaaaaaa                                      2788
<210> 10
   <211> 760
   <212> PRT
   <213> Homo sapiens
<400> 10
   Met Lys Thr Trp Val Lys Ile Val Phe Gly Val Ala Thr Ser Ala Val
   1               5                   10                  15
   Leu Ala Leu Leu Val Met Cys Ile Val Leu Arg Pro Ser Arg Val His
               20                  25                  30
   Asn Ser Glu Glu Asn Thr Met Arg Ala Leu Thr Leu Lys Asp Ile Leu
           35                  40                  45
   Asn Gly Thr Phe Ser Tyr Lys Thr Phe Phe Pro Asn Trp Ile Ser Gly
       50                  55                  60
   Gln Glu Tyr Leu His Gln Ser Ala Asp Asn Asn Ile Val Leu Tyr Asn
   65                  70                  75                  80
   Ile Glu Thr Gly Gln Ser Tyr Thr Ile Leu Ser Asn Arg Thr Met Lys
                   85                  90                  95
   Ser Val Asn Ala Ser Asn Tyr Gly Leu Ser Pro Asp Arg Gln Phe Val
               100                 105                 110
   Tyr Leu Glu Ser Asp Tyr Ser Lys Leu Trp Arg Tyr Ser Tyr Thr Ala
           115                 120                 125
   Thr Tyr Tyr Ile Tyr Asp Leu Ser Asn Gly Glu Phe Val Arg Gly Asn
       130                 135                 140
   Glu Leu Pro Arg Pro Ile Gln Tyr Leu Cys Trp Ser Pro Val Gly Ser
   145                 150                 155                 160
   Lys Leu Ala Tyr Val Tyr Gln Asn Asn Ile Tyr Leu Lys Gln Arg Pro
                   165                 170                 175
   Gly Asp Pro Pro Phe Gln Ile Thr Phe Asn Gly Arg Glu Asn Lys Ile
               180                 185                 190
   Phe Asn Gly Ile Pro Asp Trp Val Tyr Glu Glu Glu Met Leu Ala Thr
           195                 200                 205
   Lys Tyr Ala Leu Trp Trp Ser Pro Asn Gly Lys Phe Leu Ala Tyr Ala
   210 215 220
   Glu Phe Asn Asp Thr Asp Ile Pro Val Ile Ala Tyr Ser Tyr Tyr Gly
   225 230 235 240
   Asp Glu Gln Tyr Pro Arg Thr Ile Asn Ile Pro Tyr Pro Lys Ala Gly
   245 250 255
   Ala Lys Asn Pro Val Val Arg Ile Phe Ile Ile Asp Thr Thr Tyr Pro
   260 265 270
   Ala Tyr Val Gly Pro Gln Glu Val Pro Val Pro Ala Met Ile Ala Ser
   275 280 285
   Ser Asp Tyr Tyr Phe Ser Trp Leu Thr Trp Val Thr Asp Glu Arg Val
   290 295 300
   Cys Leu Gln Trp Leu Lys Arg Val Gln Asn Val Ser Val Leu Ser Ile
   305 310 315 320
   Cys Asp Phe Arg Glu Asp Trp Gln Thr Trp Asp Cys Pro Lys Thr Gln
   325 330 335
   Glu His Ile Glu Glu Ser Arg Thr Gly Trp Ala Gly Gly Phe Phe Val
   340 345 350
   Ser Thr Pro Val Phe Ser Tyr Asp Ala Ile Ser Tyr Tyr Lys Ile Phe
   355 360 365
   Ser Asp Lys Asp Gly Tyr Lys His Ile His Tyr Ile Lys Asp Thr Val
   370 375 380
   Glu Asn Ala Ile Gln Ile Thr Ser Gly Lys Trp Glu Ala Ile Asn Ile
   385 390 395 400
   Phe Arg Val Thr Gln Asp Ser Leu Phe Tyr Ser Ser Asn Glu Phe Glu
   405 410 415
   Glu Tyr Pro Gly Arg Arg Asn Ile Tyr Arg Ile Ser Ile Gly Ser Tyr
   420 425 430
   Pro Pro Ser Lys Lys Cys Val Thr Cys His Leu Arg Lys Glu Arg Cys
   435 440 445
   Gln Tyr Tyr Thr Ala Ser Phe Ser Asp Tyr Ala Lys Tyr Tyr Ala Leu
   450 455 460
   Val Cys Tyr Gly Pro Gly Ile Pro Ile Ser Thr Leu His Asp Gly Arg
   465 470 475 480
   Thr Asp Gln Glu Ile Lys Ile Leu Glu Glu Asn Lys Glu Leu Glu Asn
   485 490 495
   Ala Leu Lys Asn Ile Gln Leu Pro Lys Glu Glu Ile Lys Lys Leu Glu
   500 505 510
   Val Asp Glu Ile Thr Leu Trp Tyr Lys Met Ile Leu Pro Pro Gln Phe
   515 520 525
   Asp Arg Ser Lys Lys Tyr Pro Leu Leu Ile Gln Val Tyr Gly Gly Pro
   530 535 540
   Cys Ser Gln Ser Val Arg Ser Val Phe Ala Val Asn Trp Ile Ser Tyr
   545 550 555 560
   Leu Ala Ser Lys Glu Gly Met Val Ile Ala Leu Val Asp Gly Arg Gly
   565 570 575
   Thr Ala Phe Gln Gly Asp Lys Leu Leu Tyr Ala Val Tyr Arg Lys Leu
   580 585 590
   Gly Val Tyr Glu Val Glu Asp Gln Ile Thr Ala Val Arg Lys Phe Ile
   595 600 605
   Glu Met Gly Phe Ile Asp Glu Lys Arg Ile Ala Ile Trp Gly Trp Ser
   610 615 620
   Tyr Gly Gly Tyr Val Ser Ser Leu Ala Leu Ala Ser Gly Thr Gly Leu
   625 630 635 640
   Phe Lys Cys Gly Ile Ala Val Ala Pro Val Ser Ser Trp Glu Tyr Tyr
   645 650 655
   Ala Ser Val Tyr Thr Glu Arg Phe Met Gly Leu Pro Thr Lys Asp Asp
   660 665 670
   Asn Leu Glu His Tyr Lys Asn Ser Thr Val Met Ala Arg Ala Glu Tyr
   675 680 685
   Phe Arg Asn Val Asp Tyr Leu Leu Ile His Gly Thr Ala Asp Asp Asn
   690 695 700
   Val His Phe Gln Asn Ser Ala Gln Ile Ala Lys Ala Leu Val Asn Ala
   705                 710                 715                 720
   Gln Val Asp Phe Gln Ala Met Trp Tyr Ser Asp Gln Asn His Gly Leu
                   725                 730                 735
   Ser Gly Leu Ser Thr Asn His Leu Tyr Thr His Met Thr His Phe Leu
               740                 745                 750
   Lys Gln Cys Phe Ser Leu Ser Asp
           755                 760
<210> 11
   <211> 307
   <212> DNA
   <213> Homo sapiens
<220>
   <221> source
   <222> 1..307
   <223> /mol_type="unassigned DNA"
   /organism="Homo sapiens"
<400> 11
   tggtactgca cccatagata ctgtcacctc tactctttct tccaatcacc attagcagat     60
   gccacaggat tcctacttct gaaagttttt gggccctgca ggtggaagac tggagaagcc    120
   aataaagttt aaggctacat tttattccat ccacaaattt ggtgaaggag gaaatgttta    180
   caattctgcc atgccatgaa taggagtttt ccaccgggtg tacactgctg ttaacaaggt    240
   gtaaatactt gtccagtaaa gagaccgtac ggtactgctg atggacgtcc caacacaatg    300
   ccagatg                                                              307
<210> 12
   <211> 2541
   <212> DNA
   <213> Homo sapiens
<220>
   <221> source
   <222> 1..2541
   <223> /mol_type="unassigned DNA"
   /organism="Homo sapiens"
<400> 12
   gatacaaaca gtgatggaaa acacatgagc agtaacaagt tttaatcttg ctcctcagta      60
   ctaacatgga ctaatctgtg ggagcagttt attccagtat cacccagggt gcagccacac     120
   caggactgtg ttgaagggtg ttttttttct tttaaatgta atacctcctc atcttttctt      180
   cttacacagt gtctgagaac atttacatta tagataagta gtacatggtg gataacttct     240
   acttttagga ggactactct cttctgacag tcctagactg gtcttctaca ctaagacacc     300
   atgaaggagt atgtgctcct attattcctg gctttgtgct ctgccaaacc cttctttagc     360
   ccttcacaca tcgcactgaa gaatatgatg ctgaaggata tggaagacac agatgatgat     420
   gatgatgatg atgatgatga tgatgatgat gaggacaact ctctttttcc aacaagagag     480
   ccaagaagcc atttttttcc atttgatctg tttccaatgt gtccatttgg atgtcagtgc      540
   tattcacgag ttgtacattg ctcagattta ggtttgacct cagtcccaac caacattcca 600
   tttgatactc gaatgcttga tcttcaaaac aataaaatta aggaaatcaa agaaaatgat 660
   tttaaaggac tcacttcact ttatggtctg atcctgaaca acaacaagct aacgaagatt 720
   cacccaaaag cctttctaac cacaaagaag ttgcgaaggc tgtatctgtc ccacaatcaa 780
   ctaagtgaaa taccacttaa tcttcccaaa tcattagcag aactcagaat tcatgaaaat 840
   aaagttaaga aaatacaaaa ggacacattc aaaggaatga atgctttaca cgttttggaa 900
   atgagtgcaa accctcttga taataatggg atagagccag gggcatttga aggggtgacg 960
   gtgttccata tcagaattgc agaagcaaaa ctgacctcag ttcctaaagg cttaccacca 1020
   actttattgg agcttcactt agattataat aaaatttcaa cagtggaact tgaggatttt 1080
   aaacgataca aagaactaca aaggctgggc ctaggaaaca acaaaatcac agatatcgaa 1140
   aatgggagtc ttgctaacat accacgtgtg agagaaatac atttggaaaa caataaacta 1200
   aaaaaaatcc cttcaggatt accagagttg aaatacctcc agataatctt ccttcattct 1260
   aattcaattg caagagtggg agtaaatgac ttctgtccaa cagtgccaaa gatgaagaaa 1320
   tctttataca gtgcaataag tttattcaac aacccggtga aatactggga aatgcaacct 1380
   gcaacatttc gttgtgtttt gagcagaatg agtgttcagc ttgggaactt tggaatgtaa 1440
   taattagtaa ttggtaatgt ccatttaata taagattcaa aaatccctac atttggaata 1500
   cttgaactct attaataatg gtagtattat atatacaagc aaatatctat tctcaagtgg 1560
   taagtccact gacttatttt atgacaagaa atttcaacgg aattttgcca aactattgat 1620
   acataagggt tgagagaaac aagcatctat tgcagtttct ttttgcgtac aaatgatctt 1680
   acataaatct catgcttgac cattcctttc ttcataacaa aaaagtaaga tattcggtat 1740
   ttaacacttt gttatcaagc atattttaaa aagaactgta ctgtaaatgg aatgcttgac 1800
   ttagcaaaat ttgtgctctt tcatttgctg ttagaaaaac agaattaaca aagacagtaa 1860
   tgtgaagagt gcattacact attcttattc tttagtaact tgggtagtac tgtaatattt 1920
   ttaatcatct taaagtatga tttgatataa tcttattgaa attaccttat catgtcttag 1980
   agcccgtctt tatgtttaaa actaatttct taaaataaag ccttcagtaa atgttcatta 2040
   ccaacttgat aaatgctact cataagagct ggtttggggc tatagcatat gctttttttt 2100
   ttttaattat tacctgattt aaaaatctct gtaaaaacgt gtagtgtttc ataaaatctg 2160
   taactcgcat tttaatgatc cgctattata agcttttaat agcatgaaaa ttgttaggct 2220
   atataacatt gccacttcaa ctctaaggaa tatttttgag atatcccttt ggaagacctt 2280
   gcttggaaga gcctggacac taacaattct acaccaaatt gtctcttcaa atacgtatgg 2340
   actggataac tctgagaaac acatctagta taactgaata agcagagcat caaattaaac 2400
   agacagaaac cgaaagctct atataaatgc tcagagttct ttatgtattt cttattggca 2460
   ttcaacatat gtaaaatcag aaaacaggga aattttcatt aaaaatattg gtttgaaata 2520
   aaaaaaaaaa aaaaaaaaaa a 2541
<210> 13
   <211> 379
   <212> PRT
   <213> Homo sapiens
<400> 13
   Met Lys Glu Tyr Val Leu Leu Leu Phe Leu Ala Leu Cys Ser Ala Lys
   1               5                   10                  15
   Pro Phe Phe Ser Pro Ser His Ile Ala Leu Lys Asn Met Met Leu Lys
               20                  25                  30
   Asp Met Glu Asp Thr Asp Asp Asp Asp Asp Asp Asp Asp Asp Asp Asp
           35                  40                  45
   Asp Asp Glu Asp Asn Ser Leu Phe Pro Thr Arg Glu Pro Arg Ser His
       50                  55                  60
   Phe Phe Pro Phe Asp Leu Phe Pro Met Cys Pro Phe Gly Cys Gln Cys
   65                  70                  75                  80
   Tyr Ser Arg Val Val His Cys Ser Asp Leu Gly Leu Thr Ser Val Pro
                   85                  90                  95
   Thr Asn Ile Pro Phe Asp Thr Arg Met Leu Asp Leu Gln Asn Asn Lys
               100                 105                 110
   Ile Lys Glu Ile Lys Glu Asn Asp Phe Lys Gly Leu Thr Ser Leu Tyr
           115                 120                 125
   Gly Leu Ile Leu Asn Asn Asn Lys Leu Thr Lys Ile His Pro Lys Ala
       130                 135                 140
   Phe Leu Thr Thr Lys Lys Leu Arg Arg Leu Tyr Leu Ser His Asn Gln
   145                 150                 155                 160
   Leu Ser Glu Ile Pro Leu Asn Leu Pro Lys Ser Leu Ala Glu Leu Arg
                   165                 170                 175
   Ile His Glu Asn Lys Val Lys Lys Ile Gln Lys Asp Thr Phe Lys Gly
               180                 185                 190
   Met Asn Ala Leu His Val Leu Glu Met Ser Ala Asn Pro Leu Asp Asn
           195                 200                 205
   Asn Gly Ile Glu Pro Gly Ala Phe Glu Gly Val Thr Val Phe His Ile
       210                 215                 220
   Arg Ile Ala Glu Ala Lys Leu Thr Ser Val Pro Lys Gly Leu Pro Pro
   225                 230                 235                 240
   Thr Leu Leu Glu Leu His Leu Asp Tyr Asn Lys Ile Ser Thr Val Glu
                   245                 250                 255
   Leu Glu Asp Phe Lys Arg Tyr Lys Glu Leu Gln Arg Leu Gly Leu Gly
               260                 265                 270
   Asn Asn Lys Ile Thr Asp Ile Glu Asn Gly Ser Leu Ala Asn Ile Pro
           275                 280                 285
   Arg Val Arg Glu Ile His Leu Glu Asn Asn Lys Leu Lys Lys Ile Pro
       290                 295                 300
   Ser Gly Leu Pro Glu Leu Lys Tyr Leu Gln Ile Ile Phe Leu His Ser
   305                 310                 315                 320
   Asn Ser Ile Ala Arg Val Gly Val Asn Asp Phe Cys Pro Thr Val Pro
                   325                 330                 335
   Lys Met Lys Lys Ser Leu Tyr Ser Ala Ile Ser Leu Phe Asn Asn Pro
               340                 345                 350
   Val Lys Tyr Trp Glu Met Gln Pro Ala Thr Phe Arg Cys Val Leu Ser
           355                 360                 365
   Arg Met Ser Val Gln Leu Gly Asn Phe Gly Met
       370                 375
<210> 14
   <211> 608
   <212> DNA
   <213> Homo sapiens
<220>
   <221> source
   <222> 1..608
   <223> /mol_type="unassigned DNA"
   /organism="Homo sapiens"
<400> 14
   agaggtataa aaatcagata gctcagcact tcgctccaaa ggagctgaag gagagaggat      60
   tccactgcgt gtatgttttc tctttcacgt ctcacttttg ttctctgcca tcaggaaatg     120
   cagtagacat ttgtcaccag cagctctttg gagacttcaa tgttccttcc tcccaggatc     180
   cgaaagggga cttcagacac ggagaatcta agacaccagg agctcagaag aatggagtct     240
   caagacatca ggcaaatacc gaagagatca gagccaagtt ctaaagggca gccatttccc     300
   aaatgtcaac caagagcgaa gccaagagaa ttatgatttc ctggctatac aatgacagga     360
   aaagcttgat caaagctgga gtgcaagtta aaaagtgtaa ggtactcaag tgctagggtc     420
   tgtgtccaag cccaatcaat tgatcattag ttgtttttaa cttcaacgtt tcttttgcaa     480
   cagcacctct tccttaaagt agcaatctta gcagactgcc accttacatg atgcatctta     540
   ttctccaagg acaatgaaga ctgaactatc gcacattacc taagaaagat gggaattgac     600
   atgcacat                                                              608
<210> 15
   <211> 4955
   <212> DNA
   <213> Homo sapiens
<220>
   <221> source
   <222> 1..4955
   <223> /mol_type="unassigned DNA"
   /organism="Homo sapiens"
<400> 15
   gcttaaaaaa gcacagggag atcgcgggca gctttgcagt cgctgccttc tcgcgcctga      60
   ccatgcaccc ctgcatcttc ctgctgggcc acaggcgagc gctttatttc tggagctgag     120
   ggctaaaact tttttgactt ttcttctcct caacatctga atcatgccat gtgcccagag     180
   gagctggctt gcaaaccttt ccgtggtggc tcagctcctt aactttgggg cgctttgcta     240
   tgggagacag cctcagccag gcccggttcg cttcccggac aggaggcaag agcattttat     300
   caagggcctg ccagaatacc acgtggtggg tccagtccga gtagatgcca gtgggcattt     360
   tttgtcatat ggcttgcact atcccatcac gagcagcagg aggaagagag atttggatgg     420
   ctcagaggac tgggtgtact acagaatttc tcacgaggag aaggacctgt tttttaactt     480
   gacggtcaat caaggatttc tttccaatag ctacatcatg gagaagagat atgggaacct     540
   ctcccatgtt aagatgatgg cttcctctgc ccccctctgc catctcagtg gcacggttct      600
   acagcagggc accagagttg ggacggcagc cctcagtgcc tgccatggac tgactggatt      660
   tttccaacta ccacatggag actttttcat tgaacccgtg aagaagcatc cactggttga      720
   gggagggtac cacccgcaca tcgtttacag gaggcagaaa gttccagaaa ccaaggagcc      780
   aacctgtgga ttaaaggaca gtgttaacat ctcccagaag caagagctat ggcgggagaa      840
   gtgggagagg cacaacttgc caagcagaag cctctctcgg cgttccatca gcaaggagag      900
   atgggtggag acactggtgg tggccgacac aaagatgatt gaataccatg ggagtgagaa      960
   tgtggagtcc tacatcctca ccatcatgaa catggtcact gggttgttcc ataacccaag     1020
   cattggcaat gcaattcaca ttgttgtggt tcggctcatt ctactcgaag aagaagagca     1080
   aggactgaaa atagttcacc atgcagaaaa gacactgtct agcttctgca agtggcagaa     1140
   gagtatcaat cccaagagtg acctcaatcc tgttcatcac gacgtggctg tccttctcac     1200
   cagaaaggac atctgtgctg gtttcaatcg cccctgcgag accctgggcc tgtctcacct     1260
   ttcaggaatg tgtcagcctc accgcagttg taacatcaat gaagattcgg gactccctct     1320
   ggctttcaca attgcccatg agctaggaca cagcttcggc atccagcatg atgggaaaga     1380
   aaatgactgt gagcctgtgg gcagacatcc gtacatcatg tcccgccagc tccagtacga     1440
   tcccactccg ctgacatggt ccaagtgcag cgaggagtac atcacccgct tcttggaccg     1500
   aggctggggg ttctgtcttg atgacatacc taaaaagaaa ggcttgaagt ccaaggtcat     1560
   tgcccccgga gtgatctatg atgttcacca ccagtgccag ctacaatatg gacccaatgc     1620
   taccttctgc caggaagtag aaaacgtctg ccagacactg tggtgctccg tgaagggctt     1680
   ttgtcgctct aagctggacg ctgctgcaga tggaactcaa tgtggtgaga agaagtggtg     1740
   tatggcaggc aagtgcatca cagtggggaa gaaaccagag agcattcctg gaggctgggg     1800
   ccgctggtca ccctggtccc actgttccag gacctgtggg gctggagtcc agagcgcaga     1860
   gaggctctgc aacaaccccg agccaaagtt tggagggaaa tattgcactg gagaaagaaa     1920
   acgctatcgc ttgtgcaacg tccacccctg tcgctcagag gcaccaacat ttcggcagat     1980
   gcagtgcagt gaatttgaca ctgttcccta caagaatgaa ctctaccact ggtttcccat     2040
   ttttaaccca gcacatcctt gtgagctcta ctgccgaccc atagatggcc agttttctga     2100
   gaaaatgctg gatgctgtca ttgatggtac cccttgcttt gaaggcggca acagcagaaa     2160
   tgtctgtatt aatggcatat gtaagatggt tggctgtgac tatgagatcg attccaatgc     2220
   caccgaggat cgctgcggtg tgtgcctggg agatggctct tcctgccaga ctgtgagaaa     2280
   gatgtttaag cagaaggaag gatctggtta tgttgacatt gggctcattc caaaaggagc     2340
   aagggacata agagtgatgg aaattgaggg agctggaaac ttcctggcca tcaggagtga     2400
   agatcctgaa aaatattacc tgaatggagg gtttattatc cagtggaacg ggaactataa 2460
   gctggcaggg actgtctttc agtatgacag gaaaggagac ctggaaaagc tgatggccac 2520
   aggtcccacc aatgagtctg tgtggatcca gcttctattc caggtgacta accctggcat 2580
   caagtatgag tacacaatcc agaaagatgg ccttgacaat gatgttgagc agcagatgta 2640
   cttctggcag tacggccact ggacagagtg cagtgtgacc tgcgggacag gtatccgccg 2700
   ccaaactgcc cattgcataa agaagggccg cgggatggtg aaagctacat tctgtgaccc 2760
   agaaacacag cccaatggga gacagaagaa gtgccatgaa aaggcttgtc cacccaggtg 2820
   gtgggcaggg gagtgggaag catgctcggc gacatgcggg ccccacgggg agaagaagcg 2880
   aaccgtgctg tgcatccaga ccatggtctc tgacgagcag gctctcccgc ccacagactg 2940
   ccagcacctg ctgaagccca agaccctcct ttcctgcaac agagacatcc tgtgcccctc 3000
   ggactggaca gtgggcaact ggagtgagtg ttctgtttcc tgtggtggtg gagtgcggat 3060
   tcgcagtgtc acatgtgcca agaaccatga tgaaccttgc gatgtgacaa ggaaacccaa 3120
   cagccgagct ctgtgtggcc tccagcaatg cccttctagc cggagagttc tgaaaccaaa 3180
   caaaggcact atttccaatg gaaaaaaccc accaacacta aagcccgtcc ctccacctac 3240
   atccaggccc agaatgctga ccacacccac agggcctgag tctatgagca caagcactcc 3300
   agcaatcagc agccctagtc ctaccacagc ctccaaagaa ggagacctgg gtgggaaaca 3360
   gtggcaagat agctcaaccc aacctgagct gagctctcgc tatctcattt ccactggaag 3420
   cacttcccag cccatcctca cttcccaatc cttgagcatt cagccaagtg aggaaaatgt 3480
   ttccagttca gatactggtc ctacctcgga gggaggcctt gtagctacaa caacaagtgg 3540
   ttctggcttg tcatcttccc gcaaccctat cacttggcct gtgactccat tttacaatac 3600
   cttgaccaaa ggtccagaaa tggagattca cagtggctca ggggaagaaa gagaacagcc 3660
   tgaggacaaa gatgaaagca atcctgtaat atggaccaag atcagagtac ctggaaatga 3720
   cgctccagtg gaaagtacag aaatgccact tgcacctcca ctaacaccag atctcagcag 3780
   ggagtcctgg tggccaccct tcagcacagt aatggaagga ctgctcccca gccaaaggcc 3840
   cactacttcc gaaactggga cacccagagt tgaggggatg gttactgaaa agccagccaa 3900
   cactctgctc cctctgggag gagaccacca gccagaaccc tcaggaaaga cggcaaaccg 3960
   taaccacctg aaacttccaa acaacatgaa ccaaacaaaa agttctgaac cagtcctgac 4020
   tgaggaggat gcaacaagtc tgattactga gggctttttg ctaaatgcct ccaattacaa 4080
   gcagctcaca aacggccacg gctctgcaca ctggatcgtc ggaaactgga gcgagtgctc 4140
   caccacatgt ggcctggggg cctactggag aagggtggag tgcagcaccc agatggattc 4200
   tgactgtgcg gccatccaga gacctgaccc tgcaaaaaga tgccacctcc gtccctgtgc 4260
   tggctggaaa gtgggaaact ggagcaagtg ctccagaaac tgcagtgggg gcttcaagat 4320
   acgcgagatt cagtgcgtgg acagccggga ccaccggaac ctgaggccat ttcactgcca 4380
   gttcctggcc ggcattcctc ccccattgag catgagctgt aacccggagc cctgtgaggc 4440
   gtggcaggtg gagccttgga gccagtgctc caggtcctgt ggaggtggag ttcaggagag 4500
   aggagtgttc tgtccaggag gcctctgtga ttggacaaaa agacccacat ccaccatgtc 4560
   ttgcaatgag cacctgtgct gtcactgggc cactgggaac tgggacctgt gttccacttc 4620
   ctgtggaggt ggctttcaga agaggactgt ccaatgtgtg ccctcagagg gcaataaaac 4680
   tgaagaccaa gaccaatgtc tatgtgatca caaacccaga cctccagaat tcaaaaaatg 4740
   caaccagcag gcctgcaaga aaagtgccga tttactttgc actaaggaca aactgtcagc 4800
   cagtttctgc cagacactga aagccatgaa gaaatgttct gtgcccaccg tgagggctga 4860
   gtgctgcttc tcgtgtcccc agacacacat cacacacacc caaaggcaaa gaaggcaacg 4920
   gttgctccaa aagtcaaaag aactctaagc ccaaa 4955
<210> 16
   <211> 1594
   <212> PRT
   <213> Homo sapiens
<400> 16
   Met Pro Cys Ala Gln Arg Ser Trp Leu Ala Asn Leu Ser Val Val Ala
   1 5 10 15
   Gln Leu Leu Asn Phe Gly Ala Leu Cys Tyr Gly Arg Gln Pro Gln Pro
   20 25 30
   Gly Pro Val Arg Phe Pro Asp Arg Arg Gln Glu His Phe Ile Lys Gly
   35 40 45
   Leu Pro Glu Tyr His Val Val Gly Pro Val Arg Val Asp Ala Ser Gly
   50 55 60
   His Phe Leu Ser Tyr Gly Leu His Tyr Pro Ile Thr Ser Ser Arg Arg
   65 70 75 80
   Lys Arg Asp Leu Asp Gly Ser Glu Asp Trp Val Tyr Tyr Arg Ile Ser
   85 90 95
   His Glu Glu Lys Asp Leu Phe Phe Asn Leu Thr Val Asn Gln Gly Phe
   100 105 110
   Leu Ser Asn Ser Tyr Ile Met Glu Lys Arg Tyr Gly Asn Leu Ser His
   115 120 125
   Val Lys Met Met Ala Ser Ser Ala Pro Leu Cys His Leu Ser Gly Thr
   130 135 140
   Val Leu Gln Gln Gly Thr Arg Val Gly Thr Ala Ala Leu Ser Ala Cys
   145 150 155 160
   His Gly Leu Thr Gly Phe Phe Gln Leu Pro His Gly Asp Phe Phe Ile
   165 170 175
   Glu Pro Val Lys Lys His Pro Leu Val Glu Gly Gly Tyr His Pro His
   180 185 190
   Ile Val Tyr Arg Arg Gln Lys Val Pro Glu Thr Lys Glu Pro Thr Cys
   195 200 205
   Gly Leu Lys Asp Ser Val Asn Ile Ser Gln Lys Gln Glu Leu Trp Arg
   210 215 220
   Glu Lys Trp Glu Arg His Asn Leu Pro Ser Arg Ser Leu Ser Arg Arg
   225 230 235 240
   Ser Ile Ser Lys Glu Arg Trp Val Glu Thr Leu Val Val Ala Asp Thr
   245 250 255
   Lys Met Ile Glu Tyr His Gly Ser Glu Asn Val Glu Ser Tyr Ile Leu
   260 265 270
   Thr Ile Met Asn Met Val Thr Gly Leu Phe His Asn Pro Ser Ile Gly
   275 280 285
   Asn Ala Ile His Ile Val Val Val Arg Leu Ile Leu Leu Glu Glu Glu
   290 295 300
   Glu Gln Gly Leu Lys Ile Val His His Ala Glu Lys Thr Leu Ser Ser
   305 310 315 320
   Phe Cys Lys Trp Gln Lys Ser Ile Asn Pro Lys Ser Asp Leu Asn Pro
   325 330 335
   Val His His Asp Val Ala Val Leu Leu Thr Arg Lys Asp Ile Cys Ala
   340 345 350
   Gly Phe Asn Arg Pro Cys Glu Thr Leu Gly Leu Ser His Leu Ser Gly
   355 360 365
   Met Cys Gln Pro His Arg Ser Cys Asn Ile Asn Glu Asp Ser Gly Leu
   370 375 380
   Pro Leu Ala Phe Thr Ile Ala His Glu Leu Gly His Ser Phe Gly Ile
   385 390 395 400
   Gln His Asp Gly Lys Glu Asn Asp Cys Glu Pro Val Gly Arg His Pro
   405 410 415
   Tyr Ile Met Ser Arg Gln Leu Gln Tyr Asp Pro Thr Pro Leu Thr Trp
   420 425 430
   Ser Lys Cys Ser Glu Glu Tyr Ile Thr Arg Phe Leu Asp Arg Gly Trp
   435 440 445
   Gly Phe Cys Leu Asp Asp Ile Pro Lys Lys Lys Gly Leu Lys Ser Lys
   450 455 460
   Val Ile Ala Pro Gly Val Ile Tyr Asp Val His His Gln Cys Gln Leu
   465 470 475 480
   Gln Tyr Gly Pro Asn Ala Thr Phe Cys Gln Glu Val Glu Asn Val Cys
   485 490 495
   Gln Thr Leu Trp Cys Ser Val Lys Gly Phe Cys Arg Ser Lys Leu Asp
   500 505 510
   Ala Ala Ala Asp Gly Thr Gln Cys Gly Glu Lys Lys Trp Cys Met Ala
   515 520 525
   Gly Lys Cys Ile Thr Val Gly Lys Lys Pro Glu Ser Ile Pro Gly Gly
   530 535 540
   Trp Gly Arg Trp Ser Pro Trp Ser His Cys Ser Arg Thr Cys Gly Ala
   545 550 555 560
   Gly Val Gln Ser Ala Glu Arg Leu Cys Asn Asn Pro Glu Pro Lys Phe
   565 570 575
   Gly Gly Lys Tyr Cys Thr Gly Glu Arg Lys Arg Tyr Arg Leu Cys Asn
   580 585 590
   Val His Pro Cys Arg Ser Glu Ala Pro Thr Phe Arg Gln Met Gln Cys
   595 600 605
   Ser Glu Phe Asp Thr Val Pro Tyr Lys Asn Glu Leu Tyr His Trp Phe
   610 615 620
   Pro Ile Phe Asn Pro Ala His Pro Cys Glu Leu Tyr Cys Arg Pro Ile
   625 630 635 640
   Asp Gly Gln Phe Ser Glu Lys Met Leu Asp Ala Val Ile Asp Gly Thr
   645 650 655
   Pro Cys Phe Glu Gly Gly Asn Ser Arg Asn Val Cys Ile Asn Gly Ile
   660 665 670
   Cys Lys Met Val Gly Cys Asp Tyr Glu Ile Asp Ser Asn Ala Thr Glu
   675 680 685
   Asp Arg Cys Gly Val Cys Leu Gly Asp Gly Ser Ser Cys Gln Thr Val
   690 695 700
   Arg Lys Met Phe Lys Gln Lys Glu Gly Ser Gly Tyr Val Asp Ile Gly
   705 710 715 720
   Leu Ile Pro Lys Gly Ala Arg Asp Ile Arg Val Met Glu Ile Glu Gly
   725 730 735
   Ala Gly Asn Phe Leu Ala Ile Arg Ser Glu Asp Pro Glu Lys Tyr Tyr
   740 745 750
   Leu Asn Gly Gly Phe Ile Ile Gln Trp Asn Gly Asn Tyr Lys Leu Ala
   755 760 765
   Gly Thr Val Phe Gln Tyr Asp Arg Lys Gly Asp Leu Glu Lys Leu Met
   770 775 780
   Ala Thr Gly Pro Thr Asn Glu Ser Val Trp Ile Gln Leu Leu Phe Gln
   785 790 795 800
   Val Thr Asn Pro Gly Ile Lys Tyr Glu Tyr Thr Ile Gln Lys Asp Gly
   805 810 815
   Leu Asp Asn Asp Val Glu Gln Gln Met Tyr Phe Trp Gln Tyr Gly His
   820 825 830
   Trp Thr Glu Cys Ser Val Thr Cys Gly Thr Gly Ile Arg Arg Gln Thr
   835 840 845
   Ala His Cys Ile Lys Lys Gly Arg Gly Met Val Lys Ala Thr Phe Cys
   850 855 860
   Asp Pro Glu Thr Gln Pro Asn Gly Arg Gln Lys Lys Cys His Glu Lys
   865 870 875 880
   Ala Cys Pro Pro Arg Trp Trp Ala Gly Glu Trp Glu Ala Cys Ser Ala
   885 890 895
   Thr Cys Gly Pro His Gly Glu Lys Lys Arg Thr Val Leu Cys Ile Gln
   900 905 910
   Thr Met Val Ser Asp Glu Gln Ala Leu Pro Pro Thr Asp Cys Gln His
   915 920 925
   Leu Leu Lys Pro Lys Thr Leu Leu Ser Cys Asn Arg Asp Ile Leu Cys
   930 935 940
   Pro Ser Asp Trp Thr Val Gly Asn Trp Ser Glu Cys Ser Val Ser Cys
   945 950 955 960
   Gly Gly Gly Val Arg Ile Arg Ser Val Thr Cys Ala Lys Asn His Asp
   965 970 975
   Glu Pro Cys Asp Val Thr Arg Lys Pro Asn Ser Arg Ala Leu Cys Gly
   980 985 990
   Leu Gln Gln Cys Pro Ser Ser Arg Arg Val Leu Lys Pro Asn Lys Gly
   995 1000 1005
   Thr Ile Ser Asn Gly Lys Asn Pro Pro Thr Leu Lys Pro Val Pro Pro
   1010 1015 1020
   Pro Thr Ser Arg Pro Arg Met Leu Thr Thr Pro Thr Gly Pro Glu Ser
   1025 1030 1035 1040
   Met Ser Thr Ser Thr Pro Ala Ile Ser Ser Pro Ser Pro Thr Thr Ala
   1045 1050 1055
   Ser Lys Glu Gly Asp Leu Gly Gly Lys Gln Trp Gln Asp Ser Ser Thr
   1060 1065 1070
   Gln Pro Glu Leu Ser Ser Arg Tyr Leu Ile Ser Thr Gly Ser Thr Ser
   1075 1080 1085
   Gln Pro Ile Leu Thr Ser Gln Ser Leu Ser Ile Gln Pro Ser Glu Glu
   1090 1095 1100
   Asn Val Ser Ser Ser Asp Thr Gly Pro Thr Ser Glu Gly Gly Leu Val
   1105 1110 1115 1120
   Ala Thr Thr Thr Ser Gly Ser Gly Leu Ser Ser Ser Arg Asn Pro Ile
   1125 1130 1135
   Thr Trp Pro Val Thr Pro Phe Tyr Asn Thr Leu Thr Lys Gly Pro Glu
   1140 1145 1150
   Met Glu Ile His Ser Gly Ser Gly Glu Glu Arg Glu Gln Pro Glu Asp
   1155 1160 1165
   Lys Asp Glu Ser Asn Pro Val Ile Trp Thr Lys Ile Arg Val Pro Gly
   1170 1175 1180
   Asn Asp Ala Pro Val Glu Ser Thr Glu Met Pro Leu Ala Pro Pro Leu
   1185 1190 1195 1200
   Thr Pro Asp Leu Ser Arg Glu Ser Trp Trp Pro Pro Phe Ser Thr Val
   1205 1210 1215
   Met Glu Gly Leu Leu Pro Ser Gln Arg Pro Thr Thr Ser Glu Thr Gly
   1220 1225 1230
   Thr Pro Arg Val Glu Gly Met Val Thr Glu Lys Pro Ala Asn Thr Leu
   1235 1240 1245
   Leu Pro Leu Gly Gly Asp His Gln Pro Glu Pro Ser Gly Lys Thr Ala
   1250 1255 1260
   Asn Arg Asn His Leu Lys Leu Pro Asn Asn Met Asn Gln Thr Lys Ser
   1265                1270                1275                1280
   Ser Glu Pro Val Leu Thr Glu Glu Asp Ala Thr Ser Leu Ile Thr Glu
                   1285                1290                1295
   Gly Phe Leu Leu Asn Ala Ser Asn Tyr Lys Gln Leu Thr Asn Gly His
               1300                1305                1310
   Gly Ser Ala His Trp Ile Val Gly Asn Trp Ser Glu Cys Ser Thr Thr
           1315                1320                1325
   Cys Gly Leu Gly Ala Tyr Trp Arg Arg Val Glu Cys Ser Thr Gln Met
       1330                1335                1340
   Asp Ser Asp Cys Ala Ala Ile Gln Arg Pro Asp Pro Ala Lys Arg Cys
   1345                1350                1355                1360
   His Leu Arg Pro Cys Ala Gly Trp Lys Val Gly Asn Trp Ser Lys Cys
                   1365                1370                1375
   Ser Arg Asn Cys Ser Gly Gly Phe Lys Ile Arg Glu Ile Gln Cys Val
               1380                1385                1390
   Asp Ser Arg Asp His Arg Asn Leu Arg Pro Phe His Cys Gln Phe Leu
           1395                1400                1405
   Ala Gly Ile Pro Pro Pro Leu Ser Met Ser Cys Asn Pro Glu Pro Cys
       1410                1415                1420
   Glu Ala Trp Gln Val Glu Pro Trp Ser Gln Cys Ser Arg Ser Cys Gly
   1425                1430                1435                1440
   Gly Gly Val Gln Glu Arg Gly Val Phe Cys Pro Gly Gly Leu Cys Asp
                   1445                1450                1455
   Trp Thr Lys Arg Pro Thr Ser Thr Met Ser Cys Asn Glu His Leu Cys
               1460                1465                1470
   Cys His Trp Ala Thr Gly Asn Trp Asp Leu Cys Ser Thr Ser Cys Gly
           1475                1480                1485
   Gly Gly Phe Gln Lys Arg Thr Val Gln Cys Val Pro Ser Glu Gly Asn
       1490                1495                1500
   Lys Thr Glu Asp Gln Asp Gln Cys Leu Cys Asp His Lys Pro Arg Pro
   1505                1510                1515                1520
   Pro Glu Phe Lys Lys Cys Asn Gln Gln Ala Cys Lys Lys Ser Ala Asp
                   1525                1530                1535
   Leu Leu Cys Thr Lys Asp Lys Leu Ser Ala Ser Phe Cys Gln Thr Leu
               1540                1545                1550
   Lys Ala Met Lys Lys Cys Ser Val Pro Thr Val Arg Ala Glu Cys Cys
           1555                1560                1565
   Phe Ser Cys Pro Gln Thr His Ile Thr His Thr Gln Arg Gln Arg Arg
       1570                1575                1580
   Gln Arg Leu Leu Gln Lys Ser Lys Glu Leu
   1585                1590
<210> 17
   <211> 3676
   <212> DNA
   <213> Homo sapiens
<220>
   <221> source
   <222> 1..3676
   <223> /mol_type="unassigned DNA"
   /organism="Homo sapiens"
<400> 17
   gagagacgag ggcagcggag gaggcgagga gcgccgggta ccgggccggg ggagccgcgg      60
   gctctcgggg aagagacgga tgatgaacaa gctttacatc gggaacctga gccccgccgt     120
   caccgccgac gacctccggc agctctttgg ggacaggaag ctgcccctgg cgggacaggt     180
   cctgctgaag tccggctacg ccttcgtgga ctaccccgac cagaactggg ccatccgcgc     240
   catcgagacc ctctcgggta aagtggaatt gcatgggaaa atcatggaag ttgattactc 300
   agtctctaaa aagctaagga gcaggaaaat tcagattcga aacatccctc ctcacctgca 360
   gtgggaggtg ttggatggac ttttggctca atatgggaca gtggagaatg tggaacaagt 420
   caacacagac acagaaaccg ccgttgtcaa cgtcacatat gcaacaagag aagaagcaaa 480
   aatagccatg gagaagctaa gcgggcatca gtttgagaac tactccttca agatttccta 540
   catcccggat gaagaggtga gctccccttc gccccctcag cgagcccagc gtggggacca 600
   ctcttcccgg gagcaaggcc acgcccctgg gggcacttct caggccagac agattgattt 660
   cccgctgcgg atcctggtcc ccacccagtt tgttggtgcc atcatcggaa aggagggctt 720
   gaccataaag aacatcacta agcagaccca gtcccgggta gatatccata gaaaagagaa 780
   ctctggagct gcagagaagc ctgtcaccat ccatgccacc ccagagggga cttctgaagc 840
   atgccgcatg attcttgaaa tcatgcagaa agaggcagat gagaccaaac tagccgaaga 900
   gattcctctg aaaatcttgg cacacaatgg cttggttgga agactgattg gaaaagaagg 960
   cagaaatttg aagaaaattg aacatgaaac agggaccaag ataacaatct catctttgca 1020
   ggatttgagc atatacaacc cggaaagaac catcactgtg aagggcacag ttgaggcctg 1080
   tgccagtgct gagatagaga ttatgaagaa gctgcgtgag gcctttgaaa atgatatgct 1140
   ggctgttaac caacaagcca atctgatccc agggttgaac ctcagcgcac ttggcatctt 1200
   ttcaacagga ctgtccgtgc tatctccacc agcagggccc cgcggagctc cccccgctgc 1260
   cccctaccac cccttcacta cccactccgg atacttctcc agcctgtacc cccatcacca 1320
   gtttggcccg ttcccgcatc atcactctta tccagagcag gagattgtga atctcttcat 1380
   cccaacccag gctgtgggcg ccatcatcgg gaagaagggg gcacacatca aacagctggc 1440
   gagattcgcc ggagcctcta tcaagattgc ccctgcggaa ggcccagacg tcagcgaaag 1500
   gatggtcatc atcaccgggc caccggaagc ccagttcaag gcccagggac ggatctttgg 1560
   gaaactgaaa gaggaaaact tctttaaccc caaagaagaa gtgaagctgg aagcgcatat 1620
   cagagtgccc tcttccacag ctggccgggt gattggcaaa ggtggcaaga ccgtgaacga 1680
   actgcagaac ttaaccagtg cagaagtcat cgtgcctcgt gaccaaacgc cagatgaaaa 1740
   tgaggaagtg atcgtcagaa ttatcgggca cttctttgct agccagactg cacagcgcaa 1800
   gatcagggaa attgtacaac aggtgaagca gcaggagcag aaataccctc agggagtcgc 1860
   ctcacagcgc agcaagtgag gctcccacag gcaccagcaa aacaacggat gaatgtagcc 1920
   cttccaacac ctgacagaat gagaccaaac gcagccagcc agatcgggag caaaccaaag 1980
   accatctgag gaatgagaag tctgcggagg cggccaggga ctctgccgag gccctgagaa 2040
   ccccaggggc cgaggagggg cggggaaggt cagccaggtt tgccagaacc accgagcccc 2100
   gcctcccgcc ccccagggct tctgcaggct tcagccatcc acttcaccat ccactcggat 2160
   ctctcctgaa ctcccacgac gctatccctt ttagttgaac taacataggt gaacgtgttc 2220
   aaagccaagc aaaatgcaca ccctttttct gtggcaaatc gtctctgtac atgtgtgtac 2280
   atattagaaa gggaagatgt taagatatgt ggcctgtggg ttacacaggg tgcctgcagc 2340
   ggtaatatat tttagaaata atatatcaaa taactcaact aactccaatt tttaatcaat 2400
   tattaatttt tttttctttt taaagagaaa gcaggctttt ctagacttta aagaataaag 2460
   tctttgggag gtctcacggt gtagagagga gctttgaggc cacccgcaca aaattcaccc 2520
   agagggaaat ctcgtcggaa ggacactcac ggcagttctg gatcacctgt gtatgtcaac 2580
   agaagggata ccgtctcctt gaagaggaaa ctctgtcact cctcatgcct gtctagctca 2640
   tacacccatt tctctttgct tcacaggttt taaactggtt ttttgcatac tgctatataa 2700
   ttctctgtct ctctctgttt atctctcccc tccctcccct ccccttcttc tccatctcca 2760
   ttcttttgaa tttcctcatc cctccatctc aatcccgtat ctacgcaccc cccccccccc 2820
   aggcaaagca gtgctctgag tatcacatca cacaaaagga acaaaagcga aacacacaaa 2880
   ccagcctcaa cttacacttg gttactcaaa agaacaagag tcaatggtac ttgtcctagc 2940
   gttttggaag aggaaaacag gaacccacca aaccaaccaa tcaaccaaac aaagaaaaaa 3000
   ttccacaatg aaagaatgta ttttgtcttt ttgcattttg gtgtataagc catcaatatt 3060
   cagcaaaatg attcctttct ttaaaaaaaa aaaatgtgga ggaaagtaga aatttaccaa 3120
   ggttgttggc ccagggcgtt aaattcacag atttttttaa cgagaaaaac acacagaaga 3180
   agctacctca ggtgttttta cctcagcacc ttgctcttgt gtttccctta gagattttgt 3240
   aaagctgata gttggagcat ttttttattt ttttaataaa aatgagttgg aaaaaaaata 3300
   agatatcaac tgccagcctg gagaaggtga cagtccaagt gtgcaacagc tgttctgaat 3360
   tgtcttccgc tagccaagaa cctatatggc cttcttttgg acaaaccttg aaaatgttta 3420
   tttaaaaaaa aaaaagatga caaagaaaaa cagagagaga gaatattgga gatgtcctga 3480
   attttaatag ggtacgcgcc attagggctt tttgcgctaa aggatgaaca tgtactggtt 3540
   tatgtggaca agccattata ccaccagact gcaatgccag tttcctctac tgcaaacagt 3600
   gttctgtgac aaaaaaaaaa aaaaaaaaaa gaaaaaaaaa gaaaaaacag aaatatatcc 3660
   agctaacaag aaaaaa 3676
<210> 18
   <211> 599
   <212> PRT
   <213> Homo sapiens
<400> 18
   Met Met Asn Lys Leu Tyr Ile Gly Asn Leu Ser Pro Ala Val Thr Ala
   1 5 10 15
   Asp Asp Leu Arg Gln Leu Phe Gly Asp Arg Lys Leu Pro Leu Ala Gly
               20                  25                  30
   Gln Val Leu Leu Lys Ser Gly Tyr Ala Phe Val Asp Tyr Pro Asp Gln
           35                  40                  45
   Asn Trp Ala Ile Arg Ala Ile Glu Thr Leu Ser Gly Lys Val Glu Leu
       50                  55                  60
   His Gly Lys Ile Met Glu Val Asp Tyr Ser Val Ser Lys Lys Leu Arg
   65                  70                  75                  80
   Ser Arg Lys Ile Gln Ile Arg Asn Ile Pro Pro His Leu Gln Trp Glu
                   85                  90                  95
   Val Leu Asp Gly Leu Leu Ala Gln Tyr Gly Thr Val Glu Asn Val Glu
               100                 105                 110
   Gln Val Asn Thr Asp Thr Glu Thr Ala Val Val Asn Val Thr Tyr Ala
           115                 120                 125
   Thr Arg Glu Glu Ala Lys Ile Ala Met Glu Lys Leu Ser Gly His Gln
       130                 135                 140
   Phe Glu Asn Tyr Ser Phe Lys Ile Ser Tyr Ile Pro Asp Glu Glu Val
   145                 150                 155                 160
   Ser Ser Pro Ser Pro Pro Gln Arg Ala Gln Arg Gly Asp His Ser Ser
                   165                 170                 175
   Arg Glu Gln Gly His Ala Pro Gly Gly Thr Ser Gln Ala Arg Gln Ile
               180                 185                 190
   Asp Phe Pro Leu Arg Ile Leu Val Pro Thr Gln Phe Val Gly Ala Ile
           195                 200                 205
   Ile Gly Lys Glu Gly Leu Thr Ile Lys Asn Ile Thr Lys Gln Thr Gln
       210                 215                 220
   Ser Arg Val Asp Ile His Arg Lys Glu Asn Ser Gly Ala Ala Glu Lys
   225                 230                 235                 240
   Pro Val Thr Ile His Ala Thr Pro Glu Gly Thr Ser Glu Ala Cys Arg
                   245                 250                 255
   Met Ile Leu Glu Ile Met Gln Lys Glu Ala Asp Glu Thr Lys Leu Ala
               260                 265                 270
   Glu Glu Ile Pro Leu Lys Ile Leu Ala His Asn Gly Leu Val Gly Arg
           275                 280                 285
   Leu Ile Gly Lys Glu Gly Arg Asn Leu Lys Lys Ile Glu His Glu Thr
       290                 295                 300
   Gly Thr Lys Ile Thr Ile Ser Ser Leu Gln Asp Leu Ser Ile Tyr Asn
   305                 310                 315                 320
   Pro Glu Arg Thr Ile Thr Val Lys Gly Thr Val Glu Ala Cys Ala Ser
                   325                 330                 335
   Ala Glu Ile Glu Ile Met Lys Lys Leu Arg Glu Ala Phe Glu Asn Asp
               340                 345                 350
   Met Leu Ala Val Asn Gln Gln Ala Asn Leu Ile Pro Gly Leu Asn Leu
           355                 360                 365
   Ser Ala Leu Gly Ile Phe Ser Thr Gly Leu Ser Val Leu Ser Pro Pro
       370                 375                 380
   Ala Gly Pro Arg Gly Ala Pro Pro Ala Ala Pro Tyr His Pro Phe Thr
   385                 390                 395                 400
   Thr His Ser Gly Tyr Phe Ser Ser Leu Tyr Pro His His Gln Phe Gly
                   405                 410                 415
   Pro Phe Pro His His His Ser Tyr Pro Glu Gln Glu Ile Val Asn Leu
               420                 425                 430
   Phe Ile Pro Thr Gln Ala Val Gly Ala Ile Ile Gly Lys Lys Gly Ala
           435                 440                 445
   His Ile Lys Gln Leu Ala Arg Phe Ala Gly Ala Ser Ile Lys Ile Ala
       450                 455                 460
   Pro Ala Glu Gly Pro Asp Val Ser Glu Arg Met Val Ile Ile Thr Gly
   465                 470                 475                 480
   Pro Pro Glu Ala Gln Phe Lys Ala Gln Gly Arg Ile Phe Gly Lys Leu
                   485                 490                 495
   Lys Glu Glu Asn Phe Phe Asn Pro Lys Glu Glu Val Lys Leu Glu Ala
               500                 505                 510
   His Ile Arg Val Pro Ser Ser Thr Ala Gly Arg Val Ile Gly Lys Gly
               515                 520                 525
   Gly Lys Thr Val Asn Glu Leu Gln Asn Leu Thr Ser Ala Glu Val Ile
           530                 535                 540
   Val Pro Arg Asp Gln Thr Pro Asp Glu Asn Glu Glu Val Ile Val Arg
   545                 550                 555                 560
   Ile Ile Gly His Phe Phe Ala Ser Gln Thr Ala Gln Arg Lys Ile Arg
                   565                 570                 575
   Glu Ile Val Gln Gln Val Lys Gln Gln Glu Gln Lys Tyr Pro Gln Gly
               580                 585                 590
   Val Ala Ser Gln Arg Ser Lys
           595
<210> 19
   <211> 2418
   <212> DNA
   <213> Homo sapiens
<220>
   <221> source
   <222> 1..2418
   <223> /mol_type="unassigned DNA"
   /organism="Homo sapiens"
<400> 19
   gcaaacctta agctgaatga acaacttttc ttctcttgaa tatatcttaa cgccaaattt      60
   tgagtgcttt tttgttaccc atcctcatat gtcccagcta gaaagaatcc tgggttggag     120
   ctactgcatg ttgattgttt tgtttttcct tttggctgtt cattttggtg gctactataa     180
   ggaaatctaa cacaaacagc aactgttttt tgttgtttac ttttgcatct ttacttgtgg     240
   agctgtggca agtcctcata tcaaatacag aacatgatct tcctcctgct aatgttgagc     300
   ctggaattgc agcttcacca gatagcagct ttattcacag tgacagtccc taaggaactg     360
   tacataatag agcatggcag caatgtgacc ctggaatgca actttgacac tggaagtcat     420
   gtgaaccttg gagcaataac agccagtttg caaaaggtgg aaaatgatac atccccacac     480
   cgtgaaagag ccactttgct ggaggagcag ctgcccctag ggaaggcctc gttccacata     540
   cctcaagtcc aagtgaggga cgaaggacag taccaatgca taatcatcta tggggtcgcc     600
   tgggactaca agtacctgac tctgaaagtc aaagcttcct acaggaaaat aaacactcac     660
   atcctaaagg ttccagaaac agatgaggta gagctcacct gccaggctac aggttatcct     720
   ctggcagaag tatcctggcc aaacgtcagc gttcctgcca acaccagcca ctccaggacc     780
   cctgaaggcc tctaccaggt caccagtgtt ctgcgcctaa agccaccccc tggcagaaac     840
   ttcagctgtg tgttctggaa tactcacgtg agggaactta ctttggccag cattgacctt     900
   caaagtcaga tggaacccag gacccatcca acttggctgc ttcacatttt catccccttc     960
   tgcatcattg ctttcatttt catagccaca gtgatagccc taagaaaaca actctgtcaa    1020
   aagctgtatt cttcaaaaga cacaacaaaa agacctgtca ccacaacaaa gagggaagtg    1080
   aacagtgcta tctgaacctg tggtcttggg agccagggtg acctgatatg acatctaaag    1140
   aagcttctgg actctgaaca agaattcggt ggcctgcaga gcttgccatt tgcacttttc    1200
   aaatgccttt ggatgaccca gcactttaat ctgaaacctg caacaagact agccaacacc 1260
   tggccatgaa acttgcccct tcactgatct ggactcacct ctggagccta tggctttaag 1320
   caagcactac tgcactttac agaattaccc cactggatcc tggacccaca gaattccttc 1380
   aggatccttc ttgctgccag actgaaagca aaaggaatta tttcccctca agttttctaa 1440
   gtgatttcca aaagcagagg tgtgtggaaa tttccagtaa cagaaacaga tgggttgcca 1500
   atagagttat tttttatcta tagcttcctc tgggtactag aagaggctat tgagactatg 1560
   agctcacaga cagggcttcg cacaaactca aatcataatt gacatgtttt atggattact 1620
   ggaatcttga tagcataatg aagttgttct aattaacaga gagcatttaa atatacacta 1680
   agtgcacaaa ttgtggagta aagtcatcaa gctctgtttt tgaggtctaa gtcacaaagc 1740
   atttgtttta acctgtaatg gcaccatgtt taatggtggt tttttttttg aactacatct 1800
   ttcctttaaa aattattggt ttctttttat ttgtttttac cttagaaatc aattatatac 1860
   agtcaaaaat atttgatatg ctcatacgtt gtatctgcag caatttcaga taagtagcta 1920
   aaatggccaa agccccaaac taagcctcct tttctggccc tcaatatgac tttaaatttg 1980
   acttttcagt gcctcagttt gcacatctgt aatacagcaa tgctaagtag tcaaggcctt 2040
   tgataattgg cactatggaa atcctgcaag atcccactac atatgtgtgg agcagaaggg 2100
   taactcggct acagtaacag cttaattttg ttaaatttgt tctttatact ggagccatga 2160
   agctcagagc attagctgac ccttgaacta ttcaaatggg cacattagct agtataacag 2220
   acttacatag gtgggcctaa agcaagctcc ttaactgagc aaaatttggg gcttatgaga 2280
   atgaaagggt gtgaaattga ctaacagaca aatcatacat ctcagtttct caattctcat 2340
   gtaaatcaga gaatgccttt aaagaataaa actcaattgt tattcttcaa cgttctttat 2400
   atattctact tttgggta 2418
<210> 20
   <211> 273
   <212> PRT
   <213> Homo sapiens
<400> 20
   Met Ile Phe Leu Leu Leu Met Leu Ser Leu Glu Leu Gln Leu His Gln
   1               5                   10                  15
   Ile Ala Ala Leu Phe Thr Val Thr Val Pro Lys Glu Leu Tyr Ile Ile
               20                  25                  30
   Glu His Gly Ser Asn Val Thr Leu Glu Cys Asn Phe Asp Thr Gly Ser
           35                  40                  45
   His Val Asn Leu Gly Ala Ile Thr Ala Ser Leu Gln Lys Val Glu Asn
       50                  55                  60
   Asp Thr Ser Pro His Arg Glu Arg Ala Thr Leu Leu Glu Glu Gln Leu
   65                  70                  75                  80
   Pro Leu Gly Lys Ala Ser Phe His Ile Pro Gln Val Gln Val Arg Asp
                   85                  90                  95
   Glu Gly Gln Tyr Gln Cys Ile Ile Ile Tyr Gly Val Ala Trp Asp Tyr
               100                 105                 110
   Lys Tyr Leu Thr Leu Lys Val Lys Ala Ser Tyr Arg Lys Ile Asn Thr
           115                 120                 125
   His Ile Leu Lys Val Pro Glu Thr Asp Glu Val Glu Leu Thr Cys Gln
       130                 135                 140
   Ala Thr Gly Tyr Pro Leu Ala Glu Val Ser Trp Pro Asn Val Ser Val
   145                 150                 155                 160
   Pro Ala Asn Thr Ser His Ser Arg Thr Pro Glu Gly Leu Tyr Gln Val
                   165                 170                 175
   Thr Ser Val Leu Arg Leu Lys Pro Pro Pro Gly Arg Asn Phe Ser Cys
               180                 185                 190
   Val Phe Trp Asn Thr His Val Arg Glu Leu Thr Leu Ala Ser Ile Asp
           195                 200                 205
   Leu Gln Ser Gln Met Glu Pro Arg Thr His Pro Thr Trp Leu Leu His
       210                 215                 220
   Ile Phe Ile Pro Phe Cys Ile Ile Ala Phe Ile Phe Ile Ala Thr Val
   225                 230                 235                 240
   Ile Ala Leu Arg Lys Gln Leu Cys Gln Lys Leu Tyr Ser Ser Lys Asp
                   245                 250                 255
   Thr Thr Lys Arg Pro Val Thr Thr Thr Lys Arg Glu Val Asn Ser Ala
               260                 265                 270
   Ile
<210> 21
   <211> 2974
   <212> DNA
   <213> Homo sapiens
<220>
   <221> source
   <222> 1..2974
   <223> /mol_type="unassigned DNA"
   /organism="Homo sapiens"
<400> 21
   gcggccgagg gggagcccgc gccgcggctg cagctgccaa gggagcgttc cgagcccacg      60
   tcaggggagg tgtcgggata aatagggtcc cgcaatggcc gtggctggct gcgctccgag     120
   ctgcggagtc cgggactgga gctgcccggg cgggttcgcg ccccgaaggc tgagagctgg     180
   cgctgctcgt gccctgtgtg ccagacggcg gagctccgcg gccggacccc gcggccccgc     240
   tttgctgccg actggagttt gggggaagaa actctcctgc gccccagagg atttcttcct     300
   cggcgaaggg acagcgaaag atgagggtgg caggaagaga agggcgcttt ctgtctgccg     360
   gggtcgcagc gcgagagggc agtgccatgt tcctctccat cctagtggcg ctgtgcctgt     420
   ggctgcacct ggcgctgggc gtgcgcggcg cgccctgcga ggcggtgcgc atccctatgt     480
   gccggcacat gccctggaac atcacgcgga tgcccaacca cctgcaccac agcacgcagg     540
   agaacgccat cctggccatc gagcagtacg aggagctggt ggacgtgaac tgcagcgccg     600
   tgctgcgctt cttcctctgt gccatgtacg cgcccatttg caccctggag ttcctgcacg     660
   accctatcaa gccgtgcaag tcggtgtgcc aacgcgcgcg cgacgactgc gagcccctca     720
   tgaagatgta caaccacagc tggcccgaaa gcctggcctg cgacgagctg cctgtctatg     780
   accgtggcgt gtgcatctcg cctgaagcca tcgtcacgga cctcccggag gatgttaagt 840
   ggatagacat cacaccagac atgatggtac aggaaaggcc tcttgatgtt gactgtaaac 900
   gcctaagccc cgatcggtgc aagtgtaaaa aggtgaagcc aactttggca acgtatctca 960
   gcaaaaacta cagctatgtt attcatgcca aaataaaagc tgtgcagagg agtggctgca 1020
   atgaggtcac aacggtggtg gatgtaaaag agatcttcaa gtcctcatca cccatccctc 1080
   gaactcaagt cccgctcatt acaaattctt cttgccagtg tccacacatc ctgccccatc 1140
   aagatgttct catcatgtgt tacgagtggc gctcaaggat gatgcttctt gaaaattgct 1200
   tagttgaaaa atggagagat cagcttagta aaagatccat acagtgggaa gagaggctgc 1260
   aggaacagcg gagaacagtt caggacaaga agaaaacagc cgggcgcacc agtcgtagta 1320
   atccccccaa accaaaggga aagcctcctg ctcccaaacc agccagtccc aagaagaaca 1380
   ttaaaactag gagtgcccag aagagaacaa acccgaaaag agtgtgagct aactagtttc 1440
   caaagcggag acttccgact tccttacagg atgaggctgg gcattgcctg ggacagccta 1500
   tgtaaggcca tgtgcccctt gccctaacaa ctcactgcag tgctcttcat agacacatct 1560
   tgcagcattt ttcttaaggc tatgcttcag tttttctttg taagccatca caagccatag 1620
   tggtaggttt gccctttggt acagaaggtg agttaaagct ggtggaaaag gcttattgca 1680
   ttgcattcag agtaacctgt gtgcatactc tagaagagta gggaaaataa tgcttgttac 1740
   aattcgacct aatatgtgca ttgtaaaata aatgccatat ttcaaacaaa acacgtaatt 1800
   tttttacagt atgttttatt accttttgat atctgttgtt gcaatgttag tgatgtttta 1860
   aaatgtgatc gaaaatataa tgcttctaag aaggaacagt agtggaatga atgtctaaaa 1920
   gatctttatg tgtttatggt ctgcagaagg atttttgtga tgaaagggga ttttttgaaa 1980
   aatctagaga agtagcatat ggaaaactat aatgtgtctt ttttacaatg acttcagctc 2040
   tgtttttagc tagaaactct aaaaacaaaa ataataataa agaaaaataa ataaaaagga 2100
   gaggcagaca atgtctggat tcctgttttt tggttacctg atttcatgat catgatgctt 2160
   cttgtcaaca ccctcttaag cagcaccaga aacagtgagt ttgtctgtac cattaggagt 2220
   taggtactaa ttagttggct aatgctcaag tattttatac ccacaagaga ggtatgtcac 2280
   tcatcttact tcccaggaca tccaccctga gaataatttg acaagcttaa aaatggcctt 2340
   catgtgagtg ccaaattttg ttttcttcat ttaaatattt tctttgccta aatacatgtg 2400
   agaggagtta aatataaatg tacagagagg aaagttgagg ttccacctct gaaatgagaa 2460
   ttacttgaca gttgggatac tttaatcaga aaaaaagaac ttatcttgca gcattttatc 2520
   aacaaatttc ataattgtgg acaattggag gcatttattt taaaaaacaa ttttattggc 2580
   cttttgctaa cacagtaagc atgtattctc tataaggcat tcaataaatg cacaacgccc 2640
   aaaggaaata aaatcctatc taatcctact ctccactaca cagaggtaat cactattagt 2700
   attttggcat attattctcc aggtgtttct tatgcactta taaaatgatt tgaacaaata 2760
   aaactaggaa cctgctatac atgtgtttca taacctgcct cctttgcttg gccctttatt 2820
   gagataagtt ttcctgtcaa gaaagcagaa accatctcat ttctaacagc tgtgttatat 2880
   tccatagtat gcattactca acaaactgtt gtgctattgg atacttaggt ggtttcttca 2940
   ctgacaatac tgaataaaca tctcaatagt caaa 2974
<210> 22
   <211> 346
   <212> PRT
   <213> Homo sapiens
<400> 22
<210> 23
   <211> 2450
   <212> DNA
   <213> Homo sapiens
<220>
   <221> source
   <222> 1..2450
   <223> /mol_type="unassigned DNA"
   /organism="Homo sapiens"
<400> 23
   atatttcata cctttctaga aactgggtgt gatctcactg ttggtaaagc ccagcccttc 60
   ccaacctgca agctcacctt ccaggactgg gcccagccca tgctctccat atataagctg 120
   ctgccccgag cctgattcct agtcctgctt ctcttccctc tctcctccag cctctcacac 180
   tctcctcagc tctctcatct cctggaacca tggccagcac atccaccacc atcaggagcc 240
   acagcagcag ccgccggggt ttcagtgcca actcagccag gctccctggg gtcagccgct 300
   ctggcttcag cagcgtctcc gtgtcccgct ccaggggcag tggtggcctg ggtggtgcat 360
   gtggaggagc tggctttggc agccgcagtc tgtatggcct ggggggctcc aagaggatct 420
   ccattggagg gggcagctgt gccatcagtg gcggctatgg cagcagagcc ggaggcagct 480
   atggctttgg tggcgccggg agtggatttg gtttcggtgg tggagccggc attggctttg 540
   gtctgggtgg tggagccggc cttgctggtg gctttggggg ccctggcttc cctgtgtgcc 600
   cccctggagg catccaagag gtcaccgtca accagagtct cctgactccc ctcaacctgc 660
   aaatcgatcc caccatccag cgggtgcggg ctgaggagcg tgaacagatc aagaccctca 720
   acaacaagtt tgcctccttc atcgacaagg tgcggttcct ggagcagcag aacaaggttc 780
   tggaaacaaa gtggaccctg ctgcaggagc agggcaccaa gactgtgagg cagaacctgg 840
   agccgttgtt cgagcagtac atcaacaacc tcaggaggca gctggacagc attgtcgggg 900
   aacggggccg cctggactca gagctcagag gcatgcagga cctggtggag gacttcaaga 960
   acaaatatga ggatgaaatc aacaagcgca cagcagcaga gaatgaattt gtgactctga 1020
   agaaggatgt ggatgctgcc tacatgaaca aggttgaact gcaagccaag gcagacactc 1080
   tcacagacga gatcaacttc ctgagagcct tgtatgatgc agagctgtcc cagatgcaga 1140
   cccacatctc agacacatct gtggtgctgt ccatggacaa caaccgcaac ctggacctgg 1200
   acagcatcat cgctgaggtc aaggcccaat atgaggagat tgctcagaga agccgggctg 1260
   aggctgagtc ctggtaccag accaagtacg aggagctgca ggtcacagca ggcagacatg 1320
   gggacgacct gcgcaacacc aagcaggaga ttgctgagat caaccgcatg atccagaggc 1380
   tgagatctga gatcgaccac gtcaagaagc agtgcgccaa cctgcaggcc gccattgctg 1440
   atgctgagca gcgtggggag atggccctca aggatgccaa gaacaagctg gaagggctgg 1500
   aggatgccct gcagaaggcc aagcaggacc tggcccggct gctgaaggag taccaggagc 1560
   tgatgaatgt caagctggcc ctggacgtgg agatcgccac ctaccgcaag ctgctggagg 1620
   gtgaggagtg caggctgaat ggcgaaggcg ttggacaagt caacatctct gtggtgcagt 1680
   ccaccgtctc cagtggctat ggcggtgcca gtggtgtcgg cagtggctta ggcctgggtg 1740
   gaggaagcag ctactcctat ggcagtggtc ttggcgttgg aggtggcttc agttccagca 1800
   gtggcagagc cattgggggt ggcctcagct ctgttggagg cggcagttcc accatcaagt 1860
   acaccaccac ctcctcctcc agcaggaaga gctataagca ctaaagtgcg tctgctagct 1920
   ctcggtccca cagtcctcag gcccctctct ggctgcagag ccctctcctc aggttgcctt 1980
   tcctctcctg gcctccagtc tcccctgctg tcccaggtag agctgggtat ggatgcttag 2040
   tgccctcact tcttctctct ctctctatac catctgagca cccattgctc accatcagat 2100
   caacctctga ttttacatca tgatgtaatc accactggag cttcactgtt actaaattat 2160
   taatttcttg cctccagtgt tctatctctg aggctgagca ttataagaaa atgacctctg 2220
   ctccttttca ttgcagaaaa ttgccagggg cttatttcag aacaacttcc acttactttc 2280
   cactggctct caaactctct aacttataag tgttgtgaac ccccacccag gcagtatcca 2340
   tgaaagcaca agtgactagt cctatgatgt acaaagcctg tatctctgtg atgatttctg 2400
   tgctcttcgc tgtttgcaat tgctaaataa agcagattta taatacaata 2450
<210> 24
   <211> 564
   <212> PRT
   <213> Homo sapiens
<400> 24
<210> 25
   <211> 3685
   <212> DNA
   <213> Homo sapiens
<220>
   <221> source
   <222> 1..3685
   <223> /mol_type="unassigned DNA"
   /organism="Homo sapiens"
<400> 25
   agtggactca cgcaggcgca ggagactaca cttcccagga actccgggcc gcgttgttcg 60
   ctggtacctc cttctgactt ccggtattgc tgcggtctgt agggccaatc gggagcctgg 120
   aattgctttc ccggcgctct gattggtgca ttcgactagg ctgcctgggt tcaaaatttc 180
   aacgatactg aatgagtccc gcggcgggtt ggctcgcgct tcgttgtcag atctgaggcg 240
   aggctaggtg agccgtggga agaaaagagg gagcagctag ggcgcgggtc tccctcctcc 300
   cggagtttgg aacggctgaa gttcaccttc cagcccctag cgccgttcgc gccgctaggc 360
   ctggcttctg aggcggttgc ggtgctcggt cgccgcctag gcggggcagg gtgcgagcag 420
   gggcttcggg ccacgcttct cttggcgaca ggattttgct gtgaagtccg tccgggaaac 480
   ggaggaaaaa aagagttgcg ggaggctgtc ggctaataac ggttcttgat acatatttgc 540
   cagacttcaa gatttcagaa aaggggtgaa agagaagatt gcaactttga gtcagacctg 600
   taggcctgat agactgatta aaccacagaa ggtgacctgc tgagaaaagt ggtacaaata 660
   ctgggaaaaa cctgctcttc tgcgttaagt gggagacaat gtcacaagtt aaaagctctt 720
   attcctatga tgccccctcg gatttcatca atttttcatc cttggatgat gaaggagata 780
   ctcaaaacat agattcatgg tttgaggaga aggccaattt ggagaataag ttactgggga 840
   agaatggaac tggagggctt tttcagggca aaactccttt gagaaaggct aatcttcagc 900
   aagctattgt cacacctttg aaaccagttg acaacactta ctacaaagag gcagaaaaag 960
   aaaatcttgt ggaacaatcc attccgtcaa atgcttgttc ttccctggaa gttgaggcag 1020
   ccatatcaag aaaaactcca gcccagcctc agagaagatc tcttaggctt tctgctcaga 1080
   aggatttgga acagaaagaa aagcatcatg taaaaatgaa agccaagaga tgtgccactc 1140
   ctgtaatcat cgatgaaatt ctaccctcta agaaaatgaa agtttctaac aacaaaaaga 1200
   agccagagga agaaggcagt gctcatcaag atactgctga aaagaatgca tcttccccag 1260
   agaaagccaa gggtagacat actgtgcctt gtatgccacc tgcaaagcag aagtttctaa 1320
   aaagtactga ggagcaagag ctggagaaga gtatgaaaat gcagcaagag gtggtggaga 1380
   tgcggaaaaa gaatgaagaa ttcaagaaac ttgctctggc tggaataggg caacctgtga 1440
   agaaatcagt gagccaggtc accaaatcag ttgacttcca cttccgcaca gatgagcgaa 1500
   tcaaacaaca tcctaagaac caggaggaat ataaggaagt gaactttaca tctgaactac 1560
   gaaagcatcc ttcatctcct gcccgagtga ctaagggatg taccattgtt aagcctttca 1620
   acctgtccca aggaaagaaa agaacatttg atgaaacagt ttctacatat gtgccccttg 1680
   cacagcaagt tgaagacttc cataaacgaa cccctaacag atatcatttg aggagcaaga 1740
   aggatgatat taacctgtta ccctccaaat cttctgtgac caagatttgc agagacccac 1800
   agactcctgt actgcaaacc aaacaccgtg cacgggctgt gacctgcaaa agtacagcag 1860
   agctggaggc tgaggagctc gagaaattgc aacaatacaa attcaaagca cgtgaacttg 1920
   atcccagaat acttgaaggt gggcccatct tgcccaagaa accacctgtg aaaccaccca 1980
   ccgagcctat tggctttgat ttggaaattg agaaaagaat ccaggagcga gaatcaaaga 2040
   agaaaacaga ggatgaacac tttgaatttc attccagacc ttgccctact aagattttgg 2100
   aagatgttgt gggtgttcct gaaaagaagg tacttccaat caccgtcccc aagtcaccag 2160
   cctttgcatt gaagaacaga attcgaatgc ccaccaaaga agatgaggaa gaggacgaac 2220
   cggtagtgat aaaagctcaa cctgtgccac attatggggt gccttttaag ccccaaatcc 2280
   cagaggcaag aactgtggaa atatgccctt tctcgtttga ttctcgagac aaagaacgtc 2340
   agttacagaa ggagaagaaa ataaaagaac tgcagaaagg ggaggtgccc aagttcaagg 2400
   cacttccctt gcctcatttt gacaccatta acctgccaga gaagaaggta aagaatgtga 2460
   cccagattga acctttctgc ttggagactg acagaagagg tgctctgaag gcacagactt 2520
   ggaagcacca gctggaagaa gaactgagac agcagaaaga agcagcttgt ttcaaggctc 2580
   gtccaaacac cgtcatctct caggagccct ttgttcccaa gaaagagaag aaatcagttg 2640
   ctgagggcct ttctggttct ctagttcagg aaccttttca gctggctact gagaagagag 2700
   ccaaagagcg gcaggagctg gagaagagaa tggctgaggt agaagcccag aaagcccagc 2760
   agttggagga ggccagacta caggaggaag agcagaaaaa agaggagctg gccaggctac 2820
   ggagagaact ggtgcataag gcaaatccaa tacgcaagta ccagggtctg gagataaagt 2880
   caagtgacca gcctctgact gtgcctgtat ctcccaaatt ctccactcga ttccactgct 2940
   aaactcagct gtgagctgcg gataccgccc ggcaatggga cctgctctta acctcaaacc 3000
   taggaccgtc ttgctttgtc attgggcatg gagagaaccc atttctccag acttttacct 3060
   acccgtgcct gagaaagcat acttgacaac tgtggactcc agttttgttg agaattgttt 3120
   tcttacatta ctaaggctaa taatgagatg taactcatga atgtctcgat tagactccat 3180
   gtagttactt cctttaaacc atcagccggc cttttatatg ggtcttcact ctgactagaa 3240
   tttagtctct gtgtcagcac agtgtaatct ctattgctat tgccccttac gactctcacc 3300
   ctctccccac tttttttaaa aattttaacc agaaaataaa gatagttaaa tcctaagata 3360
   gagattaagt catggtttaa atgaggaaca atcagtaaat cagattctgt cctcttctct 3420
   gcataccgtg aatttatagt taaggatccc tttgctgtga gggtagaaaa cctcaccaac 3480
   tgcaccagtg aggaagaaga ctgcgtggat tcatggggag cctcacagca gccacgcagc 3540
   aggctctggg tggggctgcc gttaaggcac gttctttcct tactggtgct gataacaaca 3600
   gggaaccgtg cagtgtgcat tttaagacct ggcctggaat aaatacgttt tgtctttccc 3660
   tcaaaaaaaa aaaaaaaaaa aaaaa 3685
<210> 26
   <211> 747
   <212> PRT
   <213> Homo sapiens
<400> 26
   Met Ser Gln Val Lys Ser Ser Tyr Ser Tyr Asp Ala Pro Ser Asp Phe
   1               5                   10                  15
   Ile Asn Phe Ser Ser Leu Asp Asp Glu Gly Asp Thr Gln Asn Ile Asp
               20                  25                  30
   Ser Trp Phe Glu Glu Lys Ala Asn Leu Glu Asn Lys Leu Leu Gly Lys
           35                  40                  45
   Asn Gly Thr Gly Gly Leu Phe Gln Gly Lys Thr Pro Leu Arg Lys Ala
       50                  55                  60
   Asn Leu Gln Gln Ala Ile Val Thr Pro Leu Lys Pro Val Asp Asn Thr
   65                  70                  75                  80
   Tyr Tyr Lys Glu Ala Glu Lys Glu Asn Leu Val Glu Gln Ser Ile Pro
                   85                  90                  95
   Ser Asn Ala Cys Ser Ser Leu Glu Val Glu Ala Ala Ile Ser Arg Lys
               100                 105                 110
   Thr Pro Ala Gln Pro Gln Arg Arg Ser Leu Arg Leu Ser Ala Gln Lys
           115                 120                 125
   Asp Leu Glu Gln Lys Glu Lys His His Val Lys Met Lys Ala Lys Arg
       130                 135                 140
   Cys Ala Thr Pro Val Ile Ile Asp Glu Ile Leu Pro Ser Lys Lys Met
   145                 150                 155                 160
   Lys Val Ser Asn Asn Lys Lys Lys Pro Glu Glu Glu Gly Ser Ala His
                   165                 170                 175
   Gln Asp Thr Ala Glu Lys Asn Ala Ser Ser Pro Glu Lys Ala Lys Gly
               180                 185                 190
   Arg His Thr Val Pro Cys Met Pro Pro Ala Lys Gln Lys Phe Leu Lys
           195                 200                 205
   Ser Thr Glu Glu Gln Glu Leu Glu Lys Ser Met Lys Met Gln Gln Glu
       210                 215                 220
   Val Val Glu Met Arg Lys Lys Asn Glu Glu Phe Lys Lys Leu Ala Leu
   225                 230                 235                 240
   Ala Gly Ile Gly Gln Pro Val Lys Lys Ser Val Ser Gln Val Thr Lys
                   245                 250                 255
   Ser Val Asp Phe His Phe Arg Thr Asp Glu Arg Ile Lys Gln His Pro
               260                 265                 270
   Lys Asn Gln Glu Glu Tyr Lys Glu Val Asn Phe Thr Ser Glu Leu Arg
           275                 280                 285
   Lys His Pro Ser Ser Pro Ala Arg Val Thr Lys Gly Cys Thr Ile Val
       290                 295                 300
   Lys Pro Phe Asn Leu Ser Gln Gly Lys Lys Arg Thr Phe Asp Glu Thr
   305                 310                 315                 320
   Val Ser Thr Tyr Val Pro Leu Ala Gln Gln Val Glu Asp Phe His Lys
                   325                 330                 335
   Arg Thr Pro Asn Arg Tyr His Leu Arg Ser Lys Lys Asp Asp Ile Asn
               340                 345                 350
   Leu Leu Pro Ser Lys Ser Ser Val Thr Lys Ile Cys Arg Asp Pro Gln
           355                 360                 365
   Thr Pro Val Leu Gln Thr Lys His Arg Ala Arg Ala Val Thr Cys Lys
       370                 375                 380
   Ser Thr Ala Glu Leu Glu Ala Glu Glu Leu Glu Lys Leu Gln Gln Tyr
   385                 390                 395                 400
   Lys Phe Lys Ala Arg Glu Leu Asp Pro Arg Ile Leu Glu Gly Gly Pro
                   405                 410                 415
   Ile Leu Pro Lys Lys Pro Pro Val Lys Pro Pro Thr Glu Pro Ile Gly
               420                 425                 430
   Phe Asp Leu Glu Ile Glu Lys Arg Ile Gln Glu Arg Glu Ser Lys Lys
           435                 440                 445
   Lys Thr Glu Asp Glu His Phe Glu Phe His Ser Arg Pro Cys Pro Thr
       450                 455                 460
   Lys Ile Leu Glu Asp Val Val Gly Val Pro Glu Lys Lys Val Leu Pro
   465                 470                 475                 480
   Ile Thr Val Pro Lys Ser Pro Ala Phe Ala Leu Lys Asn Arg Ile Arg
                   485                 490                 495
   Met Pro Thr Lys Glu Asp Glu Glu Glu Asp Glu Pro Val Val Ile Lys
               500                 505                 510
   Ala Gln Pro Val Pro His Tyr Gly Val Pro Phe Lys Pro Gln Ile Pro
           515                 520                 525
   Glu Ala Arg Thr Val Glu Ile Cys Pro Phe Ser Phe Asp Ser Arg Asp
       530                 535                 540
   Lys Glu Arg Gln Leu Gln Lys Glu Lys Lys Ile Lys Glu Leu Gln Lys
   545                 550                 555                 560
   Gly Glu Val Pro Lys Phe Lys Ala Leu Pro Leu Pro His Phe Asp Thr
                   565                 570                 575
   Ile Asn Leu Pro Glu Lys Lys Val Lys Asn Val Thr Gln Ile Glu Pro
               580                 585                 590
   Phe Cys Leu Glu Thr Asp Arg Arg Gly Ala Leu Lys Ala Gln Thr Trp
           595                 600                 605
   Lys His Gln Leu Glu Glu Glu Leu Arg Gln Gln Lys Glu Ala Ala Cys
       610                 615                 620
   Phe Lys Ala Arg Pro Asn Thr Val Ile Ser Gln Glu Pro Phe Val Pro
   625                 630                 635                 640
   Lys Lys Glu Lys Lys Ser Val Ala Glu Gly Leu Ser Gly Ser Leu Val
                   645                 650                 655
   Gln Glu Pro Phe Gln Leu Ala Thr Glu Lys Arg Ala Lys Glu Arg Gln
               660                 665                 670
   Glu Leu Glu Lys Arg Met Ala Glu Val Glu Ala Gln Lys Ala Gln Gln
           675                 680                 685
   Leu Glu Glu Ala Arg Leu Gln Glu Glu Glu Gln Lys Lys Glu Glu Leu
       690                 695                 700
   Ala Arg Leu Arg Arg Glu Leu Val His Lys Ala Asn Pro Ile Arg Lys
   705                 710                 715                 720
   Tyr Gln Gly Leu Glu Ile Lys Ser Ser Asp Gln Pro Leu Thr Val Pro
                   725                 730                 735
   Val Ser Pro Lys Phe Ser Thr Arg Phe His Cys
               740                 745
<210> 27
   <211> 1566
   <212> DNA
   <213> Homo sapiens
<220>
   <221> source
   <222> 1..1566
   <223> /mol_type="unassigned DNA"
   /organism="Homo sapiens"
<400> 27
   gggccgccca atggggcgca agcgacgcgg tatttgaatc ctggaacaag gctacagcgt 60
   cgaagatccc cagcgctgcg ggctcggaga gcagtcctaa cggcgcctcg tacgctagtg 120
   tcctcccttt tcagtccgcg tccctccctg ggccgggctg gcactcttgc cttccccgtc 180
   cctcatggcg ctgctccgac gcccgacggt gtccagtgat ttggagaata ttgacacagg 240
   agttaattct aaagttaaga gtcatgtgac tattaggcga actgttttag aagaaattgg 300
   aaatagagtt acaaccagag cagcacaagt agctaagaaa gctcagaaca ccaaagttcc 360
   agttcaaccc accaaaacaa caaatgtcaa caaacaactg aaacctactg cttctgtcaa 420
   accagtacag atggaaaagt tggctccaaa gggtccttct cccacacctg aggatgtctc 480
   catgaaggaa gagaatctct gccaagcttt ttctgatgcc ttgctctgca aaatcgagga 540
   cattgataac gaagattggg agaaccctca gctctgcagt gactacgtta aggatatcta 600
   tcagtatctc aggcagctgg aggttttgca gtccataaac ccacatttct tagatggaag 660
   agatataaat ggacgcatgc gtgccatcct agtggattgg ctggtacaag tccactccaa 720
   gtttaggctt ctgcaggaga ctctgtacat gtgcgttggc attatggatc gatttttaca 780
   ggttcagcca gtttcccgga agaagcttca attagttggg attactgctc tgctcttggc 840
   ttccaagtat gaggagatgt tttctccaaa tattgaagac tttgtttaca tcacagacaa 900
   tgcttatacc agttcccaaa tccgagaaat ggaaactcta attttgaaag aattgaaatt 960
   tgagttgggt cgacccttgc cactacactt cttaaggcga gcatcaaaag ccggggaggt 1020
   tgatgttgaa cagcacactt tagccaagta tttgatggag ctgactctca tcgactatga 1080
   tatggtgcat tatcatcctt ctaaggtagc agcagctgct tcctgcttgt ctcagaaggt 1140
   tctaggacaa ggaaaatgga acttaaagca gcagtattac acaggataca cagagaatga 1200
   agtattggaa gtcatgcagc acatggccaa gaatgtggtg aaagtaaatg aaaacttaac 1260
   taaattcatc gccatcaaga ataagtatgc aagcagcaaa ctcctgaaga tcagcatgat 1320
   ccctcagctg aactcaaaag ccgtcaaaga ccttgcctcc ccactgatag gaaggtccta 1380
   ggctgccgtg gcccctgggg atgtgtgctt cattgtgccc tttttcttat tggtttagaa 1440
   ctcttgattt tgtacatagt cctctggtct atctcatgaa acctcttctc agaccagttt 1500
   tctaaacata tattgaggaa aaataaagcg attggttttt cttaaggtaa aaaaaaaaaa 1560
   aaaaaa 1566
<210> 28
   <211> 398
   <212> PRT
   <213> Homo sapiens
<400> 28
   Met Ala Leu Leu Arg Arg Pro Thr Val Ser Ser Asp Leu Glu Asn Ile
   1               5                   10                  15
   Asp Thr Gly Val Asn Ser Lys Val Lys Ser His Val Thr Ile Arg Arg
               20                  25                  30
   Thr Val Leu Glu Glu Ile Gly Asn Arg Val Thr Thr Arg Ala Ala Gln
           35                  40                  45
   Val Ala Lys Lys Ala Gln Asn Thr Lys Val Pro Val Gln Pro Thr Lys
       50                  55                  60
   Thr Thr Asn Val Asn Lys Gln Leu Lys Pro Thr Ala Ser Val Lys Pro
   65                  70                  75                  80
   Val Gln Met Glu Lys Leu Ala Pro Lys Gly Pro Ser Pro Thr Pro Glu
                   85                  90                  95
   Asp Val Ser Met Lys Glu Glu Asn Leu Cys Gln Ala Phe Ser Asp Ala
               100                 105                 110
   Leu Leu Cys Lys Ile Glu Asp Ile Asp Asn Glu Asp Trp Glu Asn Pro
           115                 120                 125
   Gln Leu Cys Ser Asp Tyr Val Lys Asp Ile Tyr Gln Tyr Leu Arg Gln
       130                 135                 140
   Leu Glu Val Leu Gln Ser Ile Asn Pro His Phe Leu Asp Gly Arg Asp
   145                 150                 155                 160
   Ile Asn Gly Arg Met Arg Ala Ile Leu Val Asp Trp Leu Val Gln Val
                   165                 170                 175
   His Ser Lys Phe Arg Leu Leu Gln Glu Thr Leu Tyr Met Cys Val Gly
               180                 185                 190
   Ile Met Asp Arg Phe Leu Gln Val Gln Pro Val Ser Arg Lys Lys Leu
           195                 200                 205
   Gln Leu Val Gly Ile Thr Ala Leu Leu Leu Ala Ser Lys Tyr Glu Glu
       210                 215                 220
   Met Phe Ser Pro Asn Ile Glu Asp Phe Val Tyr Ile Thr Asp Asn Ala
   225                 230                 235                 240
   Tyr Thr Ser Ser Gln Ile Arg Glu Met Glu Thr Leu Ile Leu Lys Glu
                   245                 250                 255
   Leu Lys Phe Glu Leu Gly Arg Pro Leu Pro Leu His Phe Leu Arg Arg
               260                 265                 270
   Ala Ser Lys Ala Gly Glu Val Asp Val Glu Gln His Thr Leu Ala Lys
           275                 280                 285
   Tyr Leu Met Glu Leu Thr Leu Ile Asp Tyr Asp Met Val His Tyr His
       290                 295                 300
   Pro Ser Lys Val Ala Ala Ala Ala Ser Cys Leu Ser Gln Lys Val Leu
   305                 310                 315                 320
   Gly Gln Gly Lys Trp Asn Leu Lys Gln Gln Tyr Tyr Thr Gly Tyr Thr
                   325                 330                 335
   Glu Asn Glu Val Leu Glu Val Met Gln His Met Ala Lys Asn Val Val
               340                 345                 350
   Lys Val Asn Glu Asn Leu Thr Lys Phe Ile Ala Ile Lys Asn Lys Tyr
           355                 360                 365
   Ala Ser Ser Lys Leu Leu Lys Ile Ser Met Ile Pro Gln Leu Asn Ser
       370                 375                 380
   Lys Ala Val Lys Asp Leu Ala Ser Pro Leu Ile Gly Arg Ser
   385                 390                 395
<210> 29
   <211> 4786
   <212> DNA
   <213> Homo sapiens
<220>
   <221> source
   <222> 1..4786
   <223> /mol_type="unassigned DNA"
   /organism="Homo sapiens"
<400> 29
   ctcggcgctg aaattcaaat ttgaacggct gcagaggccg agtccgtcac tggaagccga      60
   gaggagagga cagctggttg tgggagagtt cccccgcctc agactcctgg ttttttccag    120
   gagacacact gagctgagac tcacttttct cttcctgaat ttgaaccacc gtttccatcg     180
   tctcgtagtc cgacgcctgg ggcgatggat ccgtttacgg agaaactgct ggagcgaacc     240
   cgtgccaggc gagagaatct tcagagaaaa atggctgaga ggcccacagc agctccaagg     300
   tctatgactc atgctaagcg agctagacag ccactttcag aagcaagtaa ccagcagccc     360
   ctctctggtg gtgaagagaa atcttgtaca aaaccatcgc catcaaaaaa acgctgttct     420
   gacaacactg aagtagaagt ttctaacttg gaaaataaac aaccagttga gtcgacatct     480
   gcaaaatctt gttctccaag tcctgtgtct cctcaggtgc agccacaagc agcagatacc      540
   atcagtgatt ctgttgctgt cccggcatca ctgctgggca tgaggagagg gctgaactca      600
   agattggaag caactgcagc ctcctcagtt aaaacacgta tgcaaaaact tgcagagcaa      660
   cggcgccgtt gggataatga tgatatgaca gatgacattc ctgaaagctc actcttctca      720
   ccaatgccat cagaggaaaa ggctgcttcc cctcccagac ctctgctttc aaatgcctcg      780
   gcaactccag ttggcagaag gggccgtctg gccaatcttg ctgcaactat ttgctcctgg      840
   gaagatgatg taaatcactc atttgcaaaa caaaacagtg tacaagaaca gcctggtacc      900
   gcttgtttat ccaaattttc ctctgcaagt ggagcatctg ctaggatcaa tagcagcagt      960
   gttaagcagg aagctacatt ctgttcccaa agggatggcg atgcctcttt gaataaagcc     1020
   ctatcctcaa gtgctgatga tgcgtctttg gttaatgcct caatttccag ctctgtgaaa     1080
   gctacttctc cagtgaaatc tactacatct atcactgatg ctaaaagttg tgagggacaa     1140
   aatcctgagc tacttccaaa aactcctatt agtcctctga aaacgggggt atcgaaacca     1200
   attgtgaagt caactttatc ccagacagtt ccatccaagg gagaattaag tagagaaatt     1260
   tgtctgcaat ctcaatctaa agacaaatct acgacaccag gaggaacagg aattaagcct     1320
   ttcctggaac gctttggaga gcgttgtcaa gaacatagca aagaaagtcc agctcgtagc     1380
   acaccccaca gaacccccat tattactcca aatacaaagg ccatccaaga aagattattc     1440
   aagcaagaca catcttcatc tactacccat ttagcacaac agctcaagca ggaacgtcaa     1500
   aaagaactag catgtcttcg tggccgattt gacaagggca atatatggag tgcagaaaaa     1560
   ggcggaaact caaaaagcaa acaactagaa accaaacagg aaactcactg tcagagcact     1620
   cccctcaaaa aacaccaagg tgtttcaaaa actcagtcac ttccagtaac agaaaaggtg     1680
   accgaaaacc agataccagc caaaaattct agtacagaac ctaaaggttt cactgaatgc     1740
   gaaatgacga aatctagccc tttgaaaata acattgtttt tagaagagga caaatcctta     1800
   aaagtaacat cagacccaaa ggttgagcag aaaattgaag tgatacgtga aattgagatg     1860
   agtgtggatg atgatgatat caatagttcg aaagtaatta atgacctctt cagtgatgtc     1920
   ctagaggaag gtgaactaga tatggagaag agccaagagg agatggatca agcattagca     1980
   gaaagcagcg aagaacagga agatgcactg aatatctcct caatgtcttt acttgcacca     2040
   ttggcacaaa cagttggtgt ggtaagtcca gagagtttag tgtccacacc tagactggaa     2100
   ttgaaagaca ccagcagaag tgatgaaagt ccaaaaccag gaaaattcca aagaactcgt     2160
   gtccctcgag ctgaatctgg tgatagcctt ggttctgaag atcgtgatct tctttacagc     2220
   attgatgcat atagatctca aagattcaaa gaaacagaac gtccatcaat aaagcaggtg     2280
   attgttcgga aggaagatgt tacttcaaaa ctggatgaaa aaaataatgc ctttccttgt     2340
   caagttaata tcaaacagaa aatgcaggaa ctcaataacg aaataaatat gcaacagaca 2400
   gtgatctatc aagctagcca ggctcttaac tgctgtgttg atgaagaaca tggaaaaggg 2460
   tccctagaag aagctgaagc agaaagactt cttctaattg caactgggaa gagaacactt 2520
   ttgattgatg aattgaataa attgaagaac gaaggacctc agaggaagaa taaggctagt 2580
   ccccaaagtg aatttatgcc atccaaagga tcagttactt tgtcagaaat ccgcttgcct 2640
   ctaaaagcag attttgtctg cagtacggtt cagaaaccag atgcagcaaa ttactattac 2700
   ttaattatac taaaagcagg agctgaaaat atggtagcca caccattagc aagtacttca 2760
   aactctctta acggtgatgc tctgacattc actactacat ttactctgca agatgtatcc 2820
   aatgactttg aaataaatat tgaagtttac agcttggtgc aaaagaaaga tccctcaggc 2880
   cttgataaga agaaaaaaac atccaagtcc aaggctatta ctccaaagcg actcctcaca 2940
   tctataacca caaaaagcaa cattcattct tcagtcatgg ccagtccagg aggtcttagt 3000
   gctgtgcgaa ccagcaactt cgcccttgtt ggatcttaca cattatcatt gtcttcagta 3060
   ggaaatacta agtttgttct ggacaaggtc ccctttttat cttctttgga aggtcatatt 3120
   tatttaaaaa taaaatgtca agtgaattcc agtgttgaag aaagaggttt tctaaccata 3180
   tttgaagatg ttagtggttt tggtgcctgg catcgaagat ggtgtgttct ttctggaaac 3240
   tgtatatctt attggactta tccagatgat gagaaacgca agaatcccat aggaaggata 3300
   aatctggcta attgtaccag tcgtcagata gaaccagcca acagagaatt ttgtgcaaga 3360
   cgcaacactt ttgaattaat tactgtccga ccacaaagag aagatgaccg agagactctt 3420
   gtcagccaat gcagggacac actctgtgtt accaagaact ggctgtctgc agatactaaa 3480
   gaagagcggg atctctggat gcaaaaactc aatcaagttc ttgttgatat tcgcctctgg 3540
   caacctgatg cttgctacaa acctattgga aagccttaaa ccgggaaatt tccatgctat 3600
   ctagaggttt ttgatgtcat cttaagaaac acacttaaga gcatcagatt tactgattgc 3660
   attttatgct ttaagtacga aagggtttgt gccaatattc actacgtatt atgcagtatt 3720
   tatatctttt gtatgtaaaa ctttaactga tttctgtcat tcatcaatga gtagaagtaa 3780
   atacattata gttgattttg ctaaatctta atttaaaagc ctcattttcc tagaaatcta 3840
   attattcagt tattcatgac aatatttttt taaaagtaag aaattctgag ttgtcttctt 3900
   ggagctgtag gtcttgaagc agcaacgtct ttcaggggtt ggagacagaa acccattctc 3960
   caatctcagt agttttttcg aaaggctgtg atcatttatt gatcgtgata tgacttgtta 4020
   ctagggtact gaaaaaaatg tctaaggcct ttacagaaac atttttagta atgaggatga 4080
   gaactttttc aaatagcaaa tatatattgg cttaaagcat gaggctgtct tcagaaaagt 4140
   gatgtggaca taggaggcaa tgtgtgagac ttgggggttc aatattttat atagaagagt 4200
   taataagcac atggtttaca tttactcagc tactatatat gcagtgtggt gcacattttc 4260
   acagaattct ggcttcatta agatcattat ttttgctgcg tagcttacag acttagcata     4320
   ttagtttttt ctactcctac aagtgtaaat tgaaaaatct ttatattaaa aaagtaaact     4380
   gttatgaagc tgctatgtac taataatact ttgcttgcca aagtgtttgg gttttgttgt     4440
   tgtttgtttg tttgtttgtt tttggttcat gaacaacagt gtctagaaac ccattttgaa     4500
   agtggaaaat tattaagtca cctatcacct ttaaacgcct ttttttaaaa ttataaaata     4560
   ttgtaaagca gggtctcaac ttttaaatac actttgaact tcttctctga attattaaag     4620
   ttctttatga cctcatttat aaacactaaa ttctgtcacc tcctgtcatt ttatttttta     4680
   ttcattcaaa tgtatttttt cttgtgcata ttataaaaat atattttatg agctcttact     4740
   caaataaata cctgtaaatg tctaaaggaa aaaaaaaaaa aaaaaa                    4786
<210> 30
   <211> 1124
   <212> PRT
   <213> Homo sapiens
<400> 30
<210> 31
   <211> 3665
   <212> DNA
   <213> Homo sapiens
<220>
   <221> source
   <222> 1..3665
   <223> /mol_type="unassigned DNA"
   /organism="Homo sapiens"
<400> 31
   tttcaaacag cggaacaaac tgaaagctcc ggtgccagac cccacccccg gccccggccc      60
   gggaccccct cccctcccgg gatcccccgg ggttcccacc ccgcccgcac cgccggggac    120
   ccggccggtc cggcgcgagc ccccgtccgg ggccctggct cggcccccag gttggaggag     180
   cccggagccc gccttcggag ctacggccta acggcggcgg cgactgcagt ctggagggtc     240
   cacacttgtg attctcaatg gagagtgaaa acgcagattc ataatgaaaa ctagcccccg 300
   tcggccactg attctcaaaa gacggaggct gccccttcct gttcaaaatg ccccaagtga 360
   aacatcagag gaggaaccta agagatcccc tgcccaacag gagtctaatc aagcagaggc 420
   ctccaaggaa gtggcagagt ccaactcttg caagtttcca gctgggatca agattattaa 480
   ccaccccacc atgcccaaca cgcaagtagt ggccatcccc aacaatgcta atattcacag 540
   catcatcaca gcactgactg ccaagggaaa agagagtggc agtagtgggc ccaacaaatt 600
   catcctcatc agctgtgggg gagccccaac tcagcctcca ggactccggc ctcaaaccca 660
   aaccagctat gatgccaaaa ggacagaagt gaccctggag accttgggac caaaacctgc 720
   agctagggat gtgaatcttc ctagaccacc tggagccctt tgcgagcaga aacgggagac 780
   ctgtgcagat ggtgaggcag caggctgcac tatcaacaat agcctatcca acatccagtg 840
   gcttcgaaag atgagttctg atggactggg ctcccgcagc atcaagcaag agatggagga 900
   aaaggagaat tgtcacctgg agcagcgaca ggttaaggtt gaggagcctt cgagaccatc 960
   agcgtcctgg cagaactctg tgtctgagcg gccaccctac tcttacatgg ccatgataca 1020
   attcgccatc aacagcactg agaggaagcg catgactttg aaagacatct atacgtggat 1080
   tgaggaccac tttccctact ttaagcacat tgccaagcca ggctggaaga actccatccg 1140
   ccacaacctt tccctgcacg acatgtttgt ccgggagacg tctgccaatg gcaaggtctc 1200
   cttctggacc attcacccca gtgccaaccg ctacttgaca ttggaccagg tgtttaagcc 1260
   actggaccca gggtctccac aattgcccga gcacttggaa tcacagcaga aacgaccgaa 1320
   tccagagctc cgccggaaca tgaccatcaa aaccgaactc cccctgggcg cacggcggaa 1380
   gatgaagcca ctgctaccac gggtcagctc atacctggta cctatccagt tcccggtgaa 1440
   ccagtcactg gtgttgcagc cctcggtgaa ggtgccattg cccctggcgg cttccctcat 1500
   gagctcagag cttgcccgcc atagcaagcg agtccgcatt gcccccaagg tttttgggga 1560
   acaggtggtg tttggttaca tgagtaagtt ctttagtggc gatctgcgag attttggtac 1620
   acccatcacc agcttgttta attttatctt tctttgttta tcagtgctgc tagctgagga 1680
   ggggatagct cctctttctt ctgcaggacc agggaaagag gagaaactcc tgtttggaga 1740
   agggttttct cctttgcttc cagttcagac tatcaaggag gaagaaatcc agcctgggga 1800
   ggaaatgcca cacttagcga gacccatcaa agtggagagc cctcccttgg aagagtggcc 1860
   ctccccggcc ccatctttca aagaggaatc atctcactcc tgggaggatt cgtcccaatc 1920
   tcccacccca agacccaaga agtcctacag tgggcttagg tccccaaccc ggtgtgtctc 1980
   ggaaatgctt gtgattcaac acagggagag gagggagagg agccggtctc ggaggaaaca 2040
   gcatctactg cctccctgtg tggatgagcc ggagctgctc ttctcagagg ggcccagtac 2100
   ttcccgctgg gccgcagagc tcccgttccc agcagactcc tctgaccctg cctcccagct 2160
   cagctactcc caggaagtgg gaggaccttt taagacaccc attaaggaaa cgctgcccat 2220
   ctcctccacc ccgagcaaat ctgtcctccc cagaacccct gaatcctgga ggctcacgcc 2280
   cccagccaaa gtagggggac tggatttcag cccagtacaa acctcccagg gtgcctctga 2340
   ccccttgcct gaccccctgg ggctgatgga tctcagcacc actcccttgc aaagtgctcc 2400
   cccccttgaa tcaccgcaaa ggctcctcag ttcagaaccc ttagacctca tctccgtccc 2460
   ctttggcaac tcttctccct cagatataga cgtccccaag ccaggctccc cggagccaca 2520
   ggtttctggc cttgcagcca atcgttctct gacagaaggc ctggtcctgg acacaatgaa 2580
   tgacagcctc agcaagatcc tgctggacat cagctttcct ggcctggacg aggacccact 2640
   gggccctgac aacatcaact ggtcccagtt tattcctgag ctacagtaga gccctgccct 2700
   tgcccctgtg ctcaagctgt ccaccatccc gggcactcca aggctcagtg caccccaagc 2760
   ctctgagtga ggacagcagg cagggactgt tctgctcctc atagctccct gctgcctgat 2820
   tatgcaaaag tagcagtcac accctagcca ctgctgggac cttgtgttcc ccaagagtat 2880
   ctgattcctc tgctgtccct gccaggagct gaagggtggg aacaacaaag gcaatggtga 2940
   aaagagatta ggaacccccc agcctgtttc cattctctgc ccagcagtct cttaccttcc 3000
   ctgatctttg cagggtggtc cgtgtaaata gtataaattc tccaaattat cctctaatta 3060
   taaatgtaag cttatttcct tagatcatta tccagagact gccagaaggt gggtaggatg 3120
   acctggggtt tcaattgact tctgttcctt gcttttagtt ttgatagaag ggaagacctg 3180
   cagtgcacgg tttcttccag gctgaggtac ctggatcttg ggttcttcac tgcagggacc 3240
   cagacaagtg gatctgcttg ccagagtcct ttttgcccct ccctgccacc tccccgtgtt 3300
   tccaagtcag ctttcctgca agaagaaatc ctggttaaaa aagtcttttg tattgggtca 3360
   ggagttgaat ttggggtggg aggatggatg caactgaagc agagtgtggg tgcccagatg 3420
   tgcgctatta gatgtttctc tgataatgtc cccaatcata ccagggagac tggcattgac 3480
   gagaactcag gtggaggctt gagaaggccg aaagggcccc tgacctgcct ggcttcctta 3540
   gcttgcccct cagctttgca aagagccacc ctaggcccca gctgaccgca tgggtgtgag 3600
   ccagcttgag aacactaact actcaataaa agcgaaggtg gacatgaaaa aaaaaaaaaa 3660
   aaaaa 3665
<210> 32
   <211> 801
   <212> PRT
   <213> Homo sapiens
<400> 32
   Met Lys Thr Ser Pro Arg Arg Pro Leu Ile Leu Lys Arg Arg Arg Leu
   1               5                   10                  15
   Pro Leu Pro Val Gln Asn Ala Pro Ser Glu Thr Ser Glu Glu Glu Pro
               20                  25                  30
   Lys Arg Ser Pro Ala Gln Gln Glu Ser Asn Gln Ala Glu Ala Ser Lys
           35                  40                  45
   Glu Val Ala Glu Ser Asn Ser Cys Lys Phe Pro Ala Gly Ile Lys Ile
       50                  55                  60
   Ile Asn His Pro Thr Met Pro Asn Thr Gln Val Val Ala Ile Pro Asn
   65                  70                  75                  80
   Asn Ala Asn Ile His Ser Ile Ile Thr Ala Leu Thr Ala Lys Gly Lys
                   85                  90                  95
   Glu Ser Gly Ser Ser Gly Pro Asn Lys Phe Ile Leu Ile Ser Cys Gly
               100                 105                 110
   Gly Ala Pro Thr Gln Pro Pro Gly Leu Arg Pro Gln Thr Gln Thr Ser
           115                 120                 125
   Tyr Asp Ala Lys Arg Thr Glu Val Thr Leu Glu Thr Leu Gly Pro Lys
       130                 135                 140
   Pro Ala Ala Arg Asp Val Asn Leu Pro Arg Pro Pro Gly Ala Leu Cys
   145                 150                 155                 160
   Glu Gln Lys Arg Glu Thr Cys Ala Asp Gly Glu Ala Ala Gly Cys Thr
                   165                 170                 175
   Ile Asn Asn Ser Leu Ser Asn Ile Gln Trp Leu Arg Lys Met Ser Ser
               180                 185                 190
   Asp Gly Leu Gly Ser Arg Ser Ile Lys Gln Glu Met Glu Glu Lys Glu
           195                 200                 205
   Asn Cys His Leu Glu Gln Arg Gln Val Lys Val Glu Glu Pro Ser Arg
       210                 215                 220
   Pro Ser Ala Ser Trp Gln Asn Ser Val Ser Glu Arg Pro Pro Tyr Ser
   225                 230                 235                 240
   Tyr Met Ala Met Ile Gln Phe Ala Ile Asn Ser Thr Glu Arg Lys Arg
                   245                 250                 255
   Met Thr Leu Lys Asp Ile Tyr Thr Trp Ile Glu Asp His Phe Pro Tyr
               260                 265                 270
   Phe Lys His Ile Ala Lys Pro Gly Trp Lys Asn Ser Ile Arg His Asn
           275                 280                 285
   Leu Ser Leu His Asp Met Phe Val Arg Glu Thr Ser Ala Asn Gly Lys
       290                 295                 300
   Val Ser Phe Trp Thr Ile His Pro Ser Ala Asn Arg Tyr Leu Thr Leu
   305                 310                 315                 320
   Asp Gln Val Phe Lys Pro Leu Asp Pro Gly Ser Pro Gln Leu Pro Glu
                   325                 330                 335
   His Leu Glu Ser Gln Gln Lys Arg Pro Asn Pro Glu Leu Arg Arg Asn
               340                 345                 350
   Met Thr Ile Lys Thr Glu Leu Pro Leu Gly Ala Arg Arg Lys Met Lys
           355                 360                 365
   Pro Leu Leu Pro Arg Val Ser Ser Tyr Leu Val Pro Ile Gln Phe Pro
       370                 375                 380
   Val Asn Gln Ser Leu Val Leu Gln Pro Ser Val Lys Val Pro Leu Pro
   385                 390                 395                 400
   Leu Ala Ala Ser Leu Met Ser Ser Glu Leu Ala Arg His Ser Lys Arg
                   405                 410                 415
   Val Arg Ile Ala Pro Lys Val Phe Gly Glu Gln Val Val Phe Gly Tyr
               420                 425                 430
   Met Ser Lys Phe Phe Ser Gly Asp Leu Arg Asp Phe Gly Thr Pro Ile
           435                 440                 445
   Thr Ser Leu Phe Asn Phe Ile Phe Leu Cys Leu Ser Val Leu Leu Ala
       450                 455                 460
   Glu Glu Gly Ile Ala Pro Leu Ser Ser Ala Gly Pro Gly Lys Glu Glu
   465                 470                 475                 480
   Lys Leu Leu Phe Gly Glu Gly Phe Ser Pro Leu Leu Pro Val Gln Thr
                   485                 490                 495
   Ile Lys Glu Glu Glu Ile Gln Pro Gly Glu Glu Met Pro His Leu Ala
               500                 505                 510
   Arg Pro Ile Lys Val Glu Ser Pro Pro Leu Glu Glu Trp Pro Ser Pro
           515                 520                 525
   Ala Pro Ser Phe Lys Glu Glu Ser Ser His Ser Trp Glu Asp Ser Ser
       530                 535                 540
   Gln Ser Pro Thr Pro Arg Pro Lys Lys Ser Tyr Ser Gly Leu Arg Ser
   545                 550                 555                 560
   Pro Thr Arg Cys Val Ser Glu Met Leu Val Ile Gln His Arg Glu Arg
                   565                 570                 575
   Arg Glu Arg Ser Arg Ser Arg Arg Lys Gln His Leu Leu Pro Pro Cys
               580                 585                 590
   Val Asp Glu Pro Glu Leu Leu Phe Ser Glu Gly Pro Ser Thr Ser Arg
           595                 600                 605
   Trp Ala Ala Glu Leu Pro Phe Pro Ala Asp Ser Ser Asp Pro Ala Ser
       610                 615                 620
   Gln Leu Ser Tyr Ser Gln Glu Val Gly Gly Pro Phe Lys Thr Pro Ile
   625                 630                 635                 640
   Lys Glu Thr Leu Pro Ile Ser Ser Thr Pro Ser Lys Ser Val Leu Pro
                   645                 650                 655
   Arg Thr Pro Glu Ser Trp Arg Leu Thr Pro Pro Ala Lys Val Gly Gly
               660                 665                 670
   Leu Asp Phe Ser Pro Val Gln Thr Ser Gln Gly Ala Ser Asp Pro Leu
           675                 680                 685
   Pro Asp Pro Leu Gly Leu Met Asp Leu Ser Thr Thr Pro Leu Gln Ser
       690                 695                 700
   Ala Pro Pro Leu Glu Ser Pro Gln Arg Leu Leu Ser Ser Glu Pro Leu
   705                 710                 715                 720
   Asp Leu Ile Ser Val Pro Phe Gly Asn Ser Ser Pro Ser Asp Ile Asp
                   725                 730                 735
   Val Pro Lys Pro Gly Ser Pro Glu Pro Gln Val Ser Gly Leu Ala Ala
               740                 745                 750
   Asn Arg Ser Leu Thr Glu Gly Leu Val Leu Asp Thr Met Asn Asp Ser
           755                 760                 765
   Leu Ser Lys Ile Leu Leu Asp Ile Ser Phe Pro Gly Leu Asp Glu Asp
       770                 775                 780
   Pro Leu Gly Pro Asp Asn Ile Asn Trp Ser Gln Phe Ile Pro Glu Leu
   785                 790                 795                 800
   Gln
<210> 33
   <211> 1697
   <212> DNA
   <213> Homo sapiens
<220>
   <221> source
   <222> 1..1697
   <223> /mol_type="unassigned DNA"
   /organism="Homo sapiens"
<400> 33
   gaggcgtaag ccaggcgtgt taaagccggt cggaactgct ccggagggca cgggctccgt 60
   aggcaccaac tgcaaggacc cctccccctg cgggcgctcc catggcacag ttcgcgttcg 120
   agagtgacct gcactcgctg cttcagctgg atgcacccat ccccaatgca ccccctgcgc 180
   gctggcagcg caaagccaag gaagccgcag gcccggcccc ctcacccatg cgggccgcca 240
   accgatccca cagcgccggc aggactccgg gccgaactcc tggcaaatcc agttccaagg 300
   ttcagaccac tcctagcaaa cctggcggtg accgctatat cccccatcgc agtgctgccc 360
   agatggaggt ggccagcttc ctcctgagca aggagaacca gcctgaaaac agccagacgc 420
   ccaccaagaa ggaacatcag aaagcctggg ctttgaacct gaacggtttt gatgtagagg 480
   aagccaagat ccttcggctc agtggaaaac cacaaaatgc gccagagggt tatcagaaca 540
   gactgaaagt actctacagc caaaaggcca ctcctggctc cagccggaag acctgccgtt 600
   acattccttc cctgccagac cgtatcctgg atgcgcctga aatccgaaat gactattacc 660
   tgaaccttgt ggattggagt tctgggaatg tactggccgt ggcactggac aacagtgtgt 720
   acctgtggag tgcaagctct ggtgacatcc tgcagctttt gcaaatggag cagcctgggg 780
   aatatatatc ctctgtggcc tggatcaaag agggcaacta cttggctgtg ggcaccagca 840
   gtgctgaggt gcagctatgg gatgtgcagc agcagaaacg gcttcgaaat atgaccagtc 900
   actctgcccg agtgggctcc ctaagctgga acagctatat cctgtccagt ggttcacgtt 960
   ctggccacat ccaccaccat gatgttcggg tagcagaaca ccatgtggcc acactgagtg 1020
   gccacagcca ggaagtgtgt gggctgcgct gggccccaga tggacgacat ttggccagtg 1080
   gtggtaatga taacttggtc aatgtgtggc ctagtgctcc tggagagggt ggctgggttc 1140
   ctctgcagac attcacccag catcaagggg ctgtcaaggc cgtagcatgg tgtccctggc 1200
   agtccaatgt cctggcaaca ggagggggca ccagtgatcg acacattcgc atctggaatg 1260
   tgtgctctgg ggcctgtctg agtgccgtgg atgcccattc ccaggtgtgc tccatcctct 1320
   ggtctcccca ttacaaggag ctcatctcag gccatggctt tgcacagaac cagctagtta 1380
   tttggaagta cccaaccatg gccaaggtgg ctgaactcaa aggtcacaca tcccgggtcc 1440
   tgagtctgac catgagccca gatggggcca cagtggcatc cgcagcagca gatgagaccc 1500
   tgaggctatg gcgctgtttt gagttggacc ctgcgcggcg gcgggagcgg gagaaggcca 1560
   gtgcagccaa aagcagcctc atccaccaag gcatccgctg aagaccaacc catcacctca 1620
   gttgtttttt atttttctaa taaagtcatg tctcccttca tgtttttttt ttaaaaaaaa 1680
   aaaaaaaaaa aaaaaaa 1697
<210> 34
   <211> 499
   <212> PRT
   <213> Homo sapiens
<400> 34
   Met Ala Gln Phe Ala Phe Glu Ser Asp Leu His Ser Leu Leu Gln Leu
   1               5                   10                  15
   Asp Ala Pro Ile Pro Asn Ala Pro Pro Ala Arg Trp Gln Arg Lys Ala
               20                  25                  30
   Lys Glu Ala Ala Gly Pro Ala Pro Ser Pro Met Arg Ala Ala Asn Arg
           35                  40                  45
   Ser His Ser Ala Gly Arg Thr Pro Gly Arg Thr Pro Gly Lys Ser Ser
       50                  55                  60
   Ser Lys Val Gln Thr Thr Pro Ser Lys Pro Gly Gly Asp Arg Tyr Ile
   65                  70                  75                  80
   Pro His Arg Ser Ala Ala Gln Met Glu Val Ala Ser Phe Leu Leu Ser
                   85                  90                  95
   Lys Glu Asn Gln Pro Glu Asn Ser Gln Thr Pro Thr Lys Lys Glu His
               100                 105                 110
   Gln Lys Ala Trp Ala Leu Asn Leu Asn Gly Phe Asp Val Glu Glu Ala
           115                 120                 125
   Lys Ile Leu Arg Leu Ser Gly Lys Pro Gln Asn Ala Pro Glu Gly Tyr
       130                 135                 140
   Gln Asn Arg Leu Lys Val Leu Tyr Ser Gln Lys Ala Thr Pro Gly Ser
   145                 150                 155                 160
   Ser Arg Lys Thr Cys Arg Tyr Ile Pro Ser Leu Pro Asp Arg Ile Leu
                   165                 170                 175
   Asp Ala Pro Glu Ile Arg Asn Asp Tyr Tyr Leu Asn Leu Val Asp Trp
               180                 185                 190
   Ser Ser Gly Asn Val Leu Ala Val Ala Leu Asp Asn Ser Val Tyr Leu
           195                 200                 205
   Trp Ser Ala Ser Ser Gly Asp Ile Leu Gln Leu Leu Gln Met Glu Gln
       210                 215                 220
   Pro Gly Glu Tyr Ile Ser Ser Val Ala Trp Ile Lys Glu Gly Asn Tyr
   225                 230                 235                 240
   Leu Ala Val Gly Thr Ser Ser Ala Glu Val Gln Leu Trp Asp Val Gln
                   245                 250                 255
   Gln Gln Lys Arg Leu Arg Asn Met Thr Ser His Ser Ala Arg Val Gly
               260                 265                 270
   Ser Leu Ser Trp Asn Ser Tyr Ile Leu Ser Ser Gly Ser Arg Ser Gly
           275                 280                 285
   His Ile His His His Asp Val Arg Val Ala Glu His His Val Ala Thr
       290                 295                 300
   Leu Ser Gly His Ser Gln Glu Val Cys Gly Leu Arg Trp Ala Pro Asp
   305                 310                 315                 320
   Gly Arg His Leu Ala Ser Gly Gly Asn Asp Asn Leu Val Asn Val Trp
                   325                 330                 335
   Pro Ser Ala Pro Gly Glu Gly Gly Trp Val Pro Leu Gln Thr Phe Thr
               340                 345                 350
   Gln His Gln Gly Ala Val Lys Ala Val Ala Trp Cys Pro Trp Gln Ser
           355                 360                 365
   Asn Val Leu Ala Thr Gly Gly Gly Thr Ser Asp Arg His Ile Arg Ile
       370                 375                 380
   Trp Asn Val Cys Ser Gly Ala Cys Leu Ser Ala Val Asp Ala His Ser
   385                 390                 395                 400
   Gln Val Cys Ser Ile Leu Trp Ser Pro His Tyr Lys Glu Leu Ile Ser
                   405                 410                 415
   Gly His Gly Phe Ala Gln Asn Gln Leu Val Ile Trp Lys Tyr Pro Thr
               420                 425                 430
   Met Ala Lys Val Ala Glu Leu Lys Gly His Thr Ser Arg Val Leu Ser
           435                 440                 445
   Leu Thr Met Ser Pro Asp Gly Ala Thr Val Ala Ser Ala Ala Ala Asp
       450                 455                 460
   Glu Thr Leu Arg Leu Trp Arg Cys Phe Glu Leu Asp Pro Ala Arg Arg
   465                 470                 475                 480
   Arg Glu Arg Glu Lys Ala Ser Ala Ala Lys Ser Ser Leu Ile His Gln
                   485                 490                 495
   Gly Ile Arg